# EUROPÄISCHE PATENTSCHRIFT

(11) **EP 2 268 819 B1**
(45) Veröffentlichungstag und Bekanntmachung des Hinweises auf die Patenterteilung: **24.04.2013**
(21) Anmeldenummer: 09719156.3
(22) Anmeldetag: 12.03.2009
(51) Int. Cl.: C12N 15/82

(54) **VERFAHREN ZUR ERZEUGUNG EINER BREITBAND-RESISTENZ GEGENÜBER PILZEN IN TRANSGENEN PFLANZEN**
METHOD FOR CREATING BROAD-SPECTRUM RESISTANCE TO FUNGI IN TRANSGENIC PLANTS
PROCÉDÉ DE CRÉATION D'UNE RÉSISTANCE À LARGE SPECTRE CONTRE DES CHAMPIGNONS DANS DES PLANTES TRANSGÉNIQUES

(30) Priorität: 13.03.2008 DE 102008014041
(43) Veröffentlichungstag der Anmeldung: 05.01.2011
(73) Patentinhaber: Leibniz-Institut für Pflanzengenetik und Kulturpflanzenforschung (IPK), 06466 Gatersleben (DE)
(72) Erfinder: SCHWEIZER, Patrick, 06493 Ballenstedt (DE); HENSEL, Götz, 06366 Köthen (DE); GAY, Alexandra, 51519 Odenthal (DE); KUMLEHN, Jochen, 14469 Potsdam (DE)
(74) Vertreter: Neuefeind, Regina
(86) Internationale Anmeldenummer: PCT/EP2009/001805
(87) Internationale Veröffentlichungsnummer: WO 2009/112270

(56) Entgegenhaltungen:
- WO-A2-2005/019408
- WO-A2-2006/047495
- WO-A2-2006/097465
- US-A1- 2006 123 505
- US-A1- 2007 044 171
- US-A1- 2007 061 918
- DOUCHKOV D ET AL: "A high-throughput gene-silencing system for the functional assessment of defense-related genes in barley epidermal cells" MOLECULAR PLANT-MICROBE INTERACTIONS, APS PRESS, ST. PAUL, MN, US, Bd. 18, Nr. 8, 1. August 2005 (2005-08-01) , Seiten 755-761, XP002397481 ISSN: 0894-0282
- SCHWEIZER P ET AL: "Double-stranded RNA interferes with gene function at the single-cell level in cereals" PLANT JOURNAL, BLACKWELL SCIENTIFIC PUBLICATIONS, OXFORD, GB, Bd. 24, Nr. 6, 1. Dezember 2000 (2000-12-01), Seiten 895-903, XP002397482 ISSN: 0960-7412
- PADDISON P J ET AL: "A resource for large-scale RNA-interference-based screens in mammals" NATURE, NATURE PUBLISHING GROUP, LONDON, UK, Bd. 428, Nr. 6981, 25. März 2004 (2004-03-25), Seiten 427-431, XP002992273 ISSN: 0028-0836
- WATERHOUSE ET AL: "Virus resistance and gene silencing in plants can be induced by simultaneous expression of sense and antisense RNA" PROCEEDINGS OF THE NATIONAL ACADEMY OF SCIENCES OF USA, NATIONAL ACADEMY OF SCIENCE, WASHINGTON, DC, US, Bd. 95, 1. November 1998 (1998-11-01), Seiten 13959-13964, XP002114472 ISSN: 0027-8424

## Beschreibung

Die Erfindung betrifft ein Verfahren zur Erzeugung einer Breitband-Resistenz gegenüber Pilzen in transgenen Pflanzen, dadurch gekennzeichnet, dass ein rekombinantes Nukleinsäuremolekül umfassend mindestens eine Nukleinsäuresequenz in die Pflanze eingebracht wird, wobei die Nukleinsäuresequenz identisch und/oder komplementär zu einer oder mehreren der Nukleinsäuren ausgewählt aus SEQ ID Nos 1-16 und/oder einem Teil davon ist, oder eine Sequenzidentität von mindestens 85% zu einer oder mehreren der Nukleinsäuren ausgewählt aus SEQ ID Nos 1-16 aufweist und in der Pflanze transkribiert wird, so dass bei Infektion der Pflanze mit einem Pilz die Nukleinsäuresequenz oder Teile davon mit einer oder mehreren der korrespondierenden und/oder komplementären Nukleinsäuren des Pilzes derart interagiert, dass die Expression der pilzeigenen Nukleinsäuresequenz im Wesentlichen unterbunden wird.

Die Erfindung betrifft weiterhin das erfindungsgemäße Nukleinsäuremolekül, sowie Pflanzen enthaltend ein solches Nukleinsäuremolekül.

Die vorliegende Erfindung betrifft die Erzeugung einer Breitband-Resistenz in transgenen Pflanzen durch das Einbringen von inhibitorischen Nukleinsäuresequenzen, die die Expression von Pilzgenen unterbinden.

Die Nukleinsäuresequenzen von Genen, die für die Entwicklung, das Wachstum und die Vermehrung von Pilzen von Bedeutung sind, also Sequenzen von Genen mit essenzieller Funktion in Pilzen, sind oft konserviert bzw. weisen eine hohe Sequenzidentität auf. Bereiche die eine besonders hohe Sequenzidentität zwischen verschiedenen Pilzen aufweisen, werden genutzt, um inhibitorische Genkonstrukte herzustellen, etwa auf Basis der antisense-, siRNA-, shRNA-, Ribozym-Technologie und anderen Technologien, die die Hemmung der Expression und Aktivierung von Genen vermitteln. Da diese Verfahren auf der sequenzspezifischen Hybridisierung der inhibitorischen RNA-Moleküle mit entsprechenden Zielsequenzen in bestimmten Pilzgenen beruhen, wird damit die Expression aller korrespondierender Gene aus verschiedenen Pilzen ebenfalls gehemmt. Aufgrund der hohen Sequenzidentität solcher konservierter Genabschnitte werden so transgene Pflanzen hergestellt, die eine Breitband-Resistenz gegenüber verschiedenen Pilzen aufweisen.

Pflanzenkrankheiten, die durch verschiedene Pathogene wie z. B. Viren, Bakterien und Pilze verursacht werden, können beim Anbau von Kulturpflanzen zu erheblichen Ernteausfällen führen, was zum einen wirtschaftliche Folgen hat, aber auch die Sicherstellung der menschlichen Ernährung bedroht. So kann etwa der Befall von Getreide mit *Blumeria graminis,* dem Erreger des Echten Mehltaus, zu Ertragsverlusten von mehr als 30% führen.

Seit dem letzten Jahrhundert werden zur Kontrolle von Pilzerkrankungen chemische Fungizide eingesetzt. Durch den Einsatz dieser Stoffe konnte zwar das Ausmaß von Pflanzenerkrankungen reduziert werden, es ist allerdings bis heute nicht auszuschließen, dass diese Verbindungen eine schädliche Wirkung auf Mensch, Tier und Umwelt ausüben. Um langfristig den Verbrauch von herkömmlichen Pflanzenschutzmitteln auf ein Minimum zu reduzieren, ist es daher von Bedeutung, die natürliche Pathogenabwehr von verschiedenen Pflanzen gegenüber unterschiedlichen Erregern zu untersuchen und sie sich durch gentechnologische Manipulation, z. B. durch das Einführen externer Resistenzgene oder durch die Manipulation der endogenen Genexpression in den Pflanzen, zur Herstellung von pathogenresistenten Pflanzen gezielt nutzbar zu machen.

Als Resistenz bezeichnet man die Fähigkeit einer Pflanze, Befall und Besiedelung durch einen Schaderreger zu verhindern oder zumindest zu begrenzen. Die Pflanzen verfügen über ein gewisses Maß an natürlicher Resistenz, die durch die Bildung von spezifischen Abwehrsubstanzen wie Isoprenoiden, Flavonoiden, Enzymen und reaktiven Sauerstoffverbindungen vermittelt wird.

Ein Ansatz zur Herstellung resistenter Pflanzen, ist die Expression eines pflanzlichen Transgens in solchen Pflanzen, was zur Bildung dieser spezifischen Abwehrsubstanzen führt. So konnten bereits Chitinase- (WO 92/17591) und Pathogenese-verwandte Gene (WO 92/20800) sowie Gene für verschiedene oxidierende Enzyme wie etwa Glucoseoxidase (WO 95/21924) und Oxalatoxidase (WO 99/04013) in Pflanzen überexprimiert und dadurch Pflanzen mit erhöhter Pilzresistenz hergestellt werden.

Umgekehrt konnte aber auch gezeigt werden, dass manche pflanzlichen Gene erst den Eintritt des Pilzes in die Pflanze ermöglichen. Daher besteht ein alternativer Ansatz zur Herstellung transgener Pflanzen mit erhöhter Pilzresistenz darin, die Expression dieser pflanzlichen Gene, die z. B. für eine Polyphenoloxidase (WO 02/061101), NADPH-Oxidase (WO 2004/009820) und das Mlo-Gen (WO 00/01722) kodieren, in transgenen Pflanzen zu hemmen.

Eine weitere Alternative zur Auslösung einer Resistenz gegen pathogene Pilze besteht darin, Genkonstrukte, die die Expression und/oder Aktivität von Pilz-Genen hemmen, die für die Vermehrung und/oder Entwicklung von Pilzen essenziell sind, in Pflanzen einzubringen (US Patent Nr. 2007/0061918).

Nach wie vor stehen nicht gegen alle Pilzpathogene Resistenzstrategien zur Verfügung. Dies ist umso gravierender als praktisch jede Nutzpflanze von einer Vielzahl pathogener Pilze befallen werden kann. Es besteht daher ein dringender Bedarf nach Strategien mit denen eine Pflanze gleichzeitig gegen eine Vielzahl von potenziellen Schadpilzen resistent gemacht werden kann.

Aufgabe der vorliegenden Erfindung ist es daher, ein Verfahren zur Verfügung zu stellen, bei dem transgene Pflanzen mit einer Breitband-Resistenz gegenüber verschiedenen Pilzpathogenen hergestellt werden können.

Zur Lösung dieser und weiterer Aufgaben, wie sie sich aus der Beschreibung ergeben, dienen die Merkmale der unabhängigen Ansprüche.

Bevorzugte Ausführungsformen der Erfindung sind durch die Merkmale der Unteransprüche definiert.

Ein Aspekt der vorliegenden Erfindung betrifft ein Verfahren zur Erzeugung einer Breitband-Resistenz gegenüber Pilzen in transgenen Pflanzen,
dadurch gekennzeichnet, dass ein rekombinantes Nukleinsäuremolekül umfassend mindestens eine Nukleinsäuresequenz in die Pflanze eingebracht wird, wobei die Nukleinsäuresequenz
a) identisch und/oder komplementär zu einer oder mehreren der Nukleinsäuren ausgewählt aus SEQ ID Nos 1-5 und/oder einem Teil davon ist, oder eine Sequenzidentität von mindestens 85% zu einer oder mehreren der Nukleinsäuren ausgewählt aus SEQ ID Nos 1-5 aufweist, und/oder
b) identisch und/oder komplementär zu einer oder mehreren der Nukleinsäuren ausgewählt aus SEQ ID Nos 6-11 und/oder einem Teil davon ist, oder eine Sequenzidentität von mindestens 85% zu einer oder mehreren der Nukleinsäuren ausgewählt aus SEQ ID Nos 6-11 aufweist, oder
c) identisch und/oder komplementär zu einer oder mehreren der Nukleinsäuren ausgewählt aus SEQ ID Nos 12-16 und/oder einem Teil davon ist, oder eine Sequenzidentität von mindestens 85% zu einer oder mehreren der Nukleinsäuren ausgewählt aus SEQ ID Nos 12-16 aufweist,
und in der Pflanze transkribiert wird, so dass bei Infektion der Pflanze mit einem Pilz die Nukleinsäuresequenz oder Teile davon mit einer oder mehreren der korrespondierenden und/oder komplementären Nukleinsäuren des Pilzes derart interagiert, dass die Expression der pilzeigenen Nukleinsäuresequenz im Wesentlichen unterbunden wird.

Im Sinne der vorliegenden Erfindung bedeutet "Sequenzidentität" den Grad von Übereinstimmung hinsichtlich der 5'-3' Abfolge innerhalb einer Sequenz von Nukleotideinheiten innerhalb eines Nukleinsäuremoleküls beim Vergleich mit einem anderen Nukleinsäuremolekül. Die Sequenzidentität wird über eine Reihe von Programmen, die auf verschiedenen Algorithmen beruhen, bestimmt, beipielsweise mit BLASTN, ScanProsite, der Laser gene-Software etc. Alternativ dazu kann auch das BLAST Programm-Paket des National Center for Biotechnology Information (http://www.ncbi.nim.nih.gov/) verwendet werden. Hier wurde zusätzlich für Sequenzvergleiche auch das Programm Sequencher (Gene Codes Corp., Ann Arbor, MI, USA) unter Verwendung des "Dirtydata"-Algorithmus verwendet.

In einer bevorzugten Ausführungsform ist die in die Pflanze eingebrachte mindestens eine Nukleinsäuresequenz mindestens zu 50%, bevorzugt zu mindestens 60%, ebenfalls bevorzugt zu mindestens 70%, besonders bevorzugt zu mindestens 80%, insbesondere bevorzugt zu 90, 92 oder 94% und am meisten bevorzugt zu mindestens 96, 98 oder 99% oder 100% identisch zu einer Nukleinsäuresequenz aus Pilzen.

In einer weiteren bevorzugten Ausführungsform ist die in die Pflanze eingebrachte mindestens eine Nukleinsäuresequenz mindestens zu 50%, bevorzugt zu mindestens 60%, ebenfalls bevorzugt zu mindestens 70%, besonders bevorzugt zu mindestens 80%, insbesondere bevorzugt zu 90, 92 oder 94% und am meisten bevorzugt zu mindestens 96, 98 oder 99% oder 100% identisch zu einer für den Pilz essenziellen Nukleinsäuresequenz.

Eine "essenzielle Nukleinsäuresequenz für den Pilz" im Sinne der Erfindung, ist eine Nukleinsäuresequenz, die eine zentrale Aufgabe bei der Entwicklung, dem Wachstum und der Vermehrung von Pilzen erfüllt, so dass die Unterbindung der Expression dieser Nukleinsäure zum Tod des Pilzes führt oder das Wachstum und/oder die Entwicklung des Pilzes arretiert.

In einer bevorzugten Ausführungsform des erfindungsgemäßen Verfahrens werden konservierte Abschnitte innerhalb essenzieller Pilz-Nukleinsäuresequenzen als Zielsequenzen für die erfindungsgemäße Reduktion des Expressionslevels der entsprechendenden pilzlichen Nukleinsäuresequenz verwendet.

Eine "korrespondierende" Nukleinsäuresequenz im Sinne der Erfindung ist dabei synonym zu einer homologen Nukleinsäuresequenz, also einer Nukleinsäuresequenz aus einer Pilzspezies mit identischer oder nahezu identischer biologischer Funktion und üblicherweise auch einer hohen bzw. sehr hohen Sequenzidentität. Korrespondierende Pilz-Nukleinsäuresequenzen sind erfindungsgemäß zu mindestens 50%, bevorzugt mindestens 60%, ebenfalls bevorzugt mindestens 70%, besonders bevorzugt mindestens 80%, insbesondere bevorzugt 85, 90, 92 oder 94% und am meisten bevorzugt mindestens 96, 98 oder 99% oder 100% identisch zu einer Nukleinsäuresequenz aus einem anderen Pilz.

Der Begriff "konservierte Abschnitte" oder Zielsequenzen bezieht sich auf die hohe Identität von Nukleinsäuresequenzen, in der Regel die DNA-Sequenz eines Gens, bzw. die davon transkribierte RNA-Sequenz und/oder mRNA-Sequenz beim Vergleich von Organismen die evolutiv eng verwandt sind. Als geeignete konservierte Genabschnitte werden im Rahmen der vorliegenden Erfindung Bereiche verstanden, bei denen aufgrund eines Sequenzvergleichs mit dem Genom anderer Pilzarten eine Aussicht darauf besteht, dass die Reduktion des Expressionslevels des zugrundeliegende Pilzgens zu einer Resistenz mindestens diesem Pilz aber möglichst auch weiteren Pilzen gegenüber führen wird.

Die Erfinder konnten mithilfe von Sequenzvergleichen konservierte Abschnitte in essenziellen Pilzgenen ermitteln. Diese wurden als Zielsequenzen verwendet, um entsprechende RNAi-Konstrukte abzuleiten, welche eine Breitband-Resistenz gegenüber Pilzen in transgenen Pflanzen vermittelten. Bei den essenziellen Genen handelt es sich um
a) den mitochondrialen ADP/ATP-Translokator (AAC)
b) den Elongationsfaktor 1 (EF1)
c) Gamma-Aktin (GA)
d) das mitochondriale Hitzeschockprotein 70 (Hsp)
e) die Rho GTPase (Rho)

Mit RNAi-Konstrukten abgeleitet aus den entsprechenden Gensequenzen von *Fusarium culmorum* konnten die Erfinder in Gerste eine Resistenz sowohl gegenüber *Fusarium culmorum* als auch gegenüber *Blumeria graminis* erzeugen.

Dabei wurde sichergestellt, dass entsprechende Gen-Abschnitte oder eine dazu homologe Sequenz nicht endogen in der zu verwendenden Pflanze vorkommen, da anderenfalls auch endogen in der Pflanze stattfindende Prozesse gehemmt werden könnten, was unerwünscht ist.

In einer bevorzugten Ausführungsform des erfindungsgemäßen Verfahrens umfasst die erfindungsgemäße Nukleinsäuresequenz konservierte Abschnitte innerhalb essenzieller Pilzgene als Zielsequenzen für die erfindungsgemäße Reduktion des Expressionslevels der korrespondierenden pilzlichen Nukleinsäuresequenz.

In einer weiteren bevorzugten Ausführungsform weist die erfindungsgemäße mindestens eine Nukleinsäuresequenz, gegenüber korrespondierenden Nukleinsäurensequenzen bzw. Zielsequenzen in Nukleinsäuresequenzen aus Pilzen und besonders bevorzugt aus Schadpilzen über einen Bereich von mindestens 100bp, bevorzugt 150bp, bevorzugt 200bp, besonders bevorzugt 250bp und ebenfalls besonders bevorzugt 300bp eine Sequenzidentität von mindestens 85%; mindestens 90% oder mindestens 95% auf.

In einer weiteren bevorzugten Ausführungsform weist die erfindungsgemäße mindestens eine Nukleinsäuresequenz, gegenüber korrespondierenden Nukleinsäurensequenzen aus Pflanzen über ein "sliding window" von 100bp eine Sequenzidentität von maximal 80%, bevorzugt 75% und meisten bevorzugt maximal 70% auf.

Ein "sliding window" im Sinne der Erfindung ist hierbei eine Nukleotid-Abfolge von 100bp Länge, die innerhalb einer gegebenen Nukleotidsequenz beliebig verschiebbar ist.

In einer besonders bevorzugten Ausführungsform des erfindungsgemäßen Verfahrens handelt es sich bei der korrespondierenden und/oder komplementären Nukleinsäure des Pilzes, deren Expression im Wesentlichen durch die erfindungsgemäße Nukleinsäuresequenz unterbunden wird um
a) den mitochondriale ADP/ATP-Transporter (AAC)
b) den Elongationsfaktor 1 (EF1)
c) Gamma-Aktin (GA)
d) das mitochondriale Hitzeschockprotein 70 (Hsp)
e) die Rho-GTPase (Rho)

Der Begriff "Resistenz" meint das Vermindern oder Abschwächen von Krankheitssymptomen einer Pflanze infolge eines Befalls durch einen Schädling bzw. ein Pathogen und bevorzugt durch einen Pilz und besonders bevorzugt durch mehrere Pilze. Die Symptome können vielfältiger Art sein, umfassen aber bevorzugt solche, die direkt oder indirekt zu einer Beeinträchtigung der Qualität der Pflanze, der Quantität des Ertrags, der Eignung zur Verwendung als Futter- oder Nahrungsmittel führen oder aber auch Aussaat, Anbau, Ernte oder Prozessierung des Ernteguts erschweren. Ebenso bedeutet "Resistenz", dass ein Schädling bzw. ein Pathogen und bevorzugt ein Pilz und besonders bevorzugt mehrere Pilze in einer Pflanze ein vermindertes Wachstum und eine verminderte oder ausbleibende Weiterverbreitung zeigt. Der Begriff "Resistenz" beinhaltet dabei auch eine so genannte transiente Resistenz, d.h. dass die erfindungsgemäßen transgenen Pflanzen bzw. Pflanzenzellen nur für einen begrenzten Zeitraum eine gegenüber dem entsprechenden Wildtyp erhöhte Schädlings-, bzw. Pathogen- oder Pilzresistenz aufweisen.

Unter dem Begriff "Breitband-Resistenz" wird erfindungsgemäß verstanden, dass die erfindungsgemäßen transgenen Pflanzen bzw. Pflanzenzellen von mehr als einer Pilzart weniger stark und/oder weniger häufig infiziert oder befallen werden als nicht-transformierte Wildtyp-Pflanzen bzw. Pflanzenzellen, die ansonsten gleich behandelt wurden (z. B. Klima- und Anbaubedingungen, Pilzart, usw.). Bevorzugt ist der Befall durch mindestens einen Pilz, mindestens zwei verschiedene Pilze, besonders bevorzugt durch mindestens drei verschiedene Pilze, insbesondere bevorzugt durch mindestens vier verschiedene Pilze und am meisten bevorzugt durch mindestens fünf verschiedene Pilze reduziert, was sich in einer Verminderung der Ausbildung von Krankheitssymptomen äußert. Experimentell kann eine solche Pilzresistenz unter anderem durch eine Verminderung der Haustorienbildung und/oder des Hyphenwachstums nachgewiesen werden.

"Im Wesentlichen unterbunden" bedeutet im Rahmen der vorliegenden Erfindung, dass das Expressionslevel der Nukleinsäuresequenz des Pilzes in den transgenen Pflanzen auf ein Niveau reduziert ist, bei dem die Pflanzen eine erhöhte Resistenz gegenüber Pilzinfektionen zeigen bzw. nach anfänglichen Symptomen einer Pilzinfektion Anzeichen für eine Erholung aufweisen. Unter "transgenen Pflanzen mit Breitband-Resistenz" werden im Rahmen der vorliegenden Erfindung auch solche Pflanzen verstanden, die geringe Symptome einer Pilzinfektion zeigen, die jedoch so schwach ausgeprägt sind, dass die Verwendbarkeit und Nutzbarkeit der befallenen Pflanzen nicht in Frage gestellt ist. Erfindungsgemäße Pflanzen weisen einen gegenüber Wildtyp-Pflanzen im Wesentlichen unveränderten Phänotyp auf, d.h. die Verwendung als Nutz-, Nahrungs- oder Futterpflanze usw. ist nicht in Frage gestellt.

Wenn im Rahmen der vorliegenden Erfindung von Nukleinsäuresequenzen gesprochen wird, die für Pilzgene oder Teile davon kodieren, dann sind damit sowohl die komplette kodierende DNA-Sequenz des jeweiligen Pilzgens als auch die komplette mRNA-Sequenz bzw. die jeweiligen Teilbereiche gemeint.

Das Expressionslevel der Nukleinsäure des Pilzes, deren Expression durch die in der transgenen Pflanze transkribierte Nukleinsäure im Wesentlichen unterbunden wird, lässt sich in befallenen Wildtyppflanzen wie auch in den transgenen Pflanzen z. B. dadurch bestimmen, dass eine RT-PCR-Analyse oder eine Northern Blot-Analyse mit spezifischen Primern bzw. Sonden durchgeführt wird. Dem Fachmann ist klar, wie er die Sonden bzw. Primer auswählen muss, um die Expression der Nukleinsäure des Pilzes zu untersuchen. Bevorzugt ist das Expressionslevel der Nukleinsäuresequenz des Pilzes um statistisch signifikant mindestens 80%, insbesondere bevorzugt um mindestens 90%, ebenfalls insbesondere bevorzugt um mindestens 95% und am meisten bevorzugt um mindestens 98 oder 99% vermindert.

In einer weiteren bevorzugten Ausführungsform des erfindungsgemäßen Verfahrens umfasst die mindestens eine Nukleinsäuresequenz mindestens 19 Nukleotide, mindestens 20 Nukleotide, mindestens 21 Nukleotide, mindestens 22 Nukleotide, mindestens 23 Nukleotide, mindestens 24 Nukleotide, mindestens 25 Nukleotide oder mindestens 50 Nukleotide.

In einer weiteren bevorzugten Ausführungsform des erfindungsgemäßen Verfahrens umfasst die mindestens eine Nukleinsäuresequenz mindestens 50 Nukleotide, mindestens 100 Nukleotide, mindestens 150 Nukleotide, mindestens 200 Nukleotide, mindestens 250 Nukleotide, mindestens 300 Nukleotide, mindestens 400 Nukleotide oder mindestens 500 Nukleotide.

In einer bevorzugten Ausführungsform umfasst die erfindungsgemäße mindestens eine Nukleinsäuresequenz mindestens 19 Nukleotide, mindestens 20 Nukleotide, mindestens 21 Nukleotide, mindestens 22 Nukleotide, mindestens 23 Nukleotide, mindestens 24 Nukleotide, mindestens 25 Nukleotide oder mindestens 50 Nukleotide mit eine Sequenzidentität von mindestens 40%, bevorzugt mindestens 50%, weiter bevorzugt mindestens 60%, ebenfalls bevorzugt mindestens 70% und/oder 75%, besonders bevorzugt mindestens 80%, 82%, 84%, 86% und/oder 88%, insbesondere bevorzugt mindestens 90%, 92% und/oder 94% und am meisten bevorzugt mindestens 95%, 96%, 97%, 98% und/oder 99% gegenüber einer anderen Nukleinsäuresequenz, wobei die Nukleinsäuresequenz bevorzugt aus Pilzen stammt.

In einer weiteren bevorzugten Ausführungsform umfasst die erfindungsgemäße mindestens eine Nukleinsäuresequenz mindestens 50 Nukleotide, mindestens 100 Nukleotide, mindestens 150 Nukleotide, mindestens 200 Nukleotide, mindestens 250 Nukleotide, mindestens 300 Nukleotide, mindestens 400 Nukleotide oder mindestens 500 Nukleotide über eine Sequenzidentität von mindestens 40%, bevorzugt mindestens 50%, weiter bevorzugt mindestens 60%, ebenfalls bevorzugt mindestens 70% und/oder 75%, besonders bevorzugt mindestens 80%, 82%, 84%, 86% und/oder 88%, insbesondere bevorzugt mindestens 90%, 92% und/oder 94% und am meisten bevorzugt mindestens 95%, 96%, 97%, 98% und/oder 99% zu einer anderen Nukleinsäuresequenz, wobei die Nukleinsäuresequenz bevorzugt aus Pilzen stammt.

Im Sinne der vorliegenden Erfindung bezieht sich der Begriff "identisch" auf den Grad von Sequenzidentität einer Nukleinsäuresequenz gegenüber einer anderen Nukleinsäuresequenz. Identische Nukleinsäuresequenz im Sinne der Erfindung verfügen über eine Sequenzidentität von mindestens 40%, mindestens 50%, mindestens 60%, bevorzugt mindestens 70%, besonders bevorzugt mindestens 80%, ebenfalls besonders bevorzugt mindestens 90%, insbesondere bevorzugt mindestens 95% und am meisten bevorzugt mindestens 98 bzw. 100% gegenüber einer anderen Nukleinsäuresequenz

Mit dem Begriff "komplementär" wird die Fähigkeit eines Nukleinsäuresequenz beschrieben, mit einer anderen Nukleinsäuresequenz aufgrund von Wasserstoffbrücken zwischen komplementären Basen zu hybridisieren. Der Fachmann weiß, dass zwei Nukleinsäuremoleküle nicht über eine 100-prozentige Komplementarität verfügen müssen, um miteinander hybridisieren zu können. Bevorzugt ist eine Nukleinsäuresequenz, die mit einer anderen Nukleinsäuresequenz hybridisieren soll, zu dieser zu mindestens 40%, zu mindestens 50%, zu mindestens 60%, bevorzugt zu mindestens 70%, besonders bevorzugt zu mindestens 80%, ebenfalls besonders bevorzugt zu mindestens 90%, insbesondere bevorzugt zu mindestens 95% und am meisten bevorzugt zu mindestens 98 bzw. 100% komplementär.

Das "rekombinante Nukleinsäuremolekül" sind erfindungsgemäß alle Vektoren, Plasmide, Cosmide, Viren und andere in der Gentechnik gängige Vektoren, mit denen Nukleinsäuremoleküle auf Pflanzen bzw. Pflanzenzellen übertragen werden können.

Zur Vorbereitung der Einführung fremder Gene in höhere Pflanzen bzw. deren Zellen stehen eine große Anzahl von Klonierungsvektoren zur Verfügung, die ein Replikationssignal für *E. coli* und ein Markergen zur Selektion transformierter Bakterienzellen enthalten. Beispiele für derartige Vektoren sind pBR322, pUC-Serien, M13mp-Serien, pACYC184 usw. Die gewünschte Sequenz kann an einer passenden Restriktionsschnittstelle in den Vektor eingeführt werden. Das erhaltende Plasmid wird für die Transformation von E. coli-Zellen verwendet. Transformierte *E. coli*-Zellen werden in einem geeigneten Medium gezüchtet und anschließend geerntet und lysiert. Das Plasmid wird wiedergewonnen. Als Analysemethode zur Charakterisierung der gewonnenen Plasmid-DNA werden im Allgemeinen Restriktionsanalysen, Gelelektrophoresen und weitere biochemischmolekularbiologische Methoden eingesetzt. Nach jeder Manipulation kann die Plasmid-DNA gespalten und gewonnene DNA-Fragmente können mit anderen DNA-Sequenzen verknüpft werden. Jede Plasmid-DNA-Sequenz kann in das gleiche oder andere Plasmide kloniert werden. Standardverfahren zur Klonierung können Sambrook et al., 2001 (Molecular cloning: A laboratory manual, 3rd edition, Cold Spring Harbor Laboratory Press) entnommen werden.

Bei dem erfindungsgemäßen Verfahren zur Herstellung von transgenen Pflanzen mit einer Breitband-Resistenz gegenüber Pilzen kann die Eigenschaft der mindestens einen Nukleinsäuresequenz, die Suppression der Expression einer Nukleinsäuresequenz eines Pilzes zu vermitteln, die Pflanze auch gegenüber allen anderen Pilzen resistent machen, die über den entsprechenden hochkonservierten Bereich in diesem Gen verfügen. Wie unten erläutert wird, beruhen Verfahren zur Suppression der Expression einer Nukleinsäuresequenz auf spezifischer komplementärer Basenpaarung von inhibitorischen Nukleinsäuremolekülen mit Sequenzen eines pilzlichen zellulären Nukleinsäuremoleküls. Aufgrund der hohen Sequenzidentität konservierter Bereiche in einem oder mehreren essenziellen Genen verschiedener Pilze kann so beispielweise eine Nukleinsäuresequenz abgeleitet aus Pilz A verwendet werden, um die Infektion einer Pflanze durch Pilz A und Pilz B und/oder durch Pilze A, B, C und D etc. zu verhindern (vgl. u.).

In einer bevorzugten Ausführungsform umfasst die mindestens eine Nukleinsäuresequenz mehrere Nukleinsäuresequenzen, die zu mehreren verschiedenen Nukleinsäuresequenzen eines Pilzes homolog und/oder komplementär sind und die in der Pflanze transkribiert werden, so dass bei Infektion der Pflanze mit diesem Pilz die in der Pflanze transkribierten Nukleinsäuresequenzen mit den jeweils homologen und/oder komplementären Nukleinsäuresequenzen des Pilzes oder Teilen davon interagieren, so dass die Expression der Nukleinsäuresequenzen des Pilzes im Wesentlichen unterbunden wird. Dadurch kann die Expression mehrerer Pilzgene gleichzeitig durch die Pflanze gehemmt werden und der transgenen Pflanze eine erhöhte Resistenz gegen einzelne oder bevorzugt verschiedene Pilzpathogene vermittelt werden.

In einer bevorzugten Ausführungsform des erfindungsgemäßen Verfahrens umfasst das rekombinante Nukleinsäuremolekül zwei Nukleinsäuresequenzen.

In einer weiteren bevorzugten Ausführungsform des erfindungsgemäßen Verfahrens umfasst das rekombinante Nukleinsäuremolekül drei Nukleinsäuresequenzen.

Die Hemmung der Expression einer Nukleinsäure eines Organismus wird vom Fachmann auch als "gene silencing" bezeichnet. Da hier das "gene silencing" des pilzeigenen Gens durch eine Nukleinsäuresequenz bewirkt wird, die vom Wirt des Pilzes, der Pflanze, gebildet wird, wird dieses Verfahren auch als "host-induced gene silencing" (HIGS) bezeichnet.

Der Fachmann kennt verschiedene Methoden, die ein "gene silencing" bewirken können. Die Expression der Nukleinsäuresequenz eines Pilzes kann in transgenen Pflanzen z. B. durch "silencing" im Wesentlichen unterdrückt werden. Beim silencing wird eine Nukleinsäuresequenz, die identisch zu einer Nukleinsäuresequenz eines Pilzes und/oder dazu komplementär ist, auf die Pflanze übertragen. Um sicherzustellen, dass die Pflanzen für die übertragenen Nukleinsäuren transgen sind, wird die zu übertragende Nukleinsäure in der Regel durch einen Vektor, wie z. B. ein Plasmid auf die Pflanze übertragen, der in der Lage ist, sich innerhalb der Pflanzenzelle stabil zu replizieren oder die übertragene Nukleinsäure in das pflanzliche Genom zu integrieren.

In diesem Zusammenhang spricht der Fachmann hinsichtlich einer zu einer Nukleinsäuresequenz komplementären Nukleinsäuresequenz auch von einer Antisense-Nukleinsäure, wobei es sich typischerweise um eine Antisense-RNA handelt. Die Verwendung von Antisense-RNA führt zur Suppression des korrepondierenden endogenen Gens. Eine zu einer Nukleinsäuresequenz identischen Nukleinsäuresequenz wird auch als Sense-Nukleinsäure bezeichnet. Die Verwendung von Sense-RNA kann ebenfalls zur Suppression des korrepondierenden endogenen Gens führen, durch einen Prozess der "Ko-Suppression" genannt wird.

Wenn im Rahmen der vorliegenden Erfindung von Sense-Sequenzen gesprochen wird, dann sind damit diejenigen Sequenzen gemeint, die dem kodierenden Strang einer Nukleinsäuresequenz eines Pilzes entsprechen oder Teile davon umfassen. Solche Sequenzen müssen jedoch nicht zu 100% identisch mit den interessierenden Pilzgenen sein. Es sollte ausreichen, wenn diese Sequenzen zu mindestens 50% identisch sind, bevorzugt zu mindestens 60%, besonders bevorzugt zu mindestens 70%, weiterhin besonders bevorzugt zu mindestens 80%, insbesondere bevorzugt zu mindestens 90% und am meisten bevorzugt zu mindestens 95% identisch sind. Bei solchen Identitätsgraden wird erfindungsgemäß davon gesprochen, dass die Sequenzen zueinander homolog sind bzw. eine Homologie aufweisen. Die Abweichungen zu den Nukleinsäuren von Pilzen oder Teile davon können dabei durch Deletion, Addition, Substitution und/oder Insertion entstanden sein. Dem Fachmann ist natürlich klar, dass mit abnehmender Identität die Wahrscheinlichkeit steigt, dass mehrere Nukleinsäuren gesilenct unterdrückt werden. Sequenzen, deren Identitäts- bzw. Homologiegrad so gering ist, dass die Expression endogener Gene der transgenen Pflanze unterdrückt wird, sind für das erfindungsgemäße Verfahren nicht ausreichend spezifisch und nicht geeignet, da sie in den Stoffwechsel der Pflanze eingreifen können.

Wenn entsprechend von Antisense-Sequenzen gesprochen wird, dann sind erfindungsgemäß diejenigen Sequenzen gemeint, die dem kodogenen DNA-Strang der Pilzgene entsprechen. Sie sind bevorzugt zu mindestens 50% komplementär, bevorzugt zu mindestens 60% komplementär, besonders bevorzugt zu mindestens 70% komplementär, weiterhin besonders bevorzugt zu mindestens 80% komplementär, insbesondere bevorzugt zu mindestens 90% komplementär und am meisten bevorzugt zu mindestens 95%, 98% und/oder 99% komplementär sein. Wie oben ausgeführt, ist es ausreichend, wenn die Antisense-Sequenzen in der Lage sind, spezifisch mit der mRNA des jeweiligen interessierenden Pilzgens, aber nicht mit der endogenen mRNA der transgenen Pflanze, zu hybridisieren. Die Hybridisierung einer Antisense-Sequenz mit einer mRNA-Sequenz des Pilzes findet typischerweise *in vivo* unter zellulären Bedingungen oder *in vitro* statt.

In einer bevorzugten Ausführungsform des obigen Verfahrens, umfasst das rekombinante Nukleinsäuremolekül einen in Pflanzen funktionsfähigen Promotor, und operativ damit verbunden die mindestens eine Nukleinsäuresequenz.

Bei den erfindungsgemäßen Promotoren kann es sich um konstitutive, aber auch induzierbare oder gewebe- bzw. entwicklungsspezifische Promotoren handeln. Im allgemeinen können für die Ausübung der Erfindung Promotoren, die für die Expression von Genen in Pflanzen geeignet sind aus unterschiedlichen Quellen wie z. B. aus Pflanzen oder Pflanzenviren erhalten werden. Die Auswahl des Promotors wie auch anderer regulatorischer Sequenzen bestimmen dabei das räumliche und zeitliche Expressionsmuster in transgenen Pflanzen.

Die erfindungsgemäße mindestens eine Nukleinsäuresequenz kann in Antisense- oder Sense-Orientierung vorliegen (identisch oder komplementär gegenüber der Nukleinsäuresequenz des Pilzes).

In einer bevorzugten Ausführungsform der Erfindung liegt die mindestens eine Nukleinsäuresequenz in Antisense-Orientierung vor, so dass bei der Transkription dieser Sequenz in Pflanzenzellen ein RNA-Molekül entsteht, dessen Sequenz komplementär zu der Nukleinsäure des Pilzes ist. Durch Hybridisierung der Antisense-Sequenz mit der Nukleinsäuresequenz des Pilzes *in vivo* kann daher die Expression der Nukleinsäuresequenz des Pilzes in Pflanzenzellen unterdrückt werden, wodurch die Pflanze resistent gegenüber dem Pilz wird.

In einer weiteren bevorzugten Ausführungsform der Erfindung liegt die mindestens eine Nukleinsäuresequenz in Sense-Orientierung vor, so dass bei der Transkription dieser Sequenz in Pflanzenzellen ein RNA-Molekül entsteht, dessen Sequenz identisch zu der Nukleinsäure des Pilzes ist. Der Fachmann weiss, dass es in Pflanzen bei der Expression solcher Sense-Sequenzen zur Bildung von antisense-RNAs (asRNAs) kommen kann, die dann sowohl zum silencing des Transgens, also der erfindungsgemäßen mindestens einen Nukleinsäuresequenz als auch des endogenen, korrespondierenden Gens führen können (Ko-Suppression). Durch Ko-Suppression der Nukleinsäuresequenz des Pilzes *in vivo* kann daher die Expression der Nukleinsäuresequenz des Pilzes in Pflanzenzellen unterdrückt werden, wodurch die Pflanze resistent gegenüber dem Pilz wird.

Die erfindungsgemäße Nukleinsäuresequenz kann aus einer natürlich vorkommenden Nukleinsäuresequenz oder einer synthetisch hergestellten, durch Sequenzvergleich erstellten oder rekombinant hergestellten Nukleinsäuresequenz bestehen bzw. davon abgeleitet sein.

In einer bevorzugten Ausführungsform umfasst die erfindungsgemäße mindestens eine Nukleinsäuresequenz mindestens 50bp, mindestens 100bp, mindestens 150bp, mindestens 200bp, mindestens 250bp oder mindestens 300bp.

Bei einer anderen Ausführungsform des erfindungsgemäßen Verfahrens werden zur Übertragung der Nukleinsäuren auf die Pflanzenzellen Vektoren verwendet, die in 5'-3'-Orientierung einen in Pflanzen funktionsfähigen Promotor, operativ damit verbunden eine DNA-Sequenz, die für ein Ribozym kodiert, das spezifisch die Nukleinsäuresequenz eines Pilzes erkennt, sowie eine Terminationssequenz umfassen. Dem Fachmann ist bekannt, wie Ribozyme, die eine gegen eine bestimmte Nukleinsäuresequenz, z.B. eine mRNA gerichtete Endonuklease-Aktivität besitzen, hergestellt werden können. Im Rahmen dieser Erfindung sind mit dem Begriff "Ribozym" auch solche RNA-Sequenzen gemeint, die neben dem eigentlichen Ribozym noch Führungssequenzen umfassen, die zu der Nukleinsäuresequenz des Pilzes oder Teilen davon komplementär sind und so das mRNA-spezifische Ribozym noch gezielter zum mRNA-Substrat des Ribozyms führen.

In einer weiteren bevorzugten Ausführungsform des obigen Verfahrens umfasst das rekombinante Nukleinsäuremolekül einen in Pflanzen funktionsfähigen Promotor, operativ damit verbunden die mindestens eine Nukleinsäuresequenz, eine weitere Nukleinsäuresequenz, die für Ribonuklease P kodiert, und eine Terminationssequenz. Bei der Transkription solcher Vektoren entstehen in der Zelle RNA-Moleküle, die über eine Führungssequenz (die Antisense-Sequenz) verfügen, welche die RNAse P zur mRNA des Pilzgens führt, wodurch die Spaltung der mRNA durch RNAse P bewirkt wird (US-Patent Nr. 5,168,053). Vorzugsweise umfasst die Führungssequenz 10 bis 15 Nukleotide, die zur mRNA eines Pilzgens komplementär sind, und eine 3'-NCCA Nukleotidsequenz, wobei N vorzugsweise ein Purin ist. Die Transkripte der externen Führungssequenz binden an die Ziel-mRNA über die Ausbildung von Basenpaaren, was die Spaltung der mRNA durch die RNAse P am Nukleotid 5' von der gepaarten Region ermöglicht. Eine solche gespaltene mRNA kann nicht in ein funktionsfähiges Protein translatiert werden.

Dem Fachmann sind weiterhin vielfältige Methoden zur Suppression bzw. Unterbindung der Expression eines endogenen Genes bekannt, vermittelt durch kurze doppelsträngige RNA-Moleküle, sog. small interfering RNAs, oder siRNAs.

Dabei vermittelt das doppelsträngige RNA-Molekül die spezifische Degradation der korrespondierenden Nukleinsäuresequenz, also der Nukleinsäuresequenz aus der die doppelsträngige RNA-Sequenz abgeleitet wurde. Durch enzymatische Spaltung, z.B. durch den Dicer-Enzymkomplex entstehen aus doppelsträngigen RNA-Substraten RNA-Fragmente von 19-25 Nukleotiden Länge, die sog. siRNAs. Solche doppelsträngigen RNA-Moleküle (Dicer-Substrate) müssen mindestens eine Länge von 25bp aufweisen, der Fachmann weiss jedoch, dass auch wesentlich längere doppelsträngige RNA-Moleküle als Substrate geeignet sind. Aufgrund der sehr hohen Sequenzidentität der beiden siRNAs liegen die siRNAs üblicherweise als RNA-Doppelstrang vor. Die siRNAs können die Genexpression auf verschiedene Arten und Weisen hemmen bzw. unterbinden:
a) Transkription (transcriptional gene silencing oder TGS)
b) Degradation der mRNA (post transcriptional gene silencing oder PTGS
c) Translation

Dieses Verfahren der Suppression der Expression einer endogenen Nukleinsäuresequenz durch eine sequenzspezifische doppelsträngige RNA ist dem Fachmann als RNA-interference oder RNAi bekannt (Zamore et al. (2000) Cell 101:25-33; Tang et al. (2003) Genes Dev. 17:49-63, Smith et al. (2000) Nature 407: 319-320).

Die erfindungsgemäße Verwendung von RNAi-Konstrukten beruht auf den oben angeführten Mechanismen der Hemmung der Genexpression einer Nukleinsäuresequenz eines Pilzes. Dadurch kann das/die entsprechenden Polypeptide nicht gebildet werden. Wie oben ausführlich erläutert wird erfindungsgemäß die Expression essenzieller Nukleinsäuresequenzen des Pilzes unterbunden. Diese Nukleinsäuresequenzen sind zwischen verschiedenen Pilzen hochkonserviert. Dementsprechend ist dem Fachmann klar, dass die sequenzspezifische Hemmung der Expression einer solchen Nukleinsäuresequenz in einer erfindungsgemäßen transgenen Pflanze sich auf alle Pilze erstreckt, die über einen homologen Abschnitt in der korrespondierenden Nukleinsäuresequenz verfügen.

Der Fachmann weiss, dass verschiedene Strategien gewählt werden können, um doppelsträngige RNA als Dicer-Substrat in der Zelle verfügbar zu machen und so einen spezifischen RNAi-Effekt auszulösen.

In einer bevorzugten Ausführungsform des erfindungsgemäßen Verfahrens umfasst das rekombinante Nukleinsäuremolekül ein RNAi-Konstrukt.

In einer bevorzugten Ausführungsform des obigen Verfahrens umfasst das rekombinante Nukleinsäuremolekül einen in Pflanzen funktionsfähigen Promotor, operativ damit verbunden die mindestens eine Nukleinsäuresequenz, wobei die Sequenz über revers-komplementäre Bereiche verfügt, und eine Terminationssequenz. Dem Fachmannn sind entsprechende Kontrukte bekannt, in denen eine Nukleinsäuresequenz über revers komplementäre Bereiche verfügt, so dass es nach der Transkription eines solchen Konstruktes und Selbsthybridisierung innerhalb der Nukleinsäuresequenz mit den genannten revers komplementären Bereichen wiederum zur Bildung von doppelsträngiger RNA kommt, die ebenso ein Substrat für beispielsweise den Dicer-Enzymkomplex darstellt. Dementsprechend werden auch hier siRNA-Moleküle gebildet, die zur Degradation einer korrespondierenden Nukleinsäure eines Pilzes führen. Die oben beschriebenen revers komplementären Nukleinsäuresequenzen werden auch als *inverted repeats* bezeichnet.

In einer weiteren bevorzugten Ausführungsform umfasst das rekombinante Nukleinsäuremolekül einen in Pflanzen funktionsfähigen Promotor, operativ damit verbunden die mindestens eine Nukleinsäuresequenz, eine "short hairpin"-Struktur-generierende Nukleinsäure und die zur mindestens einen Nukleinsäuresequenz revers komplementäre Nukleinsäuresequenz, sowie eine Terminationssequenz.

Durch Rückfaltung der "short hairpin"-Struktur können die mindestens eine Nukleinsäuresequenz und die dazu revers komplementäre Nukleinsäuresequenz hybridisieren, doppelsträngige RNA ausbilden und das PTGS-System induzieren. Entsprechende Konstrukte und doppelsträngige RNA-Moleküle sind dem Fachmann beispielsweise als "short hairpin"-RNAs bzw. shRNAs bekannt. Typischerweise werden solche Konstrukte von U6-Promotor oder einem CaMV35S-Promoter angetrieben (Tuschl (2002) vide supra; Paul et al. (2002) Nat. Biotechnol. 20: 505-508; Paddison et al. (2002) Genes Dev. 16(8): 948-958; Brummelkamp et al. (2002) Science 296: 550-553).

In einer weiteren bevorzugten Ausführungsform umfasst das rekombinante Nukleinsäuremolekül ein RNAi-Konstrukt, wobei die mindestens eine Nukleinsäuresequenz eine Sequenz ausgewählt aus der Gruppe bestehend aus SEQ ID Nos. 1-16 oder Teile davon umfasst, sowie eine dazu revers komplementäre Sequenz.

In einer weiteren bevorzugten Ausführungsform umfasst das rekombinante Nukleinsäuremolekül ein RNAi-Konstrukt, wobei die mindestens eine Nukleinsäuresequenz zwei Sequenzen ausgewählt aus der Gruppe bestehend aus SEQ ID Nos. 1-16 oder Teile davon umfasst, sowie eine bzw. zwei dazu revers komplementäre Sequenzen.

In einer weiteren bevorzugten Ausführungsform umfasst das rekombinante Nukleinsäuremolekül ein RNAi-Konstrukt, wobei die mindestens eine Nukleinsäuresequenz drei Sequenzen ausgewählt aus der Gruppe bestehend aus SEQ ID Nos. 1-16 oder Teile davon umfasst, sowie eine bzw. mehrere dazu revers komplementäre Sequenzen.

In einer besonders bevorzugten Ausführungsform des erfindungsgemäßen Verfahrens unter Verwendung der RNAi-Methodik umfasst das rekombinante Nukleinsäuremolekül einen in Pflanzen funktionsfähigen Promotor, operativ damit verbunden die mindestens eine Nukleinsäuresequenz, ein Intron umfassend Spleißdonor- und Spleißakzeptorsequenzen, die revers komplementäre Nukleinsäuresequenz zu der erfindungsgemäßen mindestens einen Nukleinsäuresequenz sowie eine Terminationssequenz.

Bei den so genannten Terminationssequenzen handelt es sich um Sequenzen, die sicherstellen, dass die Transkription bzw. die Translation ordnungsgemäß beendet wird. Sollen die übertragenen Nukleinsäuren translatiert werden, handelt es sich bei ihnen typischerweise um Stopcodons und entsprechende regulatorische Sequenzen; sollen die übertragenen Nukleinsäuren nur transkribiert werden, handelt es sich in der Regel um poly-A-Sequenzen.

Werden solche Vektoren stabil auf Pflanzenzellen übertragen, entsteht bei der Transkription dieser Vektoren zunächst eine prä-mRNA, die aus einem ersten Exon, umfassend die erfindungsgemäße mindestens eine Nukleinsäuresequenz, einem Intron und einem zweiten Exon, das die zu der mindestens einen Nukleinsäuresequenz revers komplementäre Nukleinsäuresequenz umfasst, besteht. Da durch den Spleiß-Vorgang das Intron entfernt wird, entsteht ein kontinuierliches RNA-Molekül mit Bereichen, die zueinander komplementär sind und somit ein Substrat für spezifische Enzymkomplexe wie z.B. den Dicer-Enzymkomplex darstellen. Dem Fachmann ist dabei klar, dass die Position der Antisense (3'-5') - und Sense (5'-3')-Sequenzen im Vektor natürlich vertauscht werden kann. Dies gilt selbstverständlich auch für die oben beschriebenen erfindungsgemäßen rekombinanten Nukleinsäuremoleküle, in denen mehrere verschiedene siRNAs enthalten sind. Somit sind z.B. für drei verschiedene siRNAs verschiedene Konfigurationen der Polarität (Sense/Antisense) denkbar, wie z.B.:

| siRNA #1 | siRNA#2 | siRNA#3 | (revers #1 | revers #2 | revers #3) |
|---|---|---|---|---|---|
| 5'-3' | 5'-3' | 5'-3' | (3'-5' | 3'-5' | 3'-5') |
| 5'-3' | 5'-3' | 3'-5' | (3'-5' | 3'-5' | 5'-3') |
| 5'-3' | 3'-5' | 5'-3' | (3'-5' | 5'-3' | 3'-5') |
| 5'-3' | 3'-5' | 3'-5' | (3'-5' | 5'-3' | 5'-3') |
| 3'-5' | 5'-3' | 5'-3' | (5'-3' | 3'-5' | 3'-5') |
| 3'-5' | 5'-3' | 5'-3' | (5'-3' | 5'-3' | 5'-3') |
| 3'-5' | 3'-5' | 5'-3' | (5'-3' | 5'-3' | 3'-5') |
| 3'-5' | 3'-5' | 3'-5' | (5'-3' | 5'-3' | 5'-3') |

Selbstverständlich beinhaltet die Erfindung auch solche rekombinanten Nukleinsäuremoleküle, in denen die 5'-3' Reihenfolge variiert wird, z.B.

| | | |
|---|---|---|
| siRNA#2 | siRNA#1 | siRNA#3 |
| usw. (vgl. unten) | | |

In einer Ausführungsform des erfindungsgemäßen Verfahrens umfasst das rekombinante Nukleinsäuremolekül ein RNAi-Konstrukt, umfassend ein Intron zwischen der mindestens einen Nukleinsäuresequenz und der dazu revers komplementären Sequenz.

In einer Ausführungsform des erfindungsgemäßen Verfahrens umfasst das rekombinante Nukleinsäuremolekül ein RNAi-Konstrukt, umfassend ein Intron zwischen der mindestens einen Nukleinsäuresequenz und der dazu revers komplementären Sequenz, wobei die mindestens eine Nukleinsäuresequenz eine Nukleinsäuresequenz ausgewählt aus der Gruppe bestehend aus SEQ ID Nos. 1-16 oder Teile davon umfasst, sowie die dazu revers komplementäre Sequenz.

In einer besonders bevorzugten Ausführungsform umfasst das rekombinante Nukleinsäuremolekül ein RNAi-Konstrukt umfassend ein Intron zwischen der mindestens einen Nukleinsäuresequenz und der dazu revers komplementären Sequenz, wobei die mindestens eine Nukleinsäuresequenz eine Nukleinsäuresequenz kodierend für den mitochondrialen ADP/ATP-Translokator (AAC) aus einem Pilz oder einen Teil davon umfasst, sowie dazu revers komplementäre Sequenz.

In einer besonders bevorzugten Ausführungsform umfasst das rekombinante Nukleinsäuremolekül ein RNAi-Konstrukt umfassend ein Intron zwischen der mindestens einen Nukleinsäuresequenz und der dazu revers komplementären Sequenz, wobei die mindestens eine Nukleinsäuresequenz eine Nukleinsäuresequenz kodierend für den mitochondrialen mitochondriale Hitzeschockprotein 70 aus einem Pilz oder einen Teil davon umfasst, sowie die dazu revers komplementäre Sequenz.

In einer Ausführungsform des erfindungsgemäßen Verfahrens umfasst das rekombinante Nukleinsäuremolekül ein RNAi-Konstrukt umfassend ein Intron zwischen der mindestens einen Nukleinsäuresequenz und der dazu revers komplementären Sequenz, wobei die mindestens eine Nukleinsäuresequenz zwei Nukleinsäuresequenzen umfasst, sowie die dazu revers komplementären Sequenzen.

In einer Ausführungsform des erfindungsgemäßen Verfahrens umfasst das rekombinante Nukleinsäuremolekül ein RNAi-Konstrukt umfassend ein Intron zwischen der mindestens einen Nukleinsäuresequenz und der dazu revers komplementären Sequenz, wobei die mindestens eine Nukleinsäuresequenz zwei Nukleinsäuresequenzen ausgewählt aus der Gruppe bestehend aus SEQ ID Nos. 1-16 oder Teile davon umfasst, sowie die dazu revers komplementären Sequenzen.

In einer bevorzugten Ausführungsform des erfindungsgemäßen Verfahrens umfasst das rekombinante Nukleinsäuremolekül ein RNAi-Konstrukt, umfassend ein Intron zwischen der mindestens einen Nukleinsäuresequenz und der dazu revers komplementären Sequenz, wobei die mindestens eine Nukleinsäuresequenz zwei in beliebiger 5'-3' Reihenfolge angeordnete Nukleinsäuresequenzen ausgewählt aus der Gruppe bestehend aus Nukleinsäresequenzen kodierend für gamma-Aktin (GA) aus einem Pilz, dem mitochondrialen ADP/ATP-Translokator (AAC) aus einem Pilz, dem mitochondrialen Hitzeschockprotein 70 (Hsp) aus einem Pilz, dem Elongationsfaktor 1 (EF1) aus einem Pilz sowie der Rho GTPase (Rho) aus einem Pilz oder jeweils einen Teil davon umfasst, sowie die dazu revers komplementären Sequenzen.

In einer Ausführungsform des erfindungsgemäßen Verfahrens umfasst das rekombinante Nukleinsäuremolekül ein RNAi-Konstrukt umfassend ein Intron zwischen der mindestens einen Nukleinsäuresequenz und der dazu revers komplementären Sequenz, wobei die mindestens eine Nukleinsäuresequenz drei Nukleinsäuresequenzen umfasst, sowie die dazu revers komplementären Sequenzen.

In einer Ausführungsform des erfindungsgemäßen Verfahrens umfasst das rekombinante Nukleinsäuremolekül ein RNAi-Konstrukt umfassend ein Intron zwischen der mindestens einen Nukleinsäuresequenz und der dazu revers komplementären Sequenz, wobei die mindestens eine Nukleinsäuresequenz drei Nukleinsäuresequenzen ausgewählt aus der Gruppe bestehend aus SEQ ID Nos. 1-16 oder Teilen davon umfasst, sowie die dazu revers komplementären Sequenzen.

In einer bevorzugten Ausführungsform des erfindungsgemäßen Verfahrens umfasst das rekombinante Nukleinsäuremolekül ein RNAi-Konstrukt, umfassend ein Intron zwischen der mindestens einen Nukleinsäuresequenz und der dazu revers komplementären Sequenz, wobei die mindestens eine Nukleinsäuresequenz drei in beliebiger 5'-3' Reihenfolge angeordnete Nukleinsäuresequenzen ausgewählt aus der Gruppe bestehend aus Nukleinsäresequenzen kodierend für gamma-Aktin (GA) aus einem Pilz, dem mitochondrialen ADP/ATP-Translokator (AAC) aus einem Pilz, dem mitochondrialen Hitzeschockprotein 70 (Hsp) aus einem Pilz, dem Elongationsfaktor 1 (EF1) aus einem Pilz sowie der Rho GTPase (Rho) aus einem Pilz oder jeweils einem Teil davon umfasst, sowie die dazu revers komplementären Sequenzen.

In einer besonders bevorzugten Ausführungsform umfasst das rekombinante Nukleinsäuremolekül ein RNAi-Konstrukt umfassend ein Intron zwischen der mindestens einen Nukleinsäuresequenz und der dazu revers komplementären Sequenz, wobei die mindestens eine Nukleinsäuresequenz drei Nukleinsäuresequenzen kodierend für das gamma-Aktin (GA) aus einem Pilz oder einen Teil davon, für den mitochondrialen ADP/ATP-Translokator (AAC) aus einem Pilz oder einen Teil davon sowie für das mitochondriale Hitzeschockprotein 70 (Hsp) aus einem Pilz oder einen Teil davon umfasst, sowie dazu revers komplementäre Sequenzen.

In einer weiteren besonders bevorzugten Ausführungsform umfasst das rekombinante Nukleinsäuremolekül ein RNAi-Konstrukt umfassend ein Intron zwischen der mindestens einen Nukleinsäuresequenz und der dazu revers komplementären Sequenz, wobei die mindestens eine Nukleinsäuresequenz drei Nukleinsäuresequenzen kodierend für das gamma-Aktin (GA) aus einem Pilz oder einen Teil davon, für den mitochondrialen ADP/ATP-Translokator (AAC) aus einem Pilz oder einen Teil davon sowie für den Elongationsfaktor 1 (EF1) aus einem Pilz oder einen Teil davon umfasst, sowie dazu revers komplementäre Sequenzen.

In einer weiteren besonders bevorzugten Ausführungsform umfasst das rekombinante Nukleinsäuremolekül ein RNAi-Konstrukt umfassend ein Intron zwischen der mindestens einen Nukleinsäuresequenz und der dazu revers komplementären Sequenz, wobei die mindestens eine Nukleinsäuresequenz drei Nukleinsäuresequenzen kodierend für das gamma-Aktin (GA) aus einem Pilz oder einen Teil davon, für die Rho GTPase (Rho) aus einem Pilz oder einen Teil davon sowie für den Elongationsfaktor 1 (EF1) aus einem Pilz oder einen Teil davon umfasst, sowie dazu revers komplementäre Sequenzen.

In einer weiteren besonders bevorzugten Ausführungsform umfasst das rekombinante Nukleinsäuremolekül ein RNAi-Konstrukt umfassend ein Intron zwischen der mindestens einen Nukleinsäuresequenz und der dazu revers komplementären Sequenz, wobei die mindestens eine Nukleinsäuresequenz drei Nukleinsäuresequenzen kodierend für das gamma-Aktin (GA) aus einem Pilz oder einen Teil davon, für die Rho GTPase (Rho) aus einem Pilz oder einen Teil davon sowie für das mitochondriale Hitzeschockprotein 70 (Hsp) aus einem Pilz oder einen Teil davon umfasst, sowie dazu revers komplementäre Sequenzen.

In einer weiteren besonders bevorzugten Ausführungsform umfasst das rekombinante Nukleinsäuremolekül ein RNAi-Konstrukt umfassend ein Intron zwischen der mindestens einen Nukleinsäuresequenz und der dazu revers komplementären Sequenz, wobei die mindestens eine Nukleinsäuresequenz drei Nukleinsäuresequenzen kodierend für den mitochondrialen ADP/ATP-Translokator (AAC) aus einem Pilz oder einen Teil davon, für die Rho GTPase (Rho) aus einem Pilz oder einen Teil davon sowie für den Elongationsfaktor 1 (EF1) aus einem Pilz oder einen Teil davon umfasst, sowie dazu revers komplementäre Sequenzen.

Ebenfalls Teil der Erfindung sind selbstverständlich Ausführungsformen in denen die Hemmung bzw. Unterdrückung der Expression verschiedener Pilzgene durch die simultane Verwendung mehrerer RNAi-Konstrukte erreicht wird.

In einer Ausführungsform umfasst das erfindungsgemäßen Verfahren zwei rekombinante Nukleinsäuremoleküle, umfassend RNAi-Konstrukte, umfassend ein Intron zwischen der mindestens einen Nukleinsäuresequenz und der dazu revers komplementären Sequenz, wobei die mindestens eine Nukleinsäuresequenz eine, zwei oder drei Nukleinsäuresequenzen ausgewählt aus der Gruppe bestehend aus SEQ ID Nos. 1-16 oder Teilen davon umfasst, sowie die dazu revers komplementären Sequenzen.

In einer Ausführungsform umfasst das erfindungsgemäßen Verfahren drei rekombinante Nukleinsäuremoleküle, umfassend RNAi-Konstrukte, umfassend ein Intron zwischen der mindestens einen Nukleinsäuresequenz und der dazu revers komplementären Sequenz, wobei die mindestens eine Nukleinsäuresequenz eine, zwei oder drei Nukleinsäuresequenzen ausgewählt aus der Gruppe bestehend aus SEQ ID Nos. 1-16 oder Teilen davon umfasst, sowie die dazu revers komplementären Sequenzen.

In einer weiteren bevorzugten Ausführungsform umfasst die mindestens eine Nukleinsäuresequenz eine Nukleinsäuresequenz von mindestens 19 Nukleotiden, mindestens 20 Nukleotiden, mindestens 21 Nukleotiden, mindestens 22 Nukleotiden, mindestens 23 Nukleotiden, mindestens 24 Nukleotiden sowie mindestens 25 Nukleotiden mit eine Sequenzidentität von mindestens 40%, bevorzugt mindestens 50%, weiter bevorzugt mindestens 60%, ebenfalls bevorzugt mindestens 70% und/oder 75%, besonders bevorzugt mindestens 80%, 82%, 84%, 86% und/oder 88%, insbesondere bevorzugt mindestens 90%, 92% und/oder 94% und am meisten bevorzugt mindestens 95%, 96%, 97%, 98% und/oder 99% gegenüber einer anderen Nukleinsäuresequenz ausgewählt aus der Gruppe bestehend aus SEQ ID Nos. 1-16 oder Teilen davon.

In einer bevorzugten Ausführungsform umfasst die mindestens eine Nukleinsäuresequenz zwei Nukleinsäuresequenzen von jeweils mindestens 19 Nukleotiden, mindestens 20 Nukleotiden, mindestens 21 Nukleotiden, mindestens 22 Nukleotiden, mindestens 23 Nukleotiden, mindestens 24 Nukleotiden sowie mindestens 25 Nukleotiden, mit eine Sequenzidentität von mindestens 40%, bevorzugt mindestens 50%, weiter bevorzugt mindestens 60%, ebenfalls bevorzugt mindestens 70% und/oder 75%, besonders bevorzugt mindestens 80%, 82%, 84%, 86% und/oder 88%, insbesondere bevorzugt mindestens 90%, 92% und/oder 94% und am meisten bevorzugt mindestens 95%, 96%, 97%, 98% und/oder 99% gegenüber einer anderen Nukleinsäuresequenz ausgewählt aus der Gruppe bestehend aus SEQ ID Nos. 1-16 oder Teilen davon.

In einer bevorzugten Ausführungsform umfasst die mindestens eine Nukleinsäuresequenz drei Nukleinsäuresequenzen von jeweils mindestens 19 Nukleotiden, mindestens 20 Nukleotiden, mindestens 21 Nukleotiden, mindestens 22 Nukleotiden, mindestens 23 Nukleotiden, mindestens 24 Nukleotiden sowie mindestens 25 Nukleotiden, mit eine Sequenzidentität von mindestens 40%, bevorzugt mindestens 50%, weiter bevorzugt mindestens 60%, ebenfalls bevorzugt mindestens 70% und/oder 75%, besonders bevorzugt mindestens 80%, 82%, 84%, 86% und/oder 88%, insbesondere bevorzugt mindestens 90%, 92% und/oder 94% und am meisten bevorzugt mindestens 95%, 96%, 97%, 98% und/oder 99% gegenüber einer anderen Nukleinsäuresequenz ausgewählt aus der Gruppe bestehend aus SEQ ID Nos. 1-16 oder Teilen davon.

In einer bevorzugten Ausführungsform umfasst die mindestens eine Nukleinsäuresequenz eine Nukleinsäuresequenz von mindestens 50 Nukleotiden, mindestens 100 Nukleotiden, mindestens 150 Nukleotiden, mindestens 200 Nukleotiden, mindestens 250 Nukleotiden, mindestens 300 Nukleotiden sowie mindestens 350 Nukleotiden mit eine Sequenzidentität von mindestens 40%, bevorzugt mindestens 50%, weiter bevorzugt mindestens 60%, ebenfalls bevorzugt mindestens 70% und/oder 75%, besonders bevorzugt mindestens 80%, 82%, 84%, 86% und/oder 88%, insbesondere bevorzugt mindestens 90%, 92% und/oder 94% und am meisten bevorzugt mindestens 95%, 96%, 97%, 98% und/oder 99% gegenüber einer anderen Nukleinsäuresequenz ausgewählt aus der Gruppe bestehend aus SEQ ID Nos. 1-16 oder Teilen davon.

In einer bevorzugten Ausführungsform umfasst die mindestens eine Nukleinsäuresequenz zwei Nukleinsäuresequenzen von mindestens 50 Nukleotiden, mindestens 100 Nukleotiden, mindestens 150 Nukleotiden, mindestens 200 Nukleotiden, mindestens 250 Nukleotiden, mindestens 300 Nukleotiden sowie mindestens 350 Nukleotiden mit eine Sequenzidentität von mindestens 40%, bevorzugt mindestens 50%, weiter bevorzugt mindestens 60%, ebenfalls bevorzugt mindestens 70% und/oder 75%, besonders bevorzugt mindestens 80%, 82%, 84%, 86% und/oder 88%, insbesondere bevorzugt mindestens 90%, 92% und/oder 94% und am meisten bevorzugt mindestens 95%, 96%, 97%, 98% und/oder 99% gegenüber einer anderen Nukleinsäuresequenz ausgewählt aus der Gruppe bestehend aus SEQ ID Nos. 1-16 oder Teilen davon.

In einer bevorzugten Ausführungsform umfasst die mindestens eine Nukleinsäuresequenz drei Nukleinsäuresequenzen von mindestens 50 Nukleotiden, mindestens 100 Nukleotiden, mindestens 150 Nukleotiden, mindestens 200 Nukleotiden, mindestens 250 Nukleotiden, mindestens 300 Nukleotiden sowie mindestens 350 Nukleotiden mit eine Sequenzidentität von mindestens 40%, bevorzugt mindestens 50%, weiter bevorzugt mindestens 60%, ebenfalls bevorzugt mindestens 70% und/oder 75%, besonders bevorzugt mindestens 80%, 82%, 84%, 86% und/oder 88%, insbesondere bevorzugt mindestens 90%, 92% und/oder 94% und am meisten bevorzugt mindestens 95%, 96%, 97%, 98% und/oder 99% gegenüber einer anderen Nukleinsäuresequenz ausgewählt aus der Gruppe bestehend aus SEQ ID Nos. 1-16 oder Teilen davon.

Der Fachmann weiß, dass beim RNAi bzw. PTGS die zur Ausbildung von doppelsträngigen RNA-Molekülen verwendeten Sense- und Antisense-RNAs verschiedene Größen aufweisen können (Tuschl (2002) Nat. Biotechnol. 20: 446-448).

Ebenso Teil der vorliegenden Erfindung ist das erfindungsgemäße Verfahren in dem zur Unterbindung der Expression einer Nukleinsäure eines Pilzes synthetische doppelsträngige siRNAs verwendet werden, die typischerweise eine Länge von 19-21 Nukleotiden aufweisen. Solche synthetischen siRNAs können beispielweise durch biolistische Transformationsmethoden in die jeweilige Pflanzenzelle bzw. Pflanze eingebracht werden. Solche synthetischen siRNA-Moleküle können in Pflanzen das PTGS-System aktivieren und einen RNAi-Effekt auslösen (Hamilton and Baulcombe (1999) Science 286: 950-2).

Die Auswahl der Zielsequenz für die siRNA-Hemmung sowie das siRNA-Sequenzmotiv können nach den dem Fachmann geläufgen Regeln beispielweise aus Elbashir et al. (2001) Nature 411: 494-8 bestimmt werden. Wenn die Zielsequenz für die siRNA-mediierte Hemmung innerhalb der kodierenden Bereichs des Gens, bzw. innerhalb der mRNA liegt, so weiss der Fachmann beispielsweise dass die Zielsequenz für eine siRNA-Hemmung typischerweise 70 Nukleotide abwärts vom Start-Codon in 5'-3' Richtung liegen kann und 50 Nukleotide aufwärts vom Stop-Codon.

Der Sequenzbereich kann dann auf das Sequenzmotiv AA (N19) abgesucht werden, wobei N jedes Nukleotid sein kann. Das Sequenzmotiv umfasst typischerweise das AA-Dinukleotid gefolgt von 19 Nukleotiden und bevorzugt zwei zusätzlichen Uridin- oder Thymidin-Resten. Im allgemeinen können in der siRNA-Sequenz die Thymidin-Reste durch Uridin-Reste ersetzt werden.

Weiterhin kennt der Fachmann die Regeln, die von Reynolds et al. ((2004) Nat Biotechnol. 22:326-30) aufgestellt wurden:
1. ein Guanin/Cytosin-Gehalt von 30-50%
2. mindestens drei Adenin-oder Uracil-Reste an Positionen 15 bis 19 des sense-Stranges
3. keine intermolekularen "hairpin"-Strukturen
4. ein Adenin-Rest an Position 19 des sense-Stranges
5. ein Adenin-Rest an Position 3 des sense-Stranges
6. ein Uracil-Rest an Position 10 des sense-Stranges
7. kein Guanin- oder Cytosin-Rest an Position 19 des sense-Stranges
8. kein Guanin-Rest an Position 13 des sense-Stranges.

Diese acht Kriterien können nach folgendem Schema gewichtet werden:
(i) 1 Punkt für Kriterien 1, 3, 4, 5 und 6
(ii) 1 Punkt für jeden Adenin- oder Uridin-Rest an Position 15 bis 19, wenigstens 3 korrespondierende Basen (Kriterium 2)
(iii) Nichterfüllen der Kriterien 7-8 resultieren in jeweils -1 Punkt.

Nach Reynolds *et al.,* sollten nur siRNAs in Betracht gezogen werden, die wenigstens 6 Punkte haben. Solche siRNAs, die die oben stehenden Kriterien erfüllen, können mit entsprechenden Suchprogrammen wie z.B. BLAST kontrolliert werden, ob möglichst keine oder nur geringe Homologien zu Nukleinsäuresequenzen der Pflanze existieren.

In einer weiteren bevorzugten Ausführungsform des erfindungsgemäßen Verfahrens wird die mindestens eine Nukleinsäuresequenz ausgewählt aus der Gruppe bestehend aus
(i) Nukleinsäuren, die SEQ ID Nr. 1-16 oder Fragmente davon umfassen, und/oder
(ii) Nukleinsäuren, die unter stringenten Bedingungen mit einem komplementären Strang einer Nukleinsäure von (i) hybridisieren.

Der Begriff "Hybridisierung unter stringenten Bedingungen" bedeutet im Zusammenhang dieser Erfindung, dass die Hybridisierung *in vitro* unter Bedingungen durchgeführt wird, die stringent genug sind, um eine spezifische Hybridisierung zu gewährleisten. Stringente *in vitro*-Hybridisierungsbedingungen sind dem Fachmann bekannt und können der Literatur entnommen werden (z. B. Sambrook und Russell (2001) Molecular Cloning: A Laboratory Manual, 3. Auflage, Cold Spring Harbour Laboratory Press, Cold Spring Harbour, NY). Der Begriff "spezifische Hybridisierung" bezieht sich auf den Umstand, dass ein Molekül unter stringenten Bedingungen präferentiell an eine bestimmte Nukleinsäuresequenz, die Zielsequenz, bindet, wenn diese Teil einer komplexen Mischung von z. B. DNA- oder RNA-Molekülen ist, aber nicht oder zumindest wesentlich reduziert an andere Sequenzen.

Stringente Bedingungen sind von den Umständen abhängig. Längere Sequenzen hybridisieren spezifisch bei höheren Temperaturen. Generell werden stringente Bedingungen so ausgewählt, dass die Hybridisierungstemperatur circa 5°C unter dem Schmelzpunkt (Tₘ) für die spezifische Sequenz bei einer definierten Ionenstärke und einem definierten pH liegt. Die Tₘ ist die Temperatur (bei einem definierten pH-Wert, einer definierten Ionenstärke und einer definierten Nukleinsäurekonzentration), bei der 50% der zu der Zielsequenz komplementären Moleküle zu der Zielsequenz im Gleichgewichtszustand hybridisieren. Typischerweise sind stringente Bedingungen solche, bei denen die Salzkonzentration mindestens ungefähr 0,01 bis 1,0 M Natriumionenkonzentration (oder Konzentration eines anderen Salzes) bei einem pH zwischen 7,0 und 8,3 und die Temperatur mindestens 30°C für kurze Moleküle (d.h. z. B. 10 bis 50 Nukleotide) beträgt. Zusätzlich können stringente Bedingungen die Zugabe von Stoffen wie z. B. Formamid, das die Hybride destabilisiert, umfassen. Bei einer Hybridisierung unter stringenten Bedingungen, wie hier verwendet, bleiben gewöhnlich Nukleotidsequenzen, die mindestens 60% homolog zueinander sind, aneinander hybridisiert. Vorzugsweise sind die stringenten Bedingungen derart gewählt, dass Sequenzen, die mindestens etwa 65%, bevorzugt mindestens etwa 70% und besonders bevorzugt mindestens etwa 75% oder stärker zueinander homolog sind, gewöhnlich aneinander hybridisiert bleiben. Ein bevorzugtes, nicht-einschränkendes Beispiel für stringente Hybridisierungsbedingungen sind Hybridisierungen in 6 x Natriumchlorid/Natriumcitrat (SSC) bei etwa 45°C, gefolgt von einem oder mehreren Waschschritten in 0,2 x SSC, 0,1% SDS bei 50 bis 65°C. Die Temperatur schwankt beispielsweise unter Standardhybridisierungsbedingungen je nach dem Typ der Nukleinsäure zwischen 42°C und 58°C in wässrigem Puffer mit einer Konzentration von 0,1 bis 5 x SSC (pH 7,2).

Falls organisches Lösungsmittel, z. B. 50% Formamid, im oben genannten Puffer vorliegt, liegt die Temperatur unter Standardbedingungen bei etwa 42°C. Vorzugsweise sind die Hybridisierungsbedingungen für DNA:DNA-Hybride zum Beispiel 0,1 x SSC und 20°C bis 45°C, vorzugsweise 30°C bis 45°C. Vorzugsweise sind die Hybridisierungsbedingungen für DNA:RNA-Hybride zum Beispiel 0,1 x SSC und 30°C bis 55°C, vorzugsweise zwischen 45°C bis 55°C. Die vorstehend genannten Hybridisierungstemperaturen sind beispielsweise für eine Nukleinsäure mit etwa 100 Basenpaaren Länge und einem G/C-Gehalt von 50% in Abwesenheit von Formamid bestimmt. Der Fachmann weiß, wie die erforderlichen Hybridisierungsbedingungen anhand von Lehrbüchern, wie den vorstehend erwähnten oder den folgenden Lehrbüchern Current Protocols in Molecular Biology, John Wiley & Sons, N.Y. (1989), Hames und Higgins (Hrsgb.) 1985, Nucleic Acids Hybridization: A Practical Approach, IRL Press at Oxford University Press, Oxford; Brown (Hrsgb.) 1991, Essential Molecular Biology: A Practical Approach, IRL Press at Oxford University Press, Oxford, bestimmt werden können.

Typische Hybridisierungs- und Waschpuffer haben z. B. folgende Zusammensetzung:

### Prähybridisierungslösung:

| | |
|---|---|
| | 0,5 % SDS |
| | 5x SSC |
| | 50 mM NaPO₄, pH 6,8 |
| | 0, 1 % Na-Pyrophosphat |
| | 5x Denhardt's Lösung |
| | 100 µg/ml Lachssperma-DNA |
| | |
| *Hybridisierungslösung:* | Prähybridisierungslsg. |
| | 1x10⁶ cpm/ml Sonde (5-10 min 95°C) |
| | |
| *20x SSC:* | 3 M NaCl |
| | 0,3 M Natriumcitrat |
| | ad pH 7 mit HCl |
| *50x Denhardt's Reagenz:* | 5 g Ficoll |
| | 5 g Polyvinylpyrrolidon |
| | 5 g Rinderserumalbumin |
| | ad 500 ml A. dest. |

Eine typische Verfahrensweise für die Hybridisierung sieht folgendermaßen aus:

| | | | |
|---|---|---|---|
| *Optional:* | Blot 30 min in 1 x SSC/ 0,1% SDS bei 65°C waschen | | |
| *Prähybridisierung:* | mindestens 2 h bei 50-55°C | | |
| *Hybridisierung:* | über Nacht bei 55-60°C | | |
| *Waschen:* | 05 min | 2x SSC/ 0,1% SDS | Hybridisierungstemp. |
| 30 min | 2x SSC/0,1% SDS | Hybridisierungstemp. | |
| 30 min | 1x SSC/0,1% SDS | Hybridisierungstemp. | |
| 45 min | 0,2x SSC/ 0,1% SDS | 65°C | |
| 5 min | 0,1x SSC | Raumtemperatur | |

Der Fachmann weiß, dass die genannten Lösungen und das dargestellte Protokoll je nach Anwendung modifiziert werden können oder müssen.

Um RNAi-Konstrukte herzustellen, können die entsprechenden Sense- bzw. Antisense-Nukleinsäuresequenzen beispielsweise durch Restriktionsverdau und anschließende Ligation in einen geeigneten Vektor inseriert werden.

Alternativ dazu können die entsprechenden Sense- bzw. Antisense-Nukleinsäuresequenzen durch homologe Rekombination, wie sie etwa mit dem GATEWAY^{®}-System (Invitrogen) oder dem BD Creator™ System (BD Biosciences Clontech Co.) möglich ist, in den Vektor eingeführt werden.

In einer bevorzugten Ausführungsform werden die entsprechenden Sense- bzw. Antisense-Nukleinsäuresequenzen durch homologe Rekombination mit dem GATEWAY^{®}-System in einen Vektor eingerührt.

In einer weiteren bevorzugten Ausführungsform des obigen Verfahrens ist der Promotor ein konstitutiver Promotor, ein induzierbarer Promotor oder ein gewebespezifischer Promotor.

Neben konstitutiven Promotoren wie beispielsweise dem Ubiquitin Promotor (Binet et al. (1991) Plant Science 79:87-94) und dem Actin Promotor (McElroy et al. (1990) Plant Cell 2:163-171) kommen als gewebespezifische Promotoren die Promotoren der Phosphoenolpyruvat-Carboxylase aus Mais (Hudspeth et al. (1989) Plant Mol. Biol. 12:579) oder der Fructose-1,6-bisphosphatase aus Kartoffel (WO 98/18940), die blattspezifische Expression vermitteln, in Frage. Es können auch Verwundungs-, Licht- oder Pathogen-induzierte sowie andere entwicklungsabhängige Promotoren oder Kontrollsequenzen verwendet werden (Xu et al. (1993) Plant Mol. Biol. 22:573-588; Logemann et al. (1989) Plant Cell 1:151-158; Stockhaus et al. (1989) Plant Cell 1:805-813; Puente et al. (1996) EMBO J. 15:3732-3734; Gough et al. (1995) Mol. Gen. Genet. 247:323-337). Eine Zusammenfassung von verwendbaren Kontrollsequenzen findet sich z. B. in Zuo et al. (2000) Curr. Opin. Biotech. 11:146-151.

Geeignete Promotoren umfassen auch Promotoren, die eine Expression lediglich in Geweben garantieren, wie z. B. Epidermis-spezifische Promotoren wie der GSTA1-Promoter (Altpeter et al. (2005) Plant Mol Biol. 57:271-83) oder Promotoren photosynthetisch aktiver Gewebe wie der ST-LS1-Promotor (Stockhaus et al. (1987) Proc. Natl. Acad. Sci. USA 84:7943-7947; Stockhaus et al. (1989) EMBO J. 8:2445-2451). Ebenfalls verwendet werden können Promotoren, die während der Pflanzentransformation, der Pflanzenregeneration oder bestimmten Stadien dieser Prozesse aktiv sind, wie z. B. zellteilungsspezifische Promotoren wie der Histon H3-Promotor (Kapros et al. (1993) In Vitro Cell Dev. Biol. Plant 29:27-32) oder das chemisch induzierbare Tet-Repressor-System (Gatz et al. (1991) Mol. Gen. Genet. 227:229-237). Weitere geeignete Promotoren können der Literatur, z. B. Ward (1993, Plant Mol. Biol. 22:361-366), entnommen werden. Gleiches gilt für induzierbare und zell- bzw. gewebespezifische Promotoren, wie Meristemspezifische Promotoren, die ebenfalls in der Literatur beschrieben worden sind und im Rahmen der Erfindung ebenfalls geeignet sind.

Besonders geeignete Promotoren für die Durchführung der vorliegenden Erfindung sind pilzinduzierbare Promotoren und hier insbesondere solche, die durch Schadpilze und nicht durch Nutzpilze(z.B. Mykkorhiza im Boden) induziert werden. Solche Promotoren, wie z. B. der GER4-Promoter (WO2006/128882) ermöglichen eine hochspezifische Erzeugung einer Breitband-Resistenz in transgenen Pflanzen wobei unerwünschte Nebeneffekte deutlich reduziert bzw. eliminiert werden.

Weitere pilzinduzierbare Promotoren umfassen Promotoren wie den GAFP-2 Promotor (Sa et al. (2003) Plant Cell Rep. 22:79-84), der z. B. durch den Pilz *Trichoderma viride* induziert wird, oder den PAL-Promotor, der durch Inokulation mit *Pyricularia oryzae* induziert wird (Wang et al. (2004) Plant Cell Rep. 22:513-518).

Weiterhin bevorzugte Promotoren sind solche, die insbesondere in Früchten aktiv sind. Hierzu zählen beispielsweise der Promotor eines Polygalacturonase-Gens, z. B. aus Tomate, der während der Reife von Tomatenfrüchten Expression vermittelt (Nicholass et al. (1995) Plant Mol. Biol. 28:423-435), der Promotor einer ACC-Oxidase, z. B. aus Apfel, der in transgenen Tomaten Reife- und Fruchtspezifität vermittelt (Atkinson et al. (1998) Plant Mol. Biol. 38:449-460), oder der 2A11-Promotor aus Tomate (van Haaren et al. (1991) Plant Mol. Biol. 17:615-630). Darüber hinaus ist der Durchschnittsfachmann in der Lage, mittels Routinemethoden weitere geeignete Promotoren zu isolieren.

Der Fachmann weiß, dass die Verwendung von induzierbaren Promotoren die Herstellung von Pflanzen und Pflanzenzellen erlaubt, die die erfindungsgemäßen Sequenzen nur transient exprimieren und damit transient silencen. Eine solche transiente Expression erlaubt die Herstellung von Pflanzen, die nur eine transient erhöhte Pilzresistenz zeigen. Solch eine transient erhöhte Resistenz kann z. B. dann wünschenswert sein, wenn die Gefahr einer Pilzkontamination droht und die Pflanzen deswegen nur für einen bestimmten Zeitraum resistent gegen den Pilz sein müssen. Weitere Situationen, in denen eine transiente Resistenz wünschenswert ist, sind dem Fachmann bekannt. Dem Fachmann ist darüber hinaus auch bekannt, dass er durch die Verwendung nicht stabil in Pflanzenzellen replizierender Vektoren, die die entsprechenden Sequenzen zum Silencing von Pilzgenen tragen, eine transiente Expression und damit ein transientes Silencing und eine transiente Resistenz erzielen kann.

In einer bevorzugten Ausführungsform des erfindungsgemäßen Verfahrens wird als konstitutiver Promoter der Ubiquitin-Promoter verwendet.

In einer weiteren bevorzugten Ausführungsform des erfindungsgemäßen Verfahrens wird als gewebespezifischer Promoter der Epidermis-spezifische Promoter GSTA1 verwendet.

In einer besonders bevorzugten Ausführungsform des erfindungsgemäßen Verfahrens wird als pathogen-induzierbarer Promoter der GER4-Promoter verwendet.

Die Vektoren, die zum Silencing von Nukleinsäuren von Pilzen verwendet werden, umfassen neben der zu übertragenden Nukleinsäuresequenz weitere regulatorische Elemente. Welche konkreten regulatorischen Elemente diese Vektoren enthalten müssen, hängt dabei jeweils von der Methode ab, die mit diesen Vektoren durchgeführt werden soll. Über welche regulatorischen Elemente und auch anderen Elemente diese Vektoren verfügen müssen, ist dem Fachmann, der mit den verschiedenen oben erwähnten Verfahren zum Herstellen von transgenen Pflanzen, in denen die Expression eines Proteins unterbunden wird, vertraut ist, bekannt.

Typischerweise handelt es sich bei den regulatorischen Elementen, die die Vektoren enthalten, um solche, die die Transkription und, falls erwünscht, Translation in der Pflanzenzelle gewährleisten.

Der Begriff "operativ verbunden" bezogen auf Nukleinsäuresequenzen bzw. DNA-Abschnitte in Vektoren bezieht sich darauf, dass die Nukleinsäuresequenzen so mit den Vektoren verbunden ist, dass die Sequenz sich unter der transkriptionellen und/oder translationalen Kontrolle eines zur Expression in Pflanzen geeigneten Vektors befindet.

Die erfindungsgemäßen Vektoren können als regulatorische Elemente auch noch z. B. Enhancer-Elemente umfassen, ferner können sie Resistenzgene, Replikationssignale und weitere DNA-Bereiche enthalten, die eine Propagation der Vektoren in Bakterien wie z. B. *E.co*/*i* ermöglichen. Die regulatorischen Elemente umfassen auch Sequenzen, die eine Stabilisierung der Vektoren in den Wirtszellen bewirken. Insbesondere umfassen solche regulatorischen Elemente Sequenzen, die eine stabile Integration des Vektors in das Wirtsgenom der Pflanze oder eine autonome Replikation des Vektors in den Pflanzenzellen ermöglichen. Solche regulatorischen Elemente sind dem Fachmann bekannt.

Der Fachmann weiß ebenfalls, dass verschiedene Vektoren für das RNAi-Verfahren eingesetzt werden können. Solche Vektoren können z. B. so gebaut sein, dass die Sense- und Antisense-Sequenzen jeweils von einem U6-Promotor ausgehend transkribiert werden, in der Zelle hybridisieren und das PTGS-System induzieren (Tuschl (2002) Nat. Biotechnol. 20: 446-448; Miyagishi et al. (2002) Nat. Biotechnol. 20: 497-500; Lee et al. (2002) Nat. Biotechnol. 20: 500-505). Eine Vielzahl von RNAi-kompatiblen Vektorn sind kommerziell erhältlich.

In einer besonders bevorzugten Ausführungsform wird der pIPKb007-Vektor verwendet.

In einer weiteren besonderen Ausführungsform wird der pIPKb010-Vektor verwendet.

In einer weiteren besonderen Ausführungsform wird der p6UGER4deltaSwaIntronRNAi-Vektor verwendet.

Für den Fachmann ist es naheliegend, dass die verschiedensten Pflanzen durch Pilze befallen werden können.

In einer bevorzugten Ausführungsform des Verfahrens handelt es sich um ein Verfahren zur Erzeugung einer Breitband-Resistenz gegenüber Pilzen in monokotylen Pflanzen.

Beispiele für monokotyle Pflanzen sind Pflanzen, die zu den Gattungen Avena (Hafer), Triticum (Weizen), Secale (Roggen), Hordeum (Gerste), Oryza (Reis), Panicum, Pennisetum, Setaria, Sorghum (Hirse), Zea (Mais) und dergleichen gehören.

In einer besonders bevorzugten Ausführungsform des Verfahrens handelt es sich bei um Getreidepflanzen, insbesondere um Weizen oder Gerste.

In einer weiteren besonders bevorzugten Ausführungsform des Verfahrens handelt es sich bei der Pflanze um Gerste.

In einer weiteren bevorzugten Ausführungsform des Verfahrens handelt es sich um ein Verfahren zur Erezugung einer Breitband-Resistenz gegenüber Pilzen in dikotylen Pflanzen.

Dikotyle Pflanzen bzw. Nutzpflanzen umfassen unter anderem Baumwolle, Leguminosen wie Hülsenfrüchte und insbesondere Alfalfa, Sojabohne, Raps, Canola, Tomate, Zuckerrübe, Kartoffel, Sonnenblume, Zierpflanzen sowie Bäume. Weitere Nutzpflanzen können Obst (insbesondere Äpfel, Birnen, Kirschen, Weintrauben, Citrus, Ananas und Bananen), Ölpalmen, Tee-, Kakao- und Kaffeesträucher, Tabak, Sisal sowie bei Heilpflanzen Rauwolfia und Digitalis umfassen. Besonders bevorzugt sind die Getreide Weizen, Roggen, Hafer, Gerste, Reis, Mais und Hirse, sowie die dikotylen Pflanzen Zuckerrübe, Raps, Soja, Tomate, Kartoffel und Tabak. Weitere Nutzpflanzen können dem US-Patent Nr. 6,137,030 entnommen werden.

Bevorzugte Pflanzen sind Tagetes, Sonnenblume, Arabidopsis, Tabak, Roter Pfeffer, Soja, Tomate, Aubergine, Paprika, Möhre, Kartoffel, Mais, Salate und Kohlarten, Getreide, Alfalfa, Hafer, Gerste, Roggen, Weizen, Triticale, Hirse, Reis, Luzerne, Flachs, Baumwolle, Hanf, Brassicacaeen wie beispielsweise Raps oder Canola, Zuckerrübe, Zuckerrohr, Nuss- und Weinspezies oder Holzgewächse wie beispielsweise Espe oder Eibe.

In einer besonders bevorzugten Ausführungsform des Verfahrens handelt es sich um ein Verfahren zur Erzeugung einer Breitband-Resistenz gegenüber Pilzen mittels eines RNAi-Verfahrens zur Unterbindung der Expresssion einer Nukleinsäuresequenz eines Pilzes, wobei die entsprechenden transgene Pflanze resistent mindestens gegenüber diesem Pilz ist.

In einer besonders bevorzugten Ausführungsform handelt es sich um ein Verfahren zur Erzeugung einer Breitband-Resistenz gegenüber Pilzen mittels eines RNAi-Verfahrens zur Unterbindung der Expresssion einer Nukleinsäuresequenz eines Pilzes, wobei die entsprechenden transgene Pflanze resistent mindestens gegenüber einem anderen Pilz ist.

In einer besonders bevorzugten Ausführungsform des erfindungsgemäßen Verfahrens wird eine Breitband-Resistenz gegenüber Schadpilzen erzeugt.

Besonders bevorzugt bewirkt die Suppression der Expression einer Nukleinsäuresequenz eines Pilzes eine Breitband-Resistenz gegen
- Plasmodiophoromycota wie *Plasmodiophora brassicae* (Kohlhernie, clubroot of crucifers), *Spongospora subterranea* (powdery scab of potato tubers), *Polymyxa graminis* (root disease of cereals and grasses);
- Oomycota wie *Bremia lactucae* (Falscher Mehltau an Salat), *Peronospora* (Falscher Mehltau) bei snapdragon (*P. antirrhini*), Zwiebel (*P. destructor*), Spinat (*P. effusa*), Sojabohne (*P. manchurica*), Tabak ("blue mold" = Blauschimmel; *P. tabacina*) Alfalfa und Klee (*P. trifolium*), *Pseudoperonospora humuli* (Falscher Mehltau an Hopfen), *Plasmopara* (Falscher Mehltau bei Trauben) (*P. viticola*) und Sonnenblume (*P. halstedii*), *Sclerophtohra macrospora* (Falscher Mehltau bei Cerealien und Gäsern), *Pythium* (seed rot, seedling damping-off, and root rot and all types of plants, z.B. Wurzelbrand an Beta-Rübe durch *P. debaryanum*), *Phytophthora infestans* (Kraut- und Knollenfäule bei Kartoffel, Braunfäule bei Tomate etc.), *Albugo spec.* (white rust on cruciferous plants);
- Ascomycota wie *Microdochium nivale* (Schneeschimmel an Roggen und Weizen), *Fusarium graminearum, Fusarium culmorum* (Ährenfäule v.a. bei Weizen), *Fusarium oxysporum* (Fusarium-Welke an Tomate), *Blumeria graminis* (Echter Mehltau an Gerste (*f.sp. hordei*) und Weizen (*f.sp. tritici*)), *Erysiphe pisi* (Erbsenmehltau), *Nectria galligena* (Obstbaumkrebs), *Unicnula necator* (Echter Mehltau der Weinrebe), *Pseudopeziza tracheiphila* (Roter Brenner der Weinrebe), *Claviceps purpurea* (Mutterkom an z.B. Roggen und Gräsern), *Gaeumannomyces graminis* (Schwarzbeinigkeit an Weizen, Roggen u.a. Gräsern), *Magnaporthe grisea* (rice blast disease), *Pyrenophora graminea* (Streifenkrankheit an Gerste), *Pyrenophora teres* (Netzfleckenkrankheit an Gerste), *Pyrenophora tritici-repentis* (Blattfleckenkrankheit (Blattdürre) an Weizen), *Venturia inaequalis* (Apfelschorf), *Sclerotinia sclerotium* (Weißstengeligkeit, Rapskrebs), *Pseudopeziza medicaginis* (Klappenschorf an Luzerne, Weiß- und Rotklee);
- Basidiomyceten wie *Typhula incarnata* (Typhula-Fäule an Gerste, Roggen, Weizen), *Ustilago maydis* (Beulenbrand an Mais), *Ustilago nuda* (Flugbrand an Gerste), *Ustilago tritici* (Flugbrand an Weizen, Dinkel), *Ustilago avenae* (Flugbrand an Hafer), *Rhizoctonia solani* (Wurzeltöter an Kartoffeln), *Sphacelotheca spp.* (head smut of sorghum), *Melampsora lini* (rust of flax), *Puccinia graminis* (Schwarzrost an Weizen, Gerste, Roggen, Hafer), *Puccinia recondita* (Braunrost an Weizen), *Puccinia dispersa* (Braunrost an Roggen), *Puccinia hordei* (Braunrost an Gerste), *Puccinia coronata* (Kronenrost an Hafer), *Puccinia striiformis* (Gelbrost an Weizen, Gerste, Roggen sowie zahlreichen Gräsern), *Uromyces appendiculatus* (Bohnenrost), *Sclerotium rolfsii* (root and stem rots of many plants);
- Deuteromyceten (Fungi imperfecti) wie *Septoria nodorum* (Spelzenbräune) an Weizen (*Septoria tritici*), *Pseudocercosporella herpotrichoides* (Halmbruchkrankheit an Weizen, Gerste, Roggen), *Rynchosporium secalis* (Blattfleckenkrankheit an Roggen und Gerste), *Alternaria solani* (Dürrfleckenkrankheit an Kartoffel, Tomate), *Phoma betae* (Wurzelbrand an Beta-Rübe), *Cercospora beticola* (Cercospora-Blattfleckenkrankheit an Beta-Rübe), *Alternaria brassicae* (Rapsschwärze an Raps, Kohl u.a. Kreuzblütlern), *Verticillium dahliae* (Rapswelke und -stengelfäule), *Colletotrichum lindemuthianum* (Brennfleckenkrankheit an Bohne), *Phoma lingam -* Umfallkrankheit (Schwarzbeinigkeit an Kohl; Wurzelhals- oder Stengelfäule an Raps), *Botrytis cinerea* (Grauschimmel an Weinrebe, Erdbeere, Tomate, Hopfen etc.).

In einer besonders bevorzugten Ausführungsform des erfindungsgemäßen Verfahrens wird eine Breitband-Resistenz in einer transgenen monokotyledonen Pflanze gegenüber *Fusarium graminearum, Fusarium culmorum, Septoria tritici, Puccinia recondita, Puccinia striiformis, Puccinia triticina, Puccinia hordei, Blumeria graminis ff.ssp., Rhynchosporium secalis, Bipolaris sorokiniana, Magnaporte oryzae* und *Pyrenophora teres* erzeugt.

In einer weiteren besonders bevorzugten Ausführungsform des erfindungsgemäßen Verfahrens wird eine Breitband-Resistenz in einer transgenen dikotyledonen Pflanze gegenüber *Phytophtora infestans, Uncinula necator, Plasmopora viticola, Uromyces spec., Phakopsora pachyrhizi* und *Erysiphe sp.* erzeugt.

In einer bevorzugten Ausführungsform zur Erzeugung einer Breitband-Resistenz gegenüber Pilzen mittels eines RNAi-Verfahrens gerichtet gegen Nukleinsäuresequenzen aus *Fusarium culmorum* ist die transgene Pflanze mindestens resistent gegen *Fusarium culmorum.*

In einer besonders bevorzugten Ausführungsform zur Erzeugung einer Breitband-Resistenz gegenüber Pilzen mittels eines RNAi-Verfahrens gerichtet gegen Nukleinsäuresequenzen aus *Fusarium culmorum* ist die transgene Pflanze mindestens resistent gegen *Fusarium culmorum* und *Blumeria graminis.*

In der am meisten bevorzugten Ausführungsform handelt es sich bei der Pflanze um Gerste *(Hordeum vulgare).*

Ein weiterer Aspekt der vorliegenden Erfindung betrifft eine transgene Pflanzenzelle mit Breitband-Resistenz hergestellt nach dem obigen Verfahren.

Unter einer transgenen Pflanze bzw. Pflanzenzelle im Sinne der Erfindung ist wie oben ausgeführt zu verstehen, dass die Pflanze Nukleinsäuresequenzen enthält, die natürlicherweise nicht in der Pflanze auftreten, sondern homolog und/oder komplementär zu einer Nukleinsäure eines Pilzes sind. Diese Nukleinsäuresequenzen sind nicht in der Lage, mit endogenen Pflanzensequenzen zu interagieren und deren Expression zu beeinflussen.

In einer bevorzugten Ausführungsform enthält die transgene Pflanzenzelle ein rekombinantes Nukleinsäuremolekül umfassend einen Promotor operativ verbunden mit mindestens einer Nukleinsäuresequenz.

In einer bevorzugten Ausführungsform enthält die transgene Pflanzenzelle eines der rekombinanten Nukleinsäuremoleküle wie oben definiert.

Ein weiterer Aspekt der vorliegenden Erfindung betrifft eine transgene Pflanze, enthaltend eine der oben definierten Pflanzenzellen.

Für die Einführung von DNA in eine pflanzliche Wirtszelle steht eine Vielzahl bekannter Techniken zur Verfügung, wobei der Fachmann die jeweils geeignete Methode ohne Schwierigkeiten ermitteln kann. Diese Techniken umfassen die Transformation pflanzlicher Zellen mit T-DNA unter Verwendung von *Agrobacterium tumefaciens* oder *Agrobacterium rhizogenes* als Transformationsmittel, die Fusion von Protoplasten, den direkten Gentransfer isolierter DNA in Protoplasten, die Elektroporation von DNA, die Einbringung von DNA mittels der biolistischen Methode sowie weitere Möglichkeiten. Dabei können sowohl stabile als auch transiente Transformanten erzeugt werden.

Bei der Injektion und Elektroporation von DNA in Pflanzenzellen werden per se keine speziellen Anforderungen an die verwendeten Plasmide gestellt. Ähnliches gilt für den direkten Gentransfer. Es können einfache Plasmide wie beispielsweise pUC-Derivate verwendet werden. Sollen aber aus derartig transformierten Zellen ganze Pflanzen regeneriert werden, ist die Anwesenheit eines selektierbaren Markergens notwendig. Dem Fachmann sind die gängigen Selektionsmarker bekannt und es stellt für ihn kein Problem dar, einen geeigneten Marker auszuwählen. Gebräuchliche Seklektionsmarker sind solche, die den transformierten Pflanzenzellen Resistenz gegenüber einem Biozid oder einem Antibiotikum wie Kanamycin, G418, Bleomycin, Hygromycin, Methotrexat, Glyphosat, Streptomycin, Sulfonyl-Harnstoff, Gentamycin oder Phosphinotricin und dergleichen vermitteln.

Je nach Einführungsmethode gewünschter Gene in die Pflanzenzelle können weitere DNA-Sequenzen erforderlich sein. Werden beispielsweise für die Transformation der Pflanzenzelle das Ti- oder Ri-Plasmid verwendet, so muss mindestens die rechte Begrenzung, häufig jedoch die rechte und linke Begrenzung der im Ti- und Ri-Plasmid enthaltenen T-DNA als Flankenbereich mit den einzuführenden Genen verbunden werden.

Werden für die Transformationen Agrobakterien verwendet, muss die einzuführende DNA in spezielle Plasmide kloniert werden, und zwar entweder in einen intermediären oder in einen binären Vektor. Die intermediären Vektoren können aufgrund von Sequenzen, die homolog zu Sequenzen in der T-DNA sind, durch homologe Rekombination in das Ti- oder Ri- Plasmid der Agrobakterien integriert werden. Dieses enthält außerdem die für den Transfer der T-DNA notwendige vir-Region. Intermediäre Vektoren können nicht in Agrobakterien replizieren. Mittels eines Helferplasmids kann der intermediäre Vektor auf *Agrobacterium tumefaciens* übertragen werden (Konjugation). Binäre Vektoren können sowohl in *E. coli* als auch in Agrobakterien replizieren. Sie enthalten ein Selektionsmarker-Gen und einen Linker oder Polylinker, welche von der rechten und linken T-DNA-Grenzregion eingerahmt werden. Sie können direkt in die Agrobakterien transformiert werden (Holsters et al. (1978) Molecular and General Genetics 163: 181-187). Das als Wirtszelle dienende Agrobakterium soll ein Plasmid, das eine vir-Region trägt, enthalten. Die vir-Region ist für den Transfer der T-DNA in die Pflanzenzelle notwendig. Zusätzlich kann T-DNA vorhanden sein. Das derartig transformierte Agrobakterium wird zur Transformation von Pflanzenzellen verwendet.

Für den Transfer der DNA in die Pflanzenzelle können Pflanzen-Explantate zweckmäßigerweise mit *Agrobacterium tumefaciens* oder *Agrobacterium rhizogenes* kultiviert werden. Aus dem infizierten Pflanzenmaterial (beispielsweise Blattstücke, Stengelsegmente, Wurzeln, aber auch Protoplasten oder Suspensions-kultivierte Pflanzenzellen) können dann in einem geeignetem Medium, welches Antibiotika oder Biozide zur Selektion transformierter Zellen enthalten kann, wieder ganze Pflanzen regeneriert werden. Die Regeneration der Pflanzen erfolgt nach üblichen Regenerationsmethoden unter Verwendung bekannter Nährmedien. Die so erhaltenen Pflanzen bzw. Pflanzenzellen können dann auf Anwesenheit der eingeführten DNA untersucht werden.

Andere Möglichkeiten der Einführung fremder DNA unter Verwendung des biolistischen Verfahrens oder durch Protoplasten-Transformation sind dem Fachmann bekannt (vgl. L. Willmitzer (1993) Transgenic Plants in: Biotechnology, A Multi-Volume Comprehensive Treatise (Herausgeber: H.J. Rehm et al.), Band 2, 627-659, VCH Weinheim, Deutschland).

Während die Transformation dikotyler Pflanzen bzw. derer Zellen über Ti-Plasmid-Vektorsysteme mit Hilfe von *Agrobacterium tumefaciens* schon seit längerer Zeit wohl etabliert ist, können mittlerweile auch monokotyle Pflanzen bzw. deren Zellen mittels auf Agrobakterien basierender Vektoren transformiert werden (siehe u.a. Chan et al. (1993) Plant Mol. Biol. 22: 491-506).

Alternative Systeme zur Transformation von monokotylen Pflanzen bzw. deren Zellen sind die Transformation mittels des biolistischen Ansatzes (Wan und Lemaux (1994) Plant Physiol. 104: 37-48; Vasil et al. (1993) Bio/Technology 11: 1553-1558; Ritala et al. (1994) Plant Mol. Biol. 24: 317-325; Spencer et al. (1990) Theor. Appl. Genet. 79: 625-631), die Protoplasten-Transformation, die Elektroporation von partiell permeabilisierten Zellen sowie die Einbringung von DNA mittels Glasfasern.

Die transformierten Zellen wachsen innerhalb der Pflanze in der üblichen Weise (siehe auch McCormick et al. (1986) Plant Cell Reports 5: 81-84). Die resultierenden Pflanzen können normal angezogen werden und mit Pflanzen, die die gleiche transformierte Erbanlage oder andere Erbanlagen besitzen, gekreuzt werden. Die daraus entstehenden hybriden Individuen besitzen die entsprechenden phänotypischen Eigenschaften.

Es sollten zwei oder mehrere Generationen angezogen werden, um sicherzustellen, dass das phänotypische Merkmal stabil beibehalten und vererbt wird. Auch sollten Samen geerntet werden, um sicherzustellen, dass der entsprechende Phänotyp oder andere Eigenarten erhalten geblieben sind.

Ebenso können nach üblichen Methoden transgene Linien bestimmt werden, die für die neuen Nukleinsäuremoleküle homozygot sind und ihr phänotypisches Verhalten hinsichtlich einer vorhandenen bzw. nicht vorhandenen Pathogen-Responsivität untersucht und mit dem von hemizygoten Linien verglichen werden. Selbstverständlich können Pflanzenzellen, die die erfindungsgemäßen rekombinanten Nukleinsäuremoleküle enthalten, auch als Pflanzenzellen (einschließlich Protoplasten, Kalli, Suspensionskulturen und dergleichen) weiterkultiviert werden. Die oben dargestellten Vektoren können auf Pflanzenzellen auf verschiedene Weise übertragen werden. Ob die Vektoren in linearer oder zirkulärer Form vorliegen müssen, hängt von der jeweiligen Anwendung ab. Dem Fachmann ist bekannt, ob und wann er entsprechende linearisierte Vektoren verwenden kann oder nicht. Erfindungsgemäß umfasst der Begriff transgene Pflanze sowohl die Pflanze in ihrer Gesamtheit, als auch alle Pflanzenteile, in denen die zu Pilzgenen homologen und/oder komplementären Nukleinsäuresequenzen transkribiert werden. Bei solchen Pflanzenteilen kann es sich um Pflanzenzellen handeln, um Pflanzensamen, um Blätter, um Blüten und um Pollen. Mit "transgener Pflanze" ist erfindungsgemäß auch das Vermehrungsmaterial von erfindungsgemäßen transgenen Pflanzen gemeint wie z. B. Samen, Früchte, Stecklinge, Knollen, Wurzelstücke etc., wobei dieses Vermehrungsmaterial ggf. oben beschriebene transgene Pflanzenzellen enthält, sowie transgene Teile dieser Pflanzen wie Protoplasten, Pflanzenzellen und Kalli.

Bei der Herstellung von transgenen Pflanzen bieten sich verschiedene Methoden und Möglichkeiten an, wie bereits oben ausgeführt worden ist. Generell können Pflanzen bzw. Pflanzenzellen mit Hilfe herkömmlicher gentechnologischer Transformationsmethoden derart modifiziert werden, dass die neuen Nukleinsäuremoleküle in das pflanzliche Genom integriert werden, d.h. dass stabile Transformanten erzeugt werden und die überführten Nukleinsäuremoleküle mit dem pflanzlichen Genom repliziert werden. Je nach dem verwendeten Vektorsystem können erfindungsgemäß auch transgene Pflanzen hergestellt werden, bei denen die zu übertragenden Nukleinsäuren in der Pflanzenzelle bzw. der Pflanze als selbstständig replizierendes System enthalten sind. Die zur Übertragung der Pflanzen verwendeten Vektoren müssen dann entsprechend über DNA-Sequenzen verfügen, die die Replikation von zur Übertragung verwendeten Plasmiden innerhalb der Zelle ermöglichen.

Ein weiterer Gegenstand der vorliegenden Erfindung sind transgene Pflanzen bzw. Pflanzenzellen, die nach einem der erfindungsgemäßen Verfahren hergestellt wurden und im Vergleich zum Wildtyp über erhöhte Pilzresistenz verfügen.

Ein weiterer Gegenstand der vorliegenden Erfindung sind transgene Pflanzen bzw. Pflanzenzellen, die nach einem der erfindungsgemäßen Verfahren hergestellt wurden und im Vergleich zum Wildtyp über eine Breitband-Resistenz gegenüber Pilzen verfügen?
wobei das rekombinante Nukleinsäuremolekül zwei Nukleinsäuresequenzen umfasst, wobei eine Nukleinsäuresequenz
a) identisch und/oder komplementär zu einer oder mehreren der Nukleinsäuren ausgewählt aus SEQ ID Nos 1-5 und/oder einem Teil davon ist, oder eine Sequenzidentität von mindestens 85% zu einer oder mehreren der Nukleinsäuren ausgewählt aus SEQ ID Nos 1-5 aufweist, und eine weitere Nukleinsäuresequenz
b) identisch und/oder komplementär zu einer oder mehreren der Nukleinsäuren ausgewählt aus SEQ ID Nos 6-11 und/oder einem Teil davon ist, oder eine Sequenzidentität von mindestens 85% zu einer oder mehreren der Nukleinsäuren ausgewählt aus SEQ ID Nos 6-11 aufweist, oder
c) identisch und/oder komplementär zu einer oder mehreren der Nukleinsäuren ausgewählt aus SEQ ID Nos 12-16 und/oder einem Teil davon ist, oder eine Sequenzidentität von mindestens 85% zu einer oder mehreren der Nukleinsäuren ausgewählt aus SEQ ID Nos 12-16 aufweist
.

Unter einem "Wildtyp" wird erfindungsgemäß der entsprechende nicht genetisch veränderte Ausgangsorganismus verstanden.

Das Transformieren von Pflanzen mit dem rekombinanten Nukleinsäuremolekül der Erfindung kann durch jede bekannte Transformationsmethode erfolgen, z. B. eignen sich die biolistische Methode, die Agrobacterium-vermittelte Transformation, die Protoplasten-Transformation, die Elektroporation von partiell permeabilisierten Zellen sowie die Einbringung von DNA mittels Glasfasern. Die jeweils am besten geeignete Methode zur Transformation hängt von der verwendeten Testpflanze ab.

Bevorzugt werden die Testpflanzen durch die biolistische Transformation transformiert, weil dieser Ansatz besonders schnell und effektiv zu einer Transformation mit einer Vielzahl von verschiedenen rekombinanten Nukleinsäuremolekülen führt.

Unter einer transgenen Pflanze bzw. Pflanzenzelle im Sinne der Erfindung ist wie oben ausgeführt zu verstehen, dass die Pflanze Nukleinsäuresequenzen enthält, die natürlicherweise nicht in der Pflanze auftreten, sondern homolog und/oder komplementär zu Teilen des Pilzgenoms sind. Diese Nukleinsäuresequenzen sind nicht in der Lage, mit endogenen Pflanzensequenzen zu interagieren und deren Expression zu beeinflussen.

Ein weiterer Gegenstand der Erfindung betrifft die Verwendung der erfindungsgemäßen transgenen Pflanzen und der von ihnen abgeleiteten Zellen, Zellkulturen, Teile und transgene Vermehrungsmaterialien zur Herstellung von Nahrungs- und Futtermitteln, Pharmazeutika oder Feinchemikalien.

Die Infektion von Testpflanzen mit Pilzorganismen zur Untersuchung eventueller Resistenzphänomene ist ein dem Fachmann durchaus bekannte Verfahren. Die verwendeten Testpflanzen müssen für diesen verwendeten Pilz empfänglich sein, also als Wirtspflanze für diesen Pilz dienen können, und der Pilzbefall muss sich durch einfache Methoden nachweisen lassen. Bevorzugt handelt es sich bei den Testpflanzen um Weizen- oder Gerste-Pflanzen, die z. B. mit dem Mehltaupilz *Blumeria graminis* inokuliert werden. Unter "Inokulieren" versteht man das Inkontaktbringen der Pflanze mit dem Pilz, mit dem die Pflanze infiziert werden soll, oder den infektiösen Teilen davon, unter Bedingungen, unter denen der Pilz in eine Wildtyp-Pflanze eindringen kann.

Der Pilzbefall der Pflanze lässt sich anschließend mit einem geeigneten Auswerteverfahren auswerten. Dazu eignet sich vor allem die visuelle Auswertung, bei der die ausgebildeten Pilzstrukturen in der Pflanze detektiert und quantifiziert werden. Um die erfolgreich transformierten Zellen zu identifizieren, wird bevorzugt zusammen mit dem RNAi-Konstrukt ein Reportergen wie z.B. das β-Glucuronidase-(GUS)-Gen aus E. coli, ein Fluoreszenzgen wie etwa das Green-Fluorescence-Protein (GFP)-Gen aus *Aequorea victoria,* das Luziferase-Gen aus *Photinus pyralis* oder das β-Galaktosidase-(lacZ)-Gen aus *E*. *coli,* dessen Expression in den Pflanzenzellen durch einfache Verfahren nachgewiesen werden kann, in einem geeigneten Vektor kotransformiert. Optional können die ausgebildeten Pilzstrukturen zur besseren Bestimmung durch Verfahren, die dem Fachmann bekannt sind, angefärbt werden, z. B. durch Coomassie- oder Trypanblau-Färbung.

Ein weiterer Aspekt der vorliegenden Erfindung betrifft die oben definierten rekombinanten Nukleinsäuremoleküle umfassend mindestens zwei Nukleinsäuresequenzen in operativer Verknüpfung mit einem in Pflanzenzellen funktionsfähigen Promotor, wobei eine Nukleinsäuresequenz
a) identisch und/oder komplementär zu einer oder mehreren der Nukleinsäuren ausgewählt aus SEQ ID Nos 1-5 und/oder einem Teil davon ist, oder eine Sequenzidentität von mindestens 85% zu einer oder mehreren der Nukleinsäuren ausgewählt aus SEQ ID Nos 1-5 aufweist, und eine weitere Nukleinsäuresequenz
b) identisch und/oder komplementär zu einer oder mehreren der Nukleinsäuren ausgewählt aus SEQ ID Nos 5-10 und/oder einem Teil davon ist, oder eine Sequenzidentität von mindestens 85% zu einer oder mehreren der Nukleinsäuren ausgewählt aus SEQ ID Nos 5-10 aufweist, oder
c) identisch und/oder komplementär zu einer oder mehreren der Nukleinsäuren ausgewählt aus SEQ ID Nos 11-15 und/oder einem Teil davon ist, oder eine Sequenzidentität von mindestens 85% zu einer oder mehreren der Nukleinsäuren ausgewählt aus SEQ ID Nos 11-15 aufweist.

Die im Verfahren verwendeten Nukleinsäuremoleküle können unter Verwendung molekularbiologischer Standardtechniken und der hier bereit gestellten Sequenzinformation isoliert werden. Auch können mit Hilfe von Vergleichsalgorithmen, wie sie z. B. auf der NCBI-Homepage unter http://www.ncbi.nlm.nih.gov zu finden sind, beispielsweise eine homologe Sequenz oder homologe, konservierte Sequenzbereiche auf DNA- oder Aminosäureebene identifiziert werden. Wesentliche Teile dieser Sequenz oder die gesamte homologe Sequenz können als Hybridisierungssonde unter Verwendung von Standardhybridisierungstechniken (wie z. B. beschrieben in Sambrook et al., vide supra) zur Isolierung weiterer im Verfahren nützlicher Nukleinsäuresequenzen aus anderen Organismen durch das Screening von cDNA- und/oder genomischen Banken verwendet werden. Überdies lässt sich ein Nukleinsäuremolekül durch Polymerasekettenreaktion isolieren, wobei Oligonukleotidprimer auf der Basis der in Datenbanken angegebenen Sequenzen oder von Teilen davon verwendet werden (z. B. kann ein Nukleinsäuremolekül, umfassend die vollständige Sequenz oder einen Teil davon, durch Polymerasekettenreaktion unter Verwendung von Oligonukleotidprimern isoliert werden, die auf der Basis dieser gleichen Sequenz erstellt worden sind). Zum Beispiel lässt sich mRNA aus Zellen isolieren (z. B. durch das Guanidiniumthiocyanat-Extraktionsverfahren von Chirgwin et al. (1979) Biochemistry 18: 5294 - 5299) und daraus mittels reverser Transkriptase (z. B. Moloney-MLV-Reverse Transkriptase, erhältlich von Gibco/BRL, Bethesda, MD oder AMV-Reverse-Transkriptase, erhältlich von Seikagaku Amerika, Inc., St. Petersburg, FL) cDNA herstellen. Eine Nukleinsäure kann unter Verwendung von cDNA oder alternativ von genomischer DNA als Matrize und geeigneten Oligonukleotidprimern mittels Standard-PCR-Amplifikationstechniken amplifiziert werden. Die so amplifizierte Nukleinsäure kann in einen geeigneten Vektor kloniert werden und mittels DNA-Sequenzanalyse charakterisiert werden. Oligonukleotide, die sich zur Amplifikation der gewünschten Sequenz eignen, können durch Standardsyntheseverfahren, beispielsweise mit einem automatischen DNA-Synthesegerät, hergestellt werden.

Die beispielhafte Identifizierung von Pilzgenen, die sich im Verfahren der vorliegenden Erfindung eignen, und deren Verwendung zur Erzeugung einer Breiband-Resistenz in transgenen Pflanzen wird nun im Folgenden dargestellt. Die nachfolgenden Beispiele sollen nicht als beschränkend aufgefasst werden. Der Inhalt sämtlicher in dieser Patentanmeldung zitierten Literaturstellen, Patentanmeldungen, Patente und veröffentlichten Patentanmeldungen ist hier durch Bezugnahme aufgenommen.

### Beispiele

### A. Methode zur Erzeugung von Triple-RNAi Konstrukten

Die Herstellung der RNAi-Konstrukte der vorliegenden Erfindungsmeldung basiert auf der modifizierten GATEWAY-Technologie nach Douchkov et al. (2005) Mol. Plant. Microbe. Interact. 18:755-61*.*

Als Basis für diese Konstrukte dienen konservierte Proteine bzw. die dafür kodierenden Gene phytopathogener Pilze. Als Beispiele sind hier Gamma-Aktin, das mitochondrialer ADP/ATP carrier Protein, die RhoGTPase, der Elongationsfaktor 1 sowie das mitochondriale Hitzeschockprotein 70 Chaperon aufgeführt.

Zwischen verschiedenen Pilzen konservierte Sequenzabschnitte innerhalb der Gene, die für die oben genannten Proteine kodieren wurden durch Sequenzvergleiche ermittelt.

Dazu wurden entsprechende DNA-Sequenzdaten aus verschiedenen Pilzen mittels der Sequencher-Software (Gene Codes Corp., Ann Arbor, MI, USA) unter Verwendung des "Dirtydata"-Algorithmus verglichen, und eine Konsensussequenz abgeleitet.

Folgende Sequenzen aus der COGEME-Datenbank (http://cogeme.ex.ac.uk) und aus der NCBI-Datenbank (http://www.ncbi.nlm.nih.gov/sites/entrez) wurden für die verschiedenen Gene als Grundlage für die Sequenzvergleiche eingesetzt:
Gamma-Aktin (GA): BfCon[0048], Contig[0009], CpCon[0433], FsCon[0011], FsCon[0012], GzCon[0219], MagCon[0535], MagCon[10153a], MagCon[11943a], mg[1255], Mg_4266, Pi10229566, PiCon[0102], PsCon[0011], UmCon[0226], VD0108G03, YDL029W Chr4, YFL039C Chr6, YHR129C Chr8, YJR065C Chr10
Elongationsfaktor1 (EF1): BfCon[0035], BfCon81924], BfCon[2093], Cf8667247, CtCon[0128], DQ522321, GzCon[0015], Mag3392227, MagCon[2061], MagCon[2896], MagCon[3191], MagCon[4553], MagCon[4838], MagCon[5002], MagCon[5960], MagCon[6041], MagCon[690a], MagCon[10107a], MagCon [12375a], MagCon[12727a], MagCon[13104a], MagCon[13904a], mg[0073], mg[0358], SSPG1015, Um34332478, UmCon[0014], UmCon[1272], UmCon[2780], VD0110B10, VD0201A10
Mitochondrialer ADP/ATP Translokator (AAC): BfCon[0014], BfCon[1221], Cf8667327, CfCon[0008], Contig[0005], FsCon[0026], FsCon[0406], GzCon[0025], mg[0104], SSPG985, VD0100D40, VD0203D11
Mitochondriales Hitzeschockprotein 70 (Hsp70): BfCon[0098], Cf8667122, Cf8667223, FsCon[1028], FsCon[1857], Gz47835892, GzCon[1242], GzCon[2886], GzCon[3587], MagCon[2045], MagCon[10827a], mg[1360], mga0294f, UmCon[1735], UmCon[3134], VD0204G10
Rho GTPase (Rho): AF395859, BfCon[0909], Bg13902524, Bg27453832, BgCon[2092], CfCon[0041], Contig[0007], CpCon[0819], CtCon[0239], FsCon[0840], Gz47836309, GzCon[0911], GzCon[7209], Lm13259684, Mag14180535, Mag30400635, Mag30403197, MagCon[0027a], MagCon[1398], MagCon[4208a], MagCon[6895], MagCon[11651a], mg[1439], mga0839f, SSPG240R, Um34331745, Um37403496, Um37407871, UmCon[0162], UmCon[0987], UmCon[1018], UmCon[2074], W0AA016ZE09C1

Als Ergebnis dieser Sequenzvergleiche wurde für jedes Gen eine Konsensussequenz erstellt (Für Nukleotidsymbole siehe Tabelle auf S.120):

### Konsensussequenz für AAC

GC:AGACCGTTCTTGGGCATGCCCCCTTCGTCGCTGACTTCCTCATGGG
TGGTGTCTCCGCCGCTGTCTCCAAGACCGCTGCYGCCCCATYGAGCGTG
TCAAGCTCCTCATCCAGAACCAGGATGAGATGCTGAAGACCGGTCGTCTY
GACCGCAAGTACGACGGCATTGGTGASTGCTTCAAGCGYACCACCGCSGA
TGAGGGTGTCATGTCCCTCTGGCGWGGHAACACTGCCAACGTCATCCGTT
ACTTCCCTACCCAGGCCCTGAACTTCGCTTTCCGTGACAAGTTCAAGTCG
ATGTTCGGCTACAAGAAGGACCGTGATGGCTACGCCATGTGGATGGCYG
GTAACYTKGCCTCCGGTGGTGCTGCTGGTGCCACTTCCCTCCTCTTCGTCT
ACTCCCTCGACTACGCCCGTACYCGTCTTGCCAACGACGCCAAGAACGCC
AAGACCGGTGGTGACCGTCAGTTCAACGGTCTSGTCGATGTCTACAAGAA
GACCCTCGCCTCTGACGGTATTGCCGGTCTCTACCGTGGTTTCGGT:CCT
TCCGTTGCTGGTATCGTCGTYTACCGTGGTCTCTACTTCGGWATGTACGA
YTCCATCAAGCCAGTCCTCCTCAYYGGWMMYCTYGAGGGYAACTTCCTT
GCWTCCTTCTTGCTCGGATGGACCGTCACCACTGGTGCCGGTATCGCCTC
TTACCCATTGGACACCATCCGTCGTCGTATGATGATGACTTCTGGTGAGG
CCGTCAAGTACAAGTCTTCCTTGGATGCTGSYCGYCARATCGTYGCCAAG
GAGGGWGTYAAGTCTCTCTTCAAGGGTGCTGGTGCCAACATTCTCCGTG

GTGTTGCAGGTGCTGGTGTCYTGTCCATCTACGATCARATGCARGTCYTG
ATGTTCGGAAAGGCATTCAAGTAAAYGWATCGTA:GA:ATGGAGTGAAGG
::G::ATGAG:T:TGAKTG:TAGA:GGTGGT:TGATGATAATGCGAAATGAG:C:G
CCT::TGRGGTCG:GTC:C:G:TGGTT:GA:AA::CT::AAAAGGTTG:AG:ATSGTA
G:TACAG::ATCSATTGC:ATTG:TG:T:TRSAGATAC:TT:GMCGTG:TAGTCTG
MCAC::AR:CAWGATCT:AT:ACRA:TYTGAGT:SGCTTMTG:CA:C:AGAGT::::
CTT:TAT:CTA:ATCTTKCWTT:GCCTTCCGTTCTATGCCKV:T:GTA:ATT::T::
A:TACACCAATTCAAVT::TCC:CTTTA:CTGTCTRTTCNCTTA:A:CTGAGDDT
TMAATTTKRMKCMTSTMKTCATCCTKKTCAKTGCTGACYCYTRTKTCTM
CACTCTRTCTWTTCSYTTTCWTCCATTTGCYMYWCYCMCTCCTATCMKT
AGCATACTWCGTAYWATCYKTSTTGCCTAACGTMYKWACTACCYCYMT
TTMYTRCTTWKCGATTACTMTYYCTWTMTYYCTYCGKTWMCMYTCWCS
TWYMTGTTKCATTMTCTCTGACTCTCTCTMTYCTCCCCTCTMTAYACYYK
YYTTRTYCTKTYGCCMYTMRYCCTMTCTTCTMATTTTCCTACTTCTATTC
TCTTGCTATTTCTCTCCTATCTGTTCCATCCCTTCATTCATCATTTCTCTCT
CTCTGCTTCAACGATTATCATCACTATTCTATCTC

### Konsensussequenz für EF1

ATGGGTAAAGGAGAAGACTCACATCAACGTCGTCGTCATCGGCCACGTC
GACTCCGGCAAGTCGACCACCACCGGTCACTTGATCTACAAGTGCGGTGG
TATCGACAAGCGTACCATCGAGAAGTTCGAGAAGGAAGCCGCCGAGCTC
GGYAAGGGTTCCTTCAAGTACGCCTGGGTTCTTGACAAGCTCAAGGCCGA
GCGTGAGCGTGGTATCACCATCGACATCGCCCTCTGGAAGTTCGAGACYC
CCAAGTACTATGTTACCGTCATTGACGCCCCTGGTCACCGTGACTTCATC
AAGAACATGATCACTGGTACCTCCCAGGCCGACTGCGCCATTCTCATCAT
TGCCGCTGGTACTGGTGAGTTCGAGGCTGGTATCTCCAAGGATGGCCAGA
CTCGTGAGCACGCTCTCCTCGCCTACACCCTCGGTGTCAAGCAGCTCATC
GTYGCCATCAACAAGATGGACACCACCAAGTGGTCCGAGGACCGTTWCC

AGGAGATCATCAAGGAGACCTCCAACTTCATCAAGAAGGTCGGCTACAA
CCCCAAGACYGTCGCCTTCGTCCCCATCTCCGGTTTCAACGGTGACAACA
TGMTCGACGCCTCCACCAACTGCCCCTGGTACAAGGGTTGGAGAAGGA
GACCAAGKCWGGCAAGGYCACCGGCAAGACCCTCCTCGAGGCCATCGA
CGCCATCGAGCCCCCCAAGCGTCCCACCGACAAGCCCCTCCGTCTTCCCC
TCCAGGATGTCTACAAGATCGGYGGTATTGGMACTGTTCCCGTCGGCCGT
RTCGAGACYGGTATCATCAAGCCCGGYATGGTCGTCACCTTCGCTCCCGC
CAACGTCACCACTGAAGTCAAGTCCGTCGAGATGCACCACGAGCAGCTC
ACTGAGGGTSTTCCCGGTGACAACGTCGGTTTCAACGTCAAGAACGTTTC
CGTCAAGGACATTCGCCGTGGTAACGTCGCTGGTGACTCCAAGAACGAC
CCCCCCAAGGGTGCCGCTTCCTTCAACGCYCAGGTCATYGTCCTYAACCA
CCCTGGTCAGGTCGGTGCTGGTTACGCCCCCGTTCTCGATTGCCACACTG
CCCACATTGCCTGCAAGTTCTCCGAGCTCCTCGAGAAGATCGACCGCCGT
ACCGGTAAGTCCATTGAGGACTCCCCCAAGTTCATCAAGTCTGGTGACGC
TGCCATCGTCAAGATGGTTCCCTCCAAGCCCATGTGTGTTGAGGCCTTCA
CYGACTACCCTCCTCTGGGMCGTTTCGCCGTCCGTGACATGCGTCAGACC
GTCGCTGTCGGTGTCATCAAGTCCGTCGACAAGTCCACGACACTGCCGGT
AAGGTCACCAAGKCCGCCGYCAAGGCTGGTGCCAAGAAATAAACTTATT
TATG:AATGA::CGA:CCT:TCA:G:TBGGATSTTGCGASTTTG::WT:TCAYGCT
CA:TGAGGCTGATC:T:ACTGGCTT:GT:ACGCC:GAATCGATCAT:TTG:CA:
TGAG:C:AC:TC:GKGTAATGACT:TGSA:G:A:KT:C:TTSAGA:G:ATWTT:CAA
ATGCCT:GCTGATS:A:C:GTTGT:G:GCA:CATCA:C:ATA:GAACACT:TMGTC
KA::G:AA:AT:G:ACTTC:ARAG:AAAAAAAAAAAAAACAW:GT:CATG:ACCA
AACGCCGAGATCACTTCTGAG:GACTCTA:TGTAG:ACTAAAGC:ACAT:GT:
CAAGAATTTTTTGAGTTACGAAATCCCAAACCGTCATTTCATCATGTGC
CCCAATAAAAACCGAGTACTCTTTTGTGATAAAGCCAAAATTGACCGGA
AAACCTGCGTTTGCGAACGAGGAAAGTGCCCAAGATGCTACGACATGCT
TCCTGGCTTGTCGTGGCTCGACATTAGATGTTTGCTGKTTGGTTGATCGCA

GCVTYATATTTCTTTKGGCSGGCCCCTTGGCCACGCGGGRACCTTGGGTG
ACCCTTGSCCCTGGGGTACCCTGGGGACTTGGTCGACGGCTCTTGATGAC
ACCGAACAGCGACGGTCTGACGCATGTCACGGACGGCGAAACGACCAAG
GGGAGGGTACTCGGAGAAAGTCTCGACACACATGGGCTTGGACTCGTSC
GCBCMMGRATMMCTAGCAGACCGGRGG

### Konsensussequenz für GA

CCACGCGTCCGCCCTTTTCTGCCCAACAACTTTCCT:CTCYBTCAACGATC
TWCT::TTTCC:CA:TCAACT:T:GTCGCCTG:CAACA::A:TCTCT:GC:TCTA:CA
TCT::TCAG:A:TCTAT::C::ATTCCTCC:GCGACTT:TCG:C:GA:TTC:TTCT:WTC
:TCCTGAC:CT:TG:ATT:CA:TC::CG:::C:C::AG:C:TCTC:TTM:ATCTCC:CTATT
CCACT:A:CACA:CCT:TA:ATCMATCA:CGATGGAGGAAGAAGTCGCWGCT
CTCGTCATYGACAATGGTTCGGGTATGTGCAAGGCCGGTTTCGCCGGTGA
CGATGCTCCCCGAGCTGTCTTCCCTTCCATTGTCGGTCGCCCCCGTCACCA
TGGTATCATGATTGGTATGGGCCAGAAGGACTCGTACGTTGGTGATGAGG
CCCAGTCCAAGCGTGGTATCCTCACTCTGCGGTACCCCATCGAGCACGGT
GTYGTCACCAACTGGGACGA:CATGGAGAAGATCTGGCACCACACCTTCT
ACAACGAGCTGCGTGTCGCCCCCGAGGAGCAC:CCCGTCCTGCTGACTGA
GG:CTCCCATCAACCCCAAGTCCAACCGTGAGAAGATGACGCAGATCGTC:
TTCG:AGACCTTCAACGCCCCCGCCTTCTACGTCTCCATCCAGGCCGTKCT
GTCCCTGTACGCCTCCGGTCGTACCACTGG:TATCGTGCTCGACTCYGGTG
ACGGTGTTACTCACGTTGTGCCCATCTACGAGGGTTTCKCCCTKCCCCAC
GCCATTGCCCGTGTCGACATGGCTGGTC:GTG:A:CTTG:A:CCG::AC:T::ACC
TG:ATGAAGA:TCTTGGCTGAGCGCGGTTACA:CCTT:CTCC:ACCAC::T:GCC
GAGCGWGAAATC:GTC:CGTGA:CATCAAGGAGAAGCTCT:::GCTACGTCG
CCCTT:::GA:CTTCGAGCAGGAGATCCAGACTGCTGCCCAGTCCTCCAGC:Y
TGGAGAAGTCCTACGAGCTTCCCGACGGACAGGTTATCACCATCG:GCAA
CGAGCGC:T:TCCG:TG:CT:CCTGAGGCTCTGTTCCAGCC::TTCTGT:CC:TGG
GTCTTGAGA:GCGG:TGGTATCC:ACGT:CACCACTTTCAACTCCATCATGA
AGTGTGATGTCGA:TGTCCGAAAGGATCT:GTACGGCAACATTGTCATGTC
TGGTGG:TACCACC::ATG:TACCC:TGGTATCTCCGAC:CGTATGCA:GAAG:G
AGATCACTGCT:CTTGCCCCK:TCCTCSATGAAGGTCAAGATCATTGCTCCT
CCCGAGCGCAAGTACTCCGTCTGGATCGGTGGTTCCATTCTCGCTT:CG:C::

TGTCGACCTT:CCAGCAGATGTG::GATCTCGAAGCAGGAGTACGACGAGA
GCGGACCTTCSATCGTSCACCGCAAGTGCTTCTAAGC:G:CCTGACCG:A:CG
A:TT::GTGC:TCCAACT:CG::C:TT:ACGAA:CTG:GAK:CAGAA:ATGTC:GCGA:
CAG:ACGC:TA:AGC:KTCGDGAGG:TTG:GG:T:GCTT:CGAAG:CTCG:TCCGA
AACTCGTRTCTG:CG:AAC:A:TTTA::TTACATG:G::TCGA:T:GAGTSAACGTT
GCGCATG:TACG:AGAGATG:GATGGCCTGWT:T:T:CTTGCAGGTCGAGTA
C::AATG:CGT::CTAGGGC:TASAAG:BCGA:GGTTGACATAC:GAATA:CG:TG
TCTACTW::TCC::G:C::TTAGARCTG:ACGACA:T:AW:TC:GAGGGGTTGA:TC
AT:GCARCGYGAG::T::CHG:TC:CC:TTGCT:CTAGAGAATCRAMTTAMAWG
GTMGWAAM:TGA:AAAGAGBTCRGTGGMCATCCTGSCRMRCARTRAWMS
ATMYGMYAACGMMAMWMARWARAMRWRDMAMMWWMAAAAAAA
AAAAAAAAAAAAAAAAAAAAAAAAAAAAAAAAAAAGGGCGGCCGC
TCTAN

### Konsensussequenz für Hsp

GCACGAGGAGAAGGTTGAGGGTGAGCGCAACGTCCTCATCTTCGATCTT
GGYGSWSGWRSCTTCGATGTCTCTCTCCTTACCATTGAGGAGGGTATCTT
CGAGGTCAAGTCTACTGCCGGTGACACTCACTTGGGTGGTGAAGATTTCG
ACAACCGCCTCGTTAACCACTTCGTTAACGAGTTCAAGCGAAAGCATAAG
AAGGATCTAAGCACCAACGTCCGTGCTCTTCGACGTCTCCGAACCGCTTG
TGAGCGCGCCAAGCGAACTCTCTCTTCTTCCGCTCAGACCTCCATTGAGA
TCGACTCKCTSTWCGAGGGTATTGACTTCTACACCTCCATCACCCGTGCC
CGTTTCGAGGAGCTCTGCCAGGATCTCTTCCGATCCACCATCCAGCCCGT
CGACCGTGTCCTTACCGACGCCAAGATCGACAAGTCCCTBGTCCACGAGA
TCGTCCTCGTYGGYGGWTCYACCCGTATCCCCCGTGTCCAGAAGCTCATC
ACCGACTACTTCAACGGAAAGGAGCCCAACAAGTCCATCAACCCYGATG
AGGCTGTTGCCTACGGTGCTGCCGTCCAGGCTGCCATTCTCTCTGGTGAC
ACCACCTCCAAGTCCACCAACGAGATCCTGCTYCTCGATGTCGCCCCSCT
STCTCTCGGTATCGAGACCGCTGGTGGTATGATGACCAAGCTCATCCCCC
GCAACACCACCATCCCCACCAAGAAGTCYGAGGTCTTCTCCACCTTCTCC
GACAACCAGCCTGGTGTGCTCATCCAGGTCTACGAGGGTGAGCGCCAGC
GCACCAAGGACAACAACCTSCTGGGCAAGTTCGAGCTTACCGGCA:TCCC
MCCTGCTCCCCGTGGTGTTCCCCAGATTGAGGTCACCTTCGACCTSGATG
CCAACGGTATCATGAACGTCTCCGCCGTCGAGAAGGGCACCGGCAAGAC
CAACAAGATTGTCATCACCAACGACAAGGGCCGYCTGTCCAAGGAGGAG
ATYGAGCGCATGCTTGCYGAGGCCGAGAAGTACAAGGAGGAGGATGAG
GCYGAGGSCRMCCGTGTCTCTGCCAAGAACGGCCTYG:AGTCKTACGCCT
ACTCCCTSCGCAA:CACCCTSTCCG:ACYCCAAGGTCGASG:AGAAGCT:TG
AKGCTKCCGACAAGGAGAMSCTCAMYGCTGAGATYRACAAGRTYGTCCA
GCTGGCTYGAYGASARCCAGCAGGCTACYMRKGAGGAGTAYGAGGAGC
ACCAGAAGGAGCTYGAGGSYRWKGCYAACCCCATCATGATGAARTTCTA

CGGAGCTGSTGGTGAGGGYGCTCCCGGTGGCATGCCCGGYRYRCCCGGTS
GTGCCCCTGGTGGCTTCCCTGGTGCYGGTGGCCCYGCYCCYGGYGCCGG
YGGYGACGATGGYCCCACYGTCGAGGAGGTCGACTAAAYWMYTYGMTA
CYCYWRTAMCATCAKTCYCKAMTTSTWKWKYGRKCAYAKMTSSRTKKK
TWTWATGCGGWKKYTAGTMGTTTKMAYGSKMTGGRTSWGATTASATTG
TTCMTTTTCTYCWRTTTTTMTCGTAAYGACWTYACGRWTRKRAAAAGTC
WMMGGYGVTYYATRYACGTGGKGRWTGTATWTYAATTAGRATCWTWC
AWTSRDAAMAAMARWWMAAAAWAAAAAAACTCGAAACTAGTTCTCT
CTCTCTCTCCTCGTGC

### Konsensussequenz für Rho

AATCCATMACCATGGCTGAAATCCGCCGAAAGCTCGTCATTGTCGGHGA
YGGTGCTTGTGGTAAGACYTGTTTGTTGATTGTCTTCTCCAAGGGCACHT
TCCCMGAGGTCTACGTCCCMACCGTCTTCGAGAACTATGTCGCCGATGTC
GAGGTCGATGGCAAGCACGTCGAGCTCGCGCTATGGGATACBGCTGGTC
AGGAGGATTACGACCGTCTTCGACCTCTCTCATACCCCGACTCSCACGTT
ATCCTGATCTGCTTCGCCGTTGACTCTCCCGATTCCCTCGAYAACGTYCA
GGAGAAGTGGATCTCCGAGGTCCTSCAYTTCTGCCAGGGTCTCCCTATCA
TCCTTGTCGGCTGCAAGAAGGATTTGCGAYACGACCAGAAGACCATTGA
GGAGCTCCACAAGACCASCCAGAASCCCGTCACCCCWGARCAGGGTGAA
GAGGTCCGCAAGAAGATTGGTGCTTACAAGTATCTTGAGTGCTCAGCCAA
GACCAATGAAGGTGTCCGYGAGGTGTTCGAGCACGCCACTCGCGCYGCT
CTCCTGACGCGGAAGAAGCAGAAGAAGCGCGCAAG

Mit dem BLAST-Algorithmus (http://www.ncbi.nlm.nih.gov/blast/Blast.cgi) wurde nun aus den oben genannten COGEME- bzw. NCBI-Sequenzen jeweils diejenige ermittelt, die die höchste Sequenzhomologie zur jeweiligen Konsensussequenz aufwies (SEQ IDs Nos. 1-5):

| | | |
|---|---|---|
| **GA:** | GzCon[0219] | (95 % Identität zur GA-Consensus-Sequenz) |
| **EF1:** | GzCon[0015] | (91 % Identität zur EF1-Consensus-Sequenz) |
| **AAC:** | FsCon[0026] | (93 % Identität zur AAC-Consensus-Sequenz) |
| **Hsp:** | GzCon[2886] | (92 % Identität zur Hsp-Consensus-Sequenz) |
| **Rho:** | FsCon[0840] | (93 % Identität zur Rho-Consensus-Sequenz) |

Geeignete Bereiche für die Ableitung von siRNA-Zielsequenzen innerhalb dieser Sequenzen wiederum wurden mit dem RNAiscan-Algorithmus (http://bioinfo2.noble.org) ermittelt. Dabei wurde die Zielsequenzen so ausgewählt, dass kein bzw. nur minimales "cross-silencing" gegenüber entsprechenden Sequenzen aus Gerste und/oder Weizen zu erwarten wäre.

Primer wurden aus den jeweiligen Zielsequenzen abgeleitet und ca. 300-350bp große Fragmente (SEQ ID Nos. 6-11) wurden aus Fusarium cDNA- bzw DNA-Proben isoliert und in RNAi-Vektoren kloniert (vgl. u.). Im Falle von GA lassen sich zwei verschiedende GA-Fragmente ("GA":300bp; "GA-1":350bp) darstellen.

Die Abschnitte innerhalb der Konsensussequenzen zu denen die oben angeführte Contigs die beschriebene hohe Homologie aufweisen, also die konservierten Bereiche innerhalb der Konsensussequenzen sind als SEQ ID Nos. 12-16 weiter unten aufgeführt.

### A1. Präparation des "Entry vector" (pIPKTA38)

Bakterien, die das Plasmid enthielten wurden in LB + Kanamycin (50 µg/mL) propagiert. Plasmid DNA wurde mit dem *Jetstar* midi DNA kit präpariert. Es erfolgte ein Kontrollverdau mit dem Restriktionsenzym *Apa* I. Die DNA-Konzentration wurde gemessen und anschließend auf 150 ng/µL eingestellt.

### A2. Präparation der "Destination vectors"

Die Vektoren pIPKb007, pIPKb010 and GER4 (p6UGLP4deltaSwaIntronRNAi) wurden als "destination vectors" verwendet. Bakterien, die das jeweilige Pasmid enthielten wurden in LB + *Spectinomycin* (100 µg/mL) propagiert. Plasmid DNA wurde mit dem *Jetstar* midi DNA kit präpariert. Die Plasmid-Präparationen wurden mit *EcoRV* (Banden: pIPKb007 - 12039, 3953 bp; pIPKb010 - 12039, 3821 und 1148 bp; GER4 - 12039, 3869 und 1721 bp) kontrollverdaut.

Die DNA-Konzentration wurde gemessen und auf 150 ng/µL eingestellt.

### A3. PCR-Amplifikation der Zielsequenzen konservierter Pilzgene

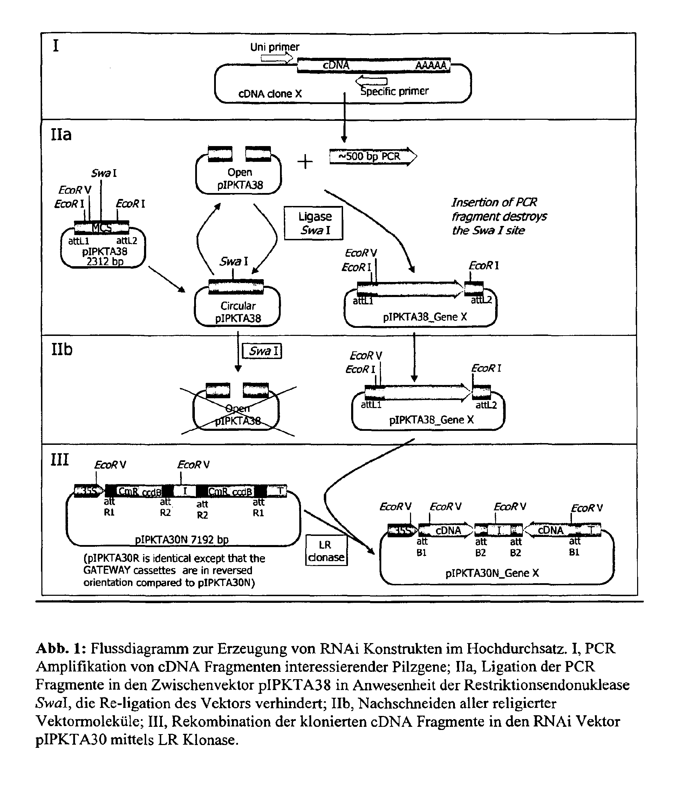

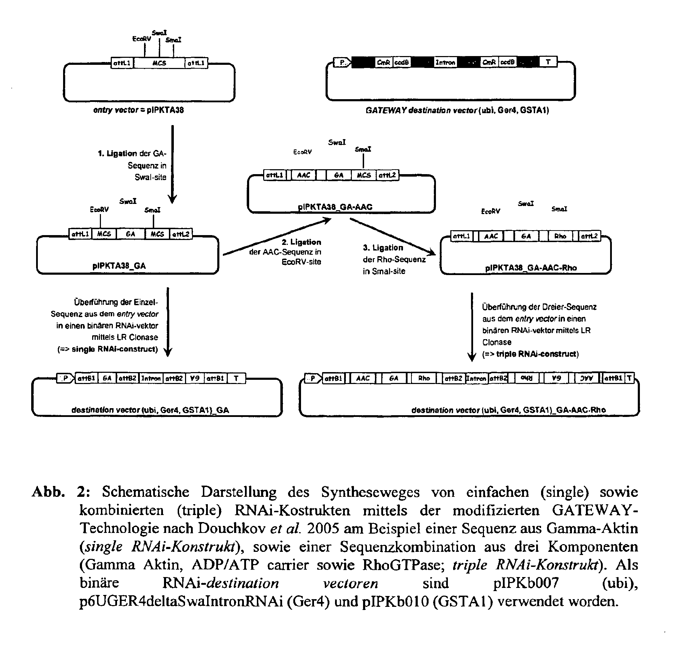

Die mittels PCR aus *Fusarium culmorum-DNA*/*cDNA* amplifizierten Fragmente von ca. 300 bp aus den kodierenden Sequenzbereichen der ausgewählten konservierten Pilz-Gene sind zuerst in den pIPKTA38 als *entry vector* ligiert worden (s. Abb.1 und 2). Hierzu wurden verschiedene enzymatische Schnittstellen (SwaI, EcoRV, SmaI) innerhalb der *multiple cloning site* genutzt. Diese wurden entsprechend der gewünschten Sequenzkombinationen der RNAi-Zielkonstrukte ausgewählt (s. Tab. 5). Die Ligation der ersten Sequenz erfolgte in die SwaI-Site des pIPKTA38. Um zwei weitere Sequenzen einzufügen wurde das pIPKTA38-Konstrukt mit der erfolgreich eingefügten ersten Sequenz verwendet. Dieses wurde über zwei weitere Ligationen zu einem Konstrukt mit drei verschiedenen Sequenzen fertig gestellt (s. Abb.2).

### "Master mixes" für 20-µL PCR Reaktionen mit zwei spezifischen Primern

Spezifische Primer wurden entworfen um ∼300 bp Fragmente aus *Fusarium culmorum* DNA oder cDNA zu amplifizieren. Die Schmelztemperatur *(Tm)* der Primer war ∼65°C.

Ein "Master Mix" (s. Tabelle 1) wurde präpariert und 3,75 µL/well wurden in wells einer 96-well PCR-Platte gegeben. Jeweils 8,5 µL "target"-spezifischer "forward"- bzw. "reverse"-Primer wurden zu jedem well gegeben.

**Tabelle 1: Zusammensetzung Master mix:**

| **Komponente** | **1x** | **10x** |
|---|---|---|
| *Thermal Ace Puffer (10x)* | *2 µL* | 20 *µL* |
| *dNTPs* (*50x, jeweils 10 mM)* | *0,5 µL* | *5 µL* |
| *Thermal Ace DNA Polymerase (2U*/*µL)* | *0,25 µL* | *2,5 µL* |
| *Template (Fusarium culmorum DNA)* | *1,0 µL* | *10 µL* |
| | *3,75 µL Master mix pro well* | |
| *Target-spezifscher Primer - forward (1 µM)* | *8,5 µL* | |
| *Target-spezifscher Primer - reverse (1 µM)* | *8,5 µL* | |

### PCR Zyklus Konditionen

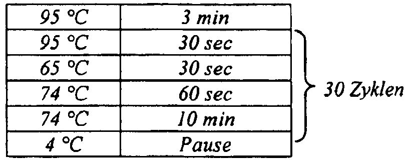

### Aufreinigung des PCR-Produkts

Zu jeder PCR-Reaktion wurden 30 µL H₂O gegeben um ein Gesamtvolumen von 50 µl einzustellen. Anschließend erfolgte die Aufreinigung mittels *Qiagen MinElute UF 96-well* kit, wobei mit 20 µL H₂O eluiert wurde.(s. das Qiagen *MinElute* Protokoll). Zur Kontrolle wurden jeweils 2 µL des aufgereinigten PCR-Produkts mittels Agarosegelektrophorese aufgetrennt.

### A4. Klonierung der PCR-Produkte

Ein Ligations-Mix wurde hergestellt (s. Tabelle 2), jeweils 6 µL Ligationsmix wurden in jedes well gegeben und 4 µL aufgereinigtes PCR-Produkt dazu pipettiert. Die Ansätze wurden für 1 h bei 25°C inkubiert, dann wurde die Reaktion durch Erhitzen auf 65°C für 10 min gestoppt. Jeweils 5 µL *Swa I* / *EcoR V* / *Sma I -Mix* (s. Tabelle 3) wurden zu jedem well gegeben. Es erfolgte eine Inkubation bei 25°C für 1 h. Als nächster Schritt erfolgte die Transformation der Ligationsansätze in kompetente Bakterien, und die Isolierung geeigneter Klone nach Miniprep und Kontrollverdauen.

**Tabelle 2: Ligations Mix für 10-µL Reaktionen (6 µL mix + 4 µL PCR-Produkt pro Reaktion)**

| ***Komponenten*** | ***1x*** |
|---|---|
| *H₂O* | *1 µL* |
| *1) pIPKTA38 (150 ng*/*µL)* | *1 µL* |
| *2) pIPKTA38_target1 (150 ng*/*µL)* | |
| *3) pIPKTA38 target1 target2 (150 ng*/*µL)* | |
| *Ligations-Puffer (10x)* | *1 µL* |
| *50% PEG 4000* | *1 µL* |
| *NaCl (0,5 M)* | *1 µL* |
| *1) Swa I (10 U*/*µL)* | *0,5 µL* |
| *2) EcoR V (10 U*/*µl)* | |
| *3) Sma I (10 U*/*µL)* | |
| *T4 DNA Ligase (5 U*/*µL)* | *0,5 µL* |
| *PCR-Produkt (aufgreinigt)* | *4 µL* |
| *1) target 1* | |
| *2) target 2* | |
| *3) target 3* | |

**Tabelle 3: Swa I / EcoR V / Sma I-Mix (5 µL pro Reaktion)**

| ***Komponenten*** | ***1x*** |
|---|---|
| *1) Swa I Puffer (10x)* | *0,5 µL* |
| *2) EcoR V Puffer (10x)* | |
| *3) Sma I Puffer (10x)* | |
| *NaCl (0,5 M)* | *1 µL* |
| *H₂O* | 3 *µL* |
| *1) Swa I (10 U*/*µL)* | *0,5 µL* |
| *2) EcoR V (10 U*/*µl)* | |
| *3) Sma I (10 U*/*µL)* | |

### A5. LR-Reaktion

Entsprechend ihrer Bestimmung wurden die Konstrukte mit einer oder drei Sequenzfragmenten (targets) mittels dem LR-Clonase-System aus dem Zwischenvektor pIPKTA38 (entry vector) in einen RNAi-Vektor übertragen. Als RNAi-Vektoren (destination vector) wurden pIPKb007 (ehemals p6UUbiRNAi), pIPKb010 (ehemals p6U-pGSTA1-RNAi) sowie p6UGLP4deltaSwaIntronRNAi (p6UGer4deltaSwaIntronRNAi) eingesetzt.

Eine graphische Darstellung der Komponenten aller drei Vektoren der *triple target* RNAi-Konstrukte ist im Anhang an dem Beispiel der Sequenz-Kombination aus GammaAktin, RhoGTPase und dem mitochondriale Hitzeschockprotein Chaperon 70 (GA-Rho-Hsp) dargestellt.

### Master-Mixes für 6-µL LR-Reaktionen

**Tabelle 4: LR-Master-Mix (5 µL Master-Mix + 1 µL pIPKTA38_target1_target2_target3 DNA pro Reaktion)**

| **Komponente** | **1x** | **Für 96 Proben** |
|---|---|---|
| *1) pIPK007 (150 ng*/*µL)* | *1 µL* | *100 µL* |
| *2) pIPK010 (150 ng*/*µL)* | | |
| *3) GER4 (p6UGLP4deltaSwaIntronRNAi) (150 ng*/*µL)* | | |
| *H₂O* | *3 µL* | *200 µL* |
| *LR Clonase Mix II* | *1 µL* | *80 µL* |
| | *5 µL Master mix pro well* | |
| *pIPKTA38_target1_traget2_target3* | *1 µL pro Reaktion* | |

Inkubation bei Raumtemperatur RT über Nacht (oder min. 6 h).

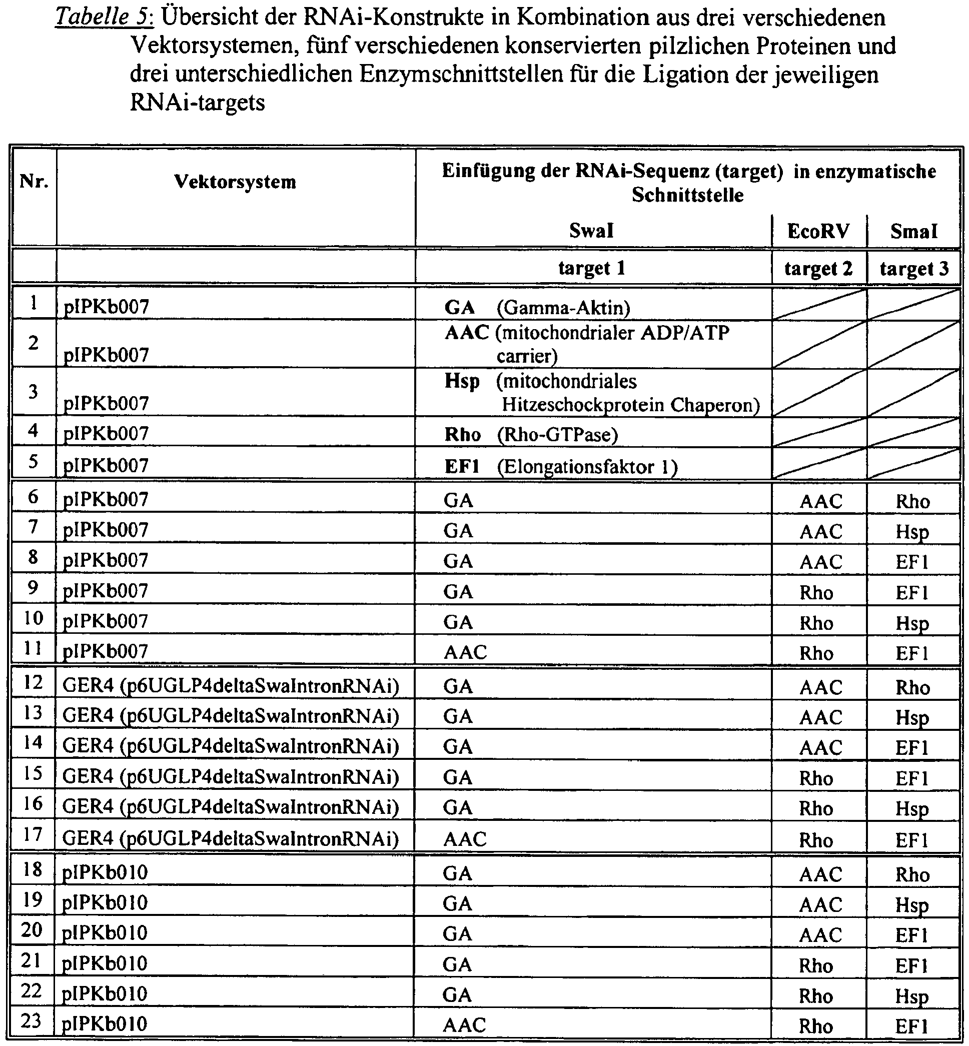

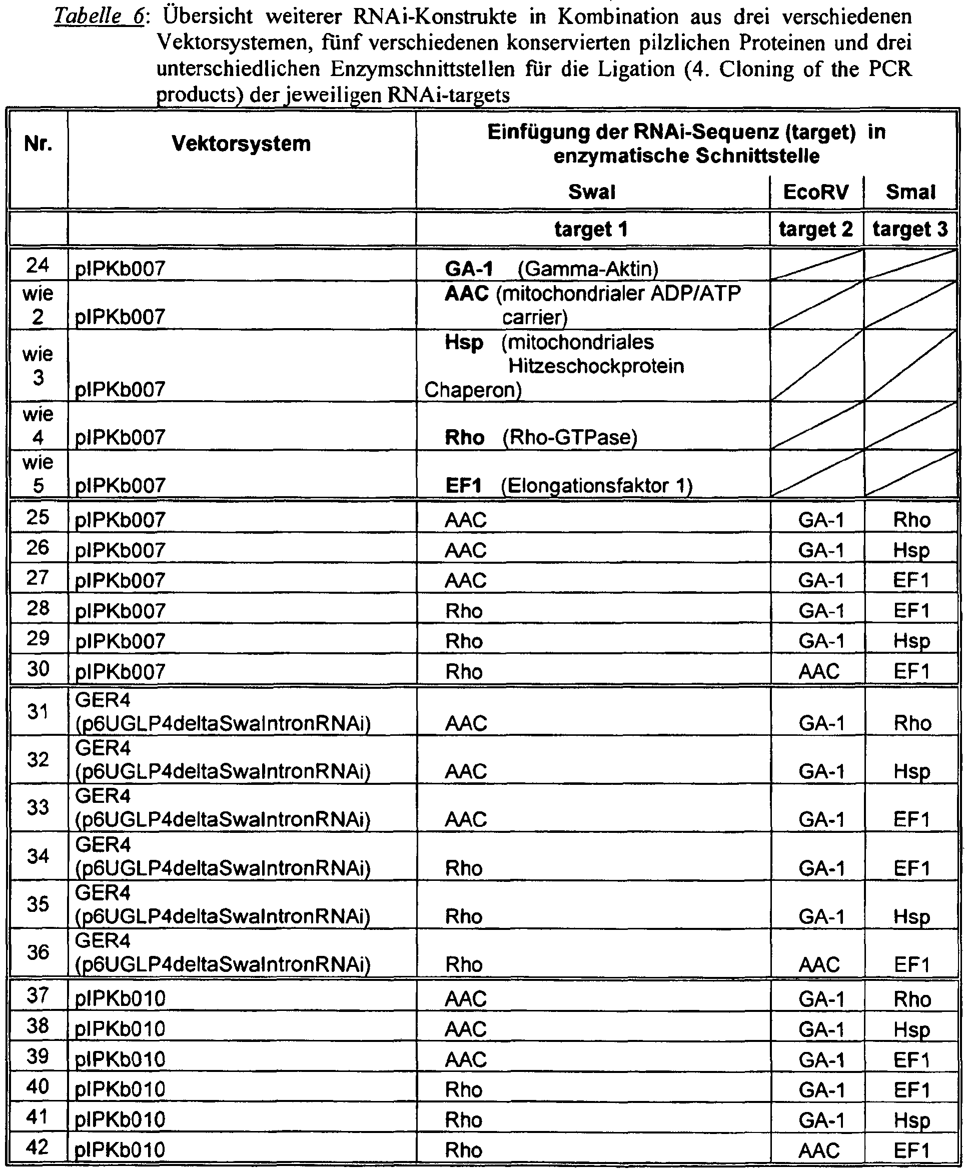

### A6. Materialien

Jetstar Plasmid Midi Prep Kit
   Genomed
   Cat. No. 210250 (250 midipreps)
NucleoSpin Robot-96 Plasmid Kit
   Macherey-Nagel
   Cat. No. 740708.4 (4x96 minipreps)
Qiagen MinElute UF 96-well
   Qiagen
   Cat. No. 2853 (4x96)
ThermalAce DNA Polymerase Kit
   Invitrogen
   Cat. No. E1000 (1000 U)
Library Efficient DB3.1 Competent cells
   Invitrogen
   Cat. No. 11782-018
Gateway® LR Clonase™ II enzyme mix
   Invitrogen
   Cat. No. 11791-100 (100 reactions)
T4 DNA ligase (5 U/µL)
   Fermentas
   Cat No. EL0331 (1000 U)
Swa I (10U/µL)
   New England BioLabs
   Cat. No. R0604S (2000 U)
EcoR V (10U/µL)
   Fermantas
   Cat. No. #ER0301 (2000 U)
Sma I (10U/µL)
   Fermantas
   Cat. No. #ER0661 (1200 U)

### Vektoren

*pIPKTA38 -* Gateway Entry vector, Kanamycin Resistenz.
pIPKb007 - binärer RNAi Vektor, Spectinomycin Resistenz. ccdB Negativ Selektionsmarker (benötigt DB3.1 cells), Chloramphenicol Resistenz (nicht getestet), Ubiqutin-Promoter.
pIPKb010 - binärer RNAi Vektor, Spectinomycin Resistenz. ccdB Negativ Selektionsmarker (benötigt DB3.1 cells), Chloramphenicol Resistenz (nicht getestet), GstA 1-Promoter.
GER4 (p6UGLP4deltaSwaIntronRNAi) - binärer RNAi Vektor, Spectinomycin Resistenz. ccdB Negativ Selektionsmarker (benötigt DB3.1 cells), Chloramphenicol Resistenz (nicht getestet), GER4-Promoter.

### B. TransGenTest und TIGS Protokoll

### Vorbereitung des Pflanzenmaterials:

Gerste wurde in IPK Getreideerde 7 Tage ohne Düngung in einem Sanyo Phytoschrank bei 20°C konstant und 60-70% relativer Luftfeucthe und einem 16 h Licht-Zyklus angezogen. Primärblätter (ca. 7 cm) wurden abgeschnitten und parallel angeordnet (adaxiale Seite nach oben)auf eine Phytoagar-Petrischale gelegt. Dabei wurden Magnetrührstäben auf die Blätter gelegt so dass sie sich abstoßen.

### Beschichtung der Goldpartikel mit DNA oder RNA:

Pro Schuss wurden 7 µl = 7 µg DNA (Plasmid) eingesetzt. Ein Bio-Rad Hepta-Adaptor (7 Macrocarrierscheiben) wurde verwendet.

| | |
|---|---|
| 7,0 µl | pUbiGUS (Reportergen) |
| 0,7 µl | pUbi-Mlo-nos (Resistenzaufhebung) |
| 7,0 µl | pIPK007_Mlo, pIPK010_Mlo, Ger4_Mlo (resistente Kontrolle) |
| **Summe:** 14,7 µl | |

Pro Schuss wurden zu 87,5 µl (Goldpartikel, 25 mg/ml in 50% Glycerol; Lagerung bei 4°C) Beschichtungssuspension wurden tröpfchenweise N µL 1 M Ca(NO₃)₂ pH 10 unter vortexen zugeben (N = Volumen DNA in µL). Die Partikelsuspension wurde mindestens für 10 min. bei Raumtemperatur stehengelassen und dabei gelegentlich angeschnippt. Die Suspension wurde zentrifugiert (15 sec, 14000 rpm) und der Überstand mit einer Pipette abgenommen und verworfen. Das Pellet wurde mit 500 µl Ethanol (70 %) gewaschen und der Ethanol mit einer Pipette abgehoben. Anschließend wurde das Pellet erneut mit 500 µl Ethanol (reinst) und anschließend in 30 µl Ethanol (reinst) resuspendiert.

### Makrocarrier-Beschichtung:

Die Zerreissscheiben und Makrocarrier wurden 30 sec. in Ethanol (reinst) eingelegt und dann bei Raumtemperatur getrocknet und mit einer Pinzette in den MakrocarrierHalter eingelegt. Die Tube mit Beschichtungssuspension (DNA/Partikelgemisches) wurde 3 sec. in ein Ultraschallbad gehalten und die Beschichtungssuspension anschließend mit einer Pipette gemischt. 3 µl Beschichtungssuspension wurden mit der Pipette auf jeden Makrocarrier auftragen und die Suspension wurde 2 min bis 5 min trocknen gelassen.

### Biolistische Transformation

Blätter und Makrocarrierhalter mit eingelegten Makrocarriern sowie **Gitter (Hepta Stop Screen)** wurden in die Kammer der eingelegt. Für die biolistische Transformation wurde ein Vakuum angelegt, wobei der Schuss bei 27,5 mm Hg Druck ausgelöst wurde.

### Inkubation und Inokulation der Blätter mit Mehltau:

Beschossene Blätter wurden zunächst 4 h in leicht geöffneten Petrischalen inkubiert. 24 h nach Beschuss wurden die Blätter in große, quadratische Petrischalen mit 1 % w/v Phytoagar mit 20 ppm Benzimidazol umgelegt. Dabei wurden Blätter aller Ansätze durchmischt. Zum Inokulieren wurden die Petrischalen offen in Schalen gelegt und Nylonnetze (100 µm Maschenweite) darüber gespannt. Die Blätter wurden mit Mehltau inokuliert (ca. 150 - 200 Konidien mm²). Zur Inokulation wurden möglichst frische Konidien verwenden, d.h. entweder von älteren Pflanzen die 24 h -48 h vor Inokulation geschüttelt wurden oder von frischen Pflanzen, die sieben Tage vorher inokuliert wurden. Anschließend wurden die Schalen in Inkubationskammer verbracht.

### GUS-Färbung (zur Anfärbung der transformierten Zellen):

48 h nach Inokulation wurden die Blätter eingesammelt, die Blattenenden abgeschnitten und die resultierenden Blätter in Greiner Röhrchen mit 10 ml X-Gluc.-Lösung überführt. Die Röhrchen wurden zur Vakuum-Infiltration in eine Saugflasche gestellt und 2-3 mal Vakuum angelegt. Die Infiltration ist vollständig wenn die Blätter durchsichtig werden und absinken. Die X-Gluc-Lösung wurde auf 14 ml aufgefüllt und die Röhrchen verschlossen. Die Röhrchen wurden über Nacht bei 37 °C im Brutschrank inkubiert.

### TCA -Färbung:

Die Blätter wurden 10 min. in Entfärbelösung gehaltlen (7.5% TCA, 50% Methanol). Und anschließend mit Aqua dest. gespült. Die Blätter wurden vorsichtig aus dem Röhrchen entnommen und mit der adaxialen Seite nach oben auf Objektträger gelegt. Pro Objektträger wurde 200 µl Aqua dest zugeben und das Deckglas vorsichtig aufgelegt.

### Lösungen:

### GUS-Färbelsg.(X-Gluc.-Lsg.):

100 mM Na-Phosphat , End.-pH 7,0
10 mM Na-EDTA
1,4 mM K-hexacyanoferrat(II)
1,4 mM K-hexacyanoferrat(III)
0, 1 % Triton X-100
20% Methanol
1 mg / ml X-Gluc.

### Ca(NO₃)₂

23,61 g Ca(NO₃)₂ auf 100 ml Millipore Wasser = 1 M
durch Zugabe von 0,1 M KOH ph 10 einstellen

### Benzimidazol-Stammlsg.

40 mg/ml Benzimidazol in Ethanol (reinst)

### Phytoagar

0,5-1 % (w/v) Agarose in Millipore Wasser mit 50 µl Benzimidazol-Stammlsg. (s.o.)

### Präparation der Goldsuspension:

27,5 mg Bio-Rad Goldpartikel (1 µm Durchmesser, Art. LLP892) wurden in 1 ml sterilem H₂O gevortext und für 20 sec. mit Ultraschall behandelt. Nach einer Zentrifugation (30 sec. 14.000 rpm) wurde der Überstand abpipettiert, die Partikel erneut in 1 ml sterilem H₂O gevortext und für 20 sec. mit Ultraschall behandelt und zentrifugiert (s.o.). Der Überstand wurde abgenommen und zum Pellet wurde 1 ml EtOH reinst gegeben und die Probe für und 20 sec. mit Ultraschall behandelt. Nach einer erneuten Zentrifugation (s.o.) wurde der Überstand abpipettiert und das Pellet mit offenem Deckel bei 40 °C für ca. 20 min im Thermomixer getrocknet. Nach Zugabe von 1 ml 50 % sterilem Glycerin wurde der Ansatz gevortext und mit Ultraschall behandelt bis das Pellet suspendiert war. Die Suspension wurde bei -20 C aufbewahrt.

### C. Versuchsablauf für Primärdatengewinnung

Zur Überprüfung der Resistenzwirkung der RNAi-Konstrukte wurden vor allem die *single* und *triple* Konstrukte in Kombination mit dem pIPKb007 Vektor in den transienten Experimenten verwendet.

Die verwendeten Gerstenpflanzen (*Hordeum vulgare,* Kultivar *'Ingrid BC mlo5'*) wurden in einem Phytoschrank (von Sanyo, bei 20°C konstant, 60-70% rel. LF, 16 h Licht) in Erde ohne Düngung angezogen. Am Tag des Beschusses waren die Pflanzen 7 Tage alt. Die Primärblätter wurden abgeschnitten, auf 0,5%igem Phytoagar mit 200 ppm Bezimidazol gelegt und mit 2,2 mg Goldpartikel, die mit einer Mischung aus 7µg Reportergen-Vektor (pUbiGUS), 0,7µg pUbi-Mlo-nos (Resistenzaufhebung) und 7µg eines Kontrollvektors (pIPKb007_MLO, pIPKb010_MLO oder Ger4_MLO (resistente Kontrolle) entsprechend der zu testenden RNAi-Konstrukte) oder einem RNAi-Konstrukt beschichtet waren, beschossen. In geschlossenen Petrischalen wurden die Blätter bis zur Inokulation bei 20°C an einem Nordfenster gelagert.

Einen Tag nach Beschuss wurden die Blätter auf 1%igem Phytoagar mit 2% Benzimidazol umgelegt. Sie wurden mit einem Nylonnetz (Maschenweite 200µm) überspannt und mit einer Konidiendichte von ungefähr 200 Konidien/mm² inokuliert. Die Konidien (von dem Pathogen *Blumeria graminis hordei*) stammten von Gerstenpflanzen (Kultivar '*Golden Promise'*)*,* die 6-7 Tagen zuvor inokuliert wurden. Bis zur GUS-Färbung wurden die Blätter in geschlossenen Petrischalen, die zur Belüftung Löcher im Deckel hatten, bei 20°C an einem Nordfenster gelagert.

Ca. 48 h nach Inokulation wurde die GUS-Färbung durchgeführt. Diese wurde nach 20 h durch Inkubation in 7,5% Trichloressigsäure, 50% V/V Methanol abgestoppt und die Blätter gebleicht.

Jeder Versuch enthielt 3-4 Parallelschüsse zu je 7 Blattsegmenten der HIGS Negativkontrolle (jeweiliger leerer Vektor). Ferner enthielt jedes Experiment 2 Parallelschüsse einer TIGS Positivkontrolle (pIPKb007_MLO, pIPKb010_MLO, Ger4_MLO, bewirkt Resistenz durch Unterdrückung des *Mlo* Gens der Gerste). Die Daten pro Experiment basieren auf dem Vergleich des Effektes der Testkonstrukte mit dem Mittelwert der 4 Negativkontrollen des jeweiligen Versuches.

Bei allen Versuchsgliedern der Versuche in Ingrid BC mlo5 wurde pUbi-MLO-nos mitgeschossen, welches eine Aufhebung der Resistenz der Ingrid BCmlo5 Zellen bewirkt. Dies erlaubt die Analyse der Haustorienentwicklung als auch der Hyphenentwicklung, da Hyphen nur von transformierten Epidermiszellen auswachsen können und somit nicht mit Hyphen von benachbarten Infektionsereignissen auf nicht-transformierten Zellen vermischen.

### D. Anwendungsbeispiele

### D1 Ergebnis des HIGS Screenings

Für die transienten Experimente wurden Gerstenblätter der Sorte Ingrid BC mlo5 (*Hordeum vulgare*) verwendet. Als Inokulum dienten Sporen des Phytopathogens *Blumeria graminis* f. sp. *hordei* (echter Gerstenmehltau). In diesem System wurden alle *single* und *triple target* RNAi-Konstrukte in Kombination mit dem Vektor pIPKb007 getestet. Es konnte in diesen Versuchen gezeigt werden, dass bei sechs der getesteten Konstrukte ein signifikanter befallsreduzierender Effekt nachweisbar ist (s. Tab. 8). Zur Kontrolle der Funktionstüchtigkeit des Systems sowie als Maß der Resistenzwirkung ist ein Konstrukt mit einer bekannten resistenten Mlo-Sequenz (ubi_mlo) mitgeführt worden, welche über alle Experimente mit einer sehr hohen Signifikanz (p≤0.0001) eine deutliche Reduzierung des Haustorien-Indexes aufweist.Die Resultate zeigen, dass RNAi Konstrukte, die gegen hochkonservierte Targetgene eines Pilzes (hier: *Fusarium culmorum*) gerichtet sind gegen einen anderen Pilz (hier: *Blumeria graminis*) wirksam sind.

**Tabelle 6: Mittelwerte, Standardabweichungen und p-Werte signifikanter Konstrukte aus transienten Experimenten unter Verwendung von Ingrid mlo5 (Hordeum vulgare) und dem Pathogen Blumeria graminis f. sp. hordei**

| **Konstrukt** (Nr. vergl. Tab.5) (repeats) | pIPKb007 (leere Vektor) | pIPKb007 _mlo | **2** AAC | **3** Hsp | **8** AAC-GA-EF1 | **9** Rho-GA-EF1 | **10** Rho-GA-Hsp | **11** Rho-AAC·EF1 |
|---|---|---|---|---|---|---|---|---|
| **n1** (Anzahl ausgewerteter Blätter) | 84 | 64 | 23 | 27 | 29 | 28 | 30 | 22 |
| **n2** (Anzahl ausgewerteter GUS-Zellen) | 5967 | 4141 | 1419 | 1598 | 1413 | 1533 | 1533 | 1185 |
| **Mittelwert in** % (des Haustorien-Indexes* in Relation zum leeren Ubi-Vektor (=100%)) | 100 | 41.88 | 56.03 | 78.02 | 82.62 | 74.93 | 72.65 | 69.14 |
| **Standardabweichun g** | * | 36.97 | 33.50 | 41.67 | 44.70 | 38.66 | 48.93 | 51.52 |
| **p-Wert** aus t-test, two tailed | * | <0.0001 | <0.0001 | 0.011 | 0.0455 | 0.002 | 0.0047 | 0.0105 |

| | | | | | | | | |
|---|---|---|---|---|---|---|---|---|
| * der Haustorien-Index entspricht dem Verhältnis zwischen der Anzahl gebildeter Haustorien zu der Anzahl der ausgewerteten GUS-Zellen | | | | | | | | |

### D2. Herstellung transgener Gerstenpflanzen

Mit den oben beschriebenen Vektoren bzw. RNAi-Konstrukten wurden mittels *Agrobacterium*-Methodik transgene Gerstenpflanzen hergestellt. Transgene Pflanzen wurden mittels Hygromycin-Resistenz selektioniert. Folgende Konstrukte wurde verwendet:

| ***Vektor** (Promotor)* | ***RNAi-Insert(s)*** |
|---|---|
| **pIPKb007**(Ubiquitin) | AAC |
| | GA - AAC - Rho |
| | GA - AAC - Hsp |
| | GA - AAC - EF1 |
| | GA - Rho - EF1 |
| | GA - Rho - Hsp |
| | AAC - Rho - EF1 |
| | Leerer Vektor |
| **pIPKb010** (GSTA1) | GA - Rho - Hsp |
| | AAC - Rho - EF1 |
| | Leerer Vektor |
| | |
| | |
| **p6UGLP4deltaSwaIntronRNAi** (GER4) | GA - Rho - Hsp |
| | AAC - Rho - EF1 |
| | Leerer Vektor |

Bei ersten Inokulationsexperimenten zeigten Pflanzen transgen für pIPKb007 GA-AAC-Hsp und pIPKb007 GA-AAC-Rho, verglichen mit Wildtyp-Pfanzen, eine Resistenz gegenüber *Fusarium culmorum* und *Blumeria graminis.*

### D3. Wirkung eines RNAi Konstruktes gegen Gerstenmehltau und Ährenfusariose in transgenen Pflanzen

| | Fusarium assay (% infizierte Blüten) | | Mehltau assay (% infizierte Blattfläche, rel. zu GP) | |
|---|---|---|---|---|
| Ansatz | MW±SDM | p | MW±SDM | p |
| GP (wt) | 5,1±0,5 | | 100 | |
| K26 transgen | 1,57±0,5 | 0,0256 | 74.8±9.3 | 0.0092 |
| K26 azygot | 3,45±2,56 | NS | 122,8±24,4 | NS |

Vier unabhängige transgene Gerstenlinien, die mit Konstrukt Nr. 26 (K26) transformiert wurden, zeigten erhöhte Resistenz sowohl gegen Ährenfusariose als auch gegen Gerstenmehltau. Azygote Segreganten der untersuchten transgenen Linien (T1 Generation) zeigten keinen signifikanten Resistenz-Phänotyp.

### D4. Sequenzen der Vektoren und triple target RNAi-Konstrukte

Im Folgenden werden die Sequenzen der *triple* RNAi-*targets* aufgeführt, welche mittels der oben beschriebenen GATEWAY-Methode in die verschiedenen Vektoren eingefügt werden. Die GATEWAY-Schnittstellen zu den binären RNAi-Vektoren sind bei allen 6 verschiedenen dreier Kombinationen (siehe Tabelle 6) farblich markiert.

Die Sequenzen sowie die Komponenten der drei verschiedenen Vektoren (pIKPb007, pIPKb010, GER4 (p6UGLP4deltaSwaIntronRNAi)) sind als Genbank-File angehängt.

Als Beispiel für die *triple target* RNAi-Konstrukte ist die Kombination aus Gamma Aktin, RhoGTPase und mitochondrialem Hitzeschockprotein 70 Chaperon in allen drei Vektorsystemen schematisch dargestellt.

_Farbmarkierung innerhalb der Insert-Sequenzen:
GATEWAY-Schnittstelle
**EcoRV-Side**
Swal-Side
SmaI-Side

targets aus den konservierten Proteinen:
**GA**
**AAC**
**Rho**
**EF1**
**Hsp**

**>AAC-Insert**

GTACAAAAAGCAGGCTTTAAAGGAACCAATTCAGTCGACCTCGAGCA
CCTGCGCGGCCGCGAATTCGATATCATTTCTTGTAGACGTCGACGAGAC
CGTTGAACTGACGGTCACCACCGGACTTGGCGTTCTTGGCATCGTTGG
CAAGACGAGTACGGGCGTAGTCCAGAGAGTAGACGAAGAGCAGAGA
AGTGGCACCAGCAGCACCACCGGAGGCAAGGTTACCAGCCATCCACA
TGGCGTAGCCATCCTTGTCCTTCTTGTAGCCGAACATCTTCTTGAACTT
GTCACGGAAAGCGAAGTTCAGGGCCTGGGTGGGGAAGTATCGGATGA
CGTTGGCGGTGTTTCCTCGCCAGAGGGACATGACACCCTCATCGAAAT
GCCCGGGCGAATTCTCTAGACCCAGCTTT

Die obenstehende Sequenz des AAC-siRNA Inserts ist in antisense-Orientierung (3'-5'), die komplementäre "Sense"-Sequenz (5'-3') entspricht SEQ ID Nr.6.

### >EF1-Insert

GTACAAAAAAGCAGGCTTTAAAGGAACCAATTCAGTCGACCTCGAGCA
CCTGCGCGGCCGCGAATTCGATATCATTTGCAGGCAATGTGGGCAGTG
TGGCAATCGAGGACGGGAGCGTAACCAGCACCGACCTGACCGGGGT
GGTTGAGGACGATGACCTGGGCGGTGAAAGAAGCGGCACCCATGGG
GGGGGTCGTTCTTGGAGTCACCAGCGACGTTACCACGTCGGATGTCCT
TGACGGAAACGTTCTTCACGTTGAAACCAACGTTGTCACCGGGCTGTC
CCTCAGTGAGCTGCTCGTGGTGCATCTCAACGGACTTGACTTCAGTGG
TGACGTTGGAAGGAGCGAAGGTAACGACCATACCGGGCTTGATAAAT
GCCCGGGCGAATTCTCTAGACCCAGCTTT

Die obenstehende Sequenz des EF1-siRNA Inserts ist in antisense-Orientierung (3'-5'), die komplementäre "Sense"-Sequenz (5'-3') entspricht SEQ ID Nr.7.

### >GA-Insert

GTACAAAAAAGCAGGCTTTAAAGGAACCAATTCAGTCGACCTCGAGCA
CCTGCGCGGCCGCGAATTCGATATCATTTGGAAGAAGGAGCAAGAGCA
GTGATCTCCTTCTGCATACGGTCGGAGAGACCGGGGTACATGGTGGT
ACCACCAGACATGACAATGTTTCCGTAGAGATCCTTTCGGACATCGA
CATCACACTTCATGATGGAGTTGAAAGTGGTGACGTGGATACCACCG
CTCTCAAGACCAAGGACAGAAGGCTGGAAGAGAGCCTCAGGAGCAC
GGAATCGCTCGTTACCAATGGTGATAACCTGACCGTCAGGAAGCTCG
TAAGACTTCTCCAAGCTGGAGCTCTGGGCAGCAGTCTGGATCTCCTG
CTCGAAAATGCCCGGGCGAATTCTCTAGACCCAGCTTT

Die obenstehende Sequenz des GA-siRNA Inserts ist in antisense-Orientierung (3'-5'), die komplementäre "Sense"-Sequenz (5'-3') entspricht SEQ ID Nr.8.

### >GA-1-Insert

GTACAAAAAAGCAGGCTTTAAAGGAACCAATTCAGTCGACCTCGAGCA
CCTGCGCGGCCGCTAATTCGATATCATTTGGAAGAAGGAGCAAGAGCA
GTGATCTCCTTCTGCATACGGTCGGAGAGACCGGGGTACATGGTGGT
ACCACCAGACTAAAGACATGTTAGAACAGTTGGAGTGCTTTCGATTT
GCAGAACTTACCATGACAATGTTTCCGTAGAGATCCTTTCGGACATC
GACATCACACTTCATGATGGAGTTGAAAGTGGTGACGTGGATACCAC
CGCTCTCAAGACCAAGGACAGAAGGCTGGAAGAGAGCCTCAGGAGC
ACGGAATCGCTCGTTACCAATGGTGATAACCTGACCGTCAGGAAGCT
CGTAAGACTTCTCCAAGCTGGAGCTCTGGGCAGCAGTCTGGATCTCC
TGCTCGAAAAATGCCCGGGCGAATTCTCTAGACCCAGCTTT

Die obenstehende Sequenz des GA-1-siRNA Inserts ist in antisense-Orientierung (3'-5'), die komplementäre "Sense"-Sequenz (5'-3') entspricht SEQ ID Nr.9.

### >Hsp-Insert

GTACAAAAAAGCAGGCTTTAAAGGAACCAATTCAGTCGACCTCGAGCA
CCTGCGCGGCCGCGAATTCGATATCATTTACTTGCCCATGAGGTTGTTG
TCCTTGGTGCGCTGGCGCTCACCCTCGTAGACCTGGATAAGGACACC
AGGCTGGTTGTCGGAGAAGGTAGAGAAGACCTCGGACTTCTTGGTGG

GAATGGTGGTGTTGCGGGGGATGAGCTTGGTCATCATACCACCAGCG
GTCTCGATACCGAGAGAGAGGGGGGCGACGTCGAGGAGCAGAATCT
CGTTGGTGGCCTTGCTAGAGGTGTCACCAGAGAGAATAGCAGCCTGG
ACAGCGGCACCGTAGGCAACAGCCTCATCAGGATTGATGGACTTGTT
GGGCTCCTTTCCGTTGAAGTAGTCGGTGATGAGCTTCTGGACACGGGG
GATACGGAAATGCCCGGGCGAATTCTCTAGACCCAGCTTT

Die obenstehende Sequenz des Hsp-siRNA Inserts ist in antisense-Orientierung (3'-5'), die komplementäre "Sense"-Sequenz (5'-3') entspricht SEQ ID Nr.10.

### >Rho-Insert

GTACAAAAAAGCAGGCTTTAAAGGAACCAATTCAGTCGACCTCGAGCA
CCTGCGCGGCCGCGAATTCGATATCATTTTGCTTGTGGTAAAACCTGTTT
GTTGATCGTTTTCTCCAAGGGCACTTTCCCCGAGGTCTACGTCCCCAC
CGTCTTCGAGAACTATGTCGCCGATGTCGAGGTTGACGGCAAGCACG
TCGAGCTCGCCCTATGGGATACTGCTGGTCAGGAGGATTACGACCGT
CTTCGACCTCTCTCTTACCCCGACTCCCACGTTATCTTGATCTGCTTCG
CTGTTGACTCTCCCGACTCTCTCGACAACGTCCAGGAGAAGTGGATCT
CTGAGGTTCTGCACTTCTGCCAGGGKCTCCCTATCATCCTTGTCGGCT
AAATGCCCGGGCGAATTCTCTAGACCCAGCTTT

Die obenstehende Sequenz des Rho-siRNA Inserts ist in antisense-Orientierung (3'-5'), die komplementäre "Sense"-Sequenz (5'-3') entspricht SEQ ID Nr.11.

### >GA_AAC_Rho-Insert

GTACAAAAAGCAGGCTTTAAAGGAACCAATTCAGTCGACCTCGAGCA
CCTGCGCGGCCGCGAATTCGATCTTGTAGACGTCGACGAGACCGTTGA
ACTGACGGTCACCACCGGACTTGGCGTTCTTGGCATCGTTGGCAAGAC
GAGTACGGGCGTAGTCCAGAGAGTAGACGAAGAGCAGAGAAGTGGC
ACCAGCAGCACCACCGGAGGCAAGGTTACCAGCCATCCACATGGCGT
AGCCATCCTTGTCCTTCTTGTAGCCGAACATCTTCTTGAACTTGTCACG
GAAAGCGAAGTTCAGGGCCTGGGTGGGGAAGTATCGGATGACGTTGG
CGGTGTTTCCTCGCCAGAGGGACATGACACCCTCATCGATCATTTGGA
AGAAGGAGCAAGAGCAGTGATCTCCTTCTGCATACGGTCGGAGAGA
CCGGGGTACATGGTGGTACCACCAGACATGACAATGTTTCCGTAGAG
ATCCTTTCGGACATCGACATCACACTTCATGATGGAGTTGAAAGTGG
TGACGTGGATACCACCGCTCTCAAGACCAAGGACAGAAGGCTGGAA
GAGAGCCTCAGGAGCACGGAATCGCTCGTTACCAATGGTGATAACCT

GACCGTCAGGAAGCTCGTAAGACTTCTCCAAGCTGGAGCTCTGGGCA
GCAGTCTGGATCTCCTGCTCGAAAATGCCCTGCTTGTGGTAAAACCTG
TTTGTTGATCGTTTTCTCCAAGGGCACTTTCCCCGAGGTCTACGTCCCC
ACCGTCTTCGAGAACTATGTCGCCGATGTCGAGGTTGACGGCAAGCA
CGTCGAGCTCGCCCTATGGGATACTGCTGGTCAGGAGGATTACGACC
GTCTTCGACCTCTCTCTTACCCCGACTCCCACGTTATCTTGATCTGCTT
CGCTGTTGACTCTCCCGACTCTCTCGACAACGTCCAGGAGAAGTGGAT
CTCTGAGGTTCTGCACTTCTGCCAGGGKCTCCCTATCATCCTTGTCGG
CTGGGCGAATTCTCTAGACCCAGCTTT

### >GA_AAC_mHspCh-Insert

GTACAAAAAAGCAGGCTTTAAAGGAACCAATTCAGTCGACCTCGAGCA
CCTGCGCGGCCGCGAATTCGATCTTGTAGACGTCGACGAGACCGTTGA
ACTGACGGTCACCACCGGACTTGGCGTTCTTGGCATCGTTGGCAAGAC
GAGTACGGGCGTAGTCCAGAGAGTAGACGAAGAGCAGAGAAGTGGC
ACCAGCAGCACCACCGGAGGCAAGGTTACCAGCCATCCACATGGCGT
AGCCATCCTTGTCCTTCTTGTAGCCGAACATCTTCTTGAACTTGTCACG
GAAAGCGAAGTTCAGGGCCTGGGTGGGGAAGTATCGGATGACGTTGG
CGGTGTTTCCTCGCCAGAGGGACATGACACCCTCATCGATCATTTGGA
AGAAGGAGCAAGAGCAGTGATCTCCTTCTGCATACGGTCGGAGAGA
CCGGGGTACATGGTGGTACCACCAGACATGACAATGTTTCCGTAGAG
ATCCTTTCGGACATCGACATCACACTTCATGATGGAGTTGAAAGTGG
TGACGTGGATACCACCGCTCTCAAGACCAAGGACAGAAGGCTGGAA
GAGAGCCTCAGGAGCACGGAATCGCTCGTTACCAATGGTGATAACCT
GACCGTCAGGAAGCTCGTAAGACTTCTCCAAGCTGGAGCTCTGGGCA
GCAGTCTGGATCTCCTGCTCGAAAATGCCCCTCACCCTCGTAGACCTG
GATAAGGACACCAGGCTGGTTGTCGGAGAAGGTAGAGAAGACCTCGG
ACTTCTTGGTGGGAATGGTGGTGTTGCGGGGGATGAGCTTGGTCATCA
TACCACCAGCGGTCTCGATACCGAGAGAGAGGGGGGCGACGTCGAG
GAGCAGAATCTCGTTGGTGGCCTTGCTAGAGGTGTCACCAGAGAGAA
TAGCAGCCTGGACAGCGGCACCGTAGGCAACAGCCTCATCAGGATTG
ATGGACTTGTTGGGCTCCTTTCCGTTGAAGTAGTCGGTGATGAGCTTCT
GGGGGCGAATTCTCTAGACCCAGCTTT

### >GA_AAC_EF1-Insert

TACAAAAAAGCAGGCTTTAAAGGAACCAATTCAGTCGACCTCGAGCAC
CTGCGCGGCCGCGAATTCGATCTTGTAGACGTCGACGAGACCGTTGAA
CTGACGGTCACCACCGGACTTGGCGTTCTTGGCATCGTTGGCAAGAC
GAGTACGGGCGTAGTCCAGAGAGTAGACGAAGAGCAGAGAAGTGGC
ACCAGCAGCACCACCGGAGGCAAGGTTACCAGCCATCCACATGGCGT
AGCCATCCTTGTCCTTCTTGTAGCCGAACATCTTCTTGAACTTGTCACG

GAAAGCGAAGTTCAGGGCCTGGGTGGGGAAGTATCGGATGACGTTGG
CGGTGTTTCCTCGCCAGAGGGACATGACACCCTCATCGATCATTTGGA
AGAAGGAGCAAGAGCAGTGATCTCCTTCTGCATACGGTCGGAGAGA
CCGGGGTACATGGTGGTACCACCAGACATGACAATGTTTCCGTAGAG
ATCCTTTCGGACATCGACATCACACTTCATGATGGAGTTGAAAGTGG
TGACGTGGATACCACCGCTCTCAAGACCAAGGACAGAAGGCTGGAA
GAGAGCCTCAGGAGCACGGAATCGCTCGTTACCAATGGTGATAACCT
GACCGTCAGGAAGCTCGTAAGACTTCTCCAAGCTGGAGCTCTGGGCA
GCAGTCTGGATCTCCTGCTCGAAAATGCCCGCAGGCAATGTGGGCAG
TGTGGCAATCGAGGACGGGAGCGTAACCAGCACCGACCTGACCGGG
GTGGTTGAGGACGATGACCTGGGCGGTGAAAGAAGCGGCACCCATG
GGGGGGGTCGTTCTTGGAGTCACCAGCGACGTTACCACGTCGGATGT
CCTTGACGGAAACGTTCTTCACGTTGAAACCAACGTTGTCACCGGGCT
GTCCCTCAGTGAGCTGCTCGTGGTGCATCTCAACGGACTTGACTTCAG
TGGTGACGTTGGAAGGAGCGAAGGTAACGACCATACCGGGCTTGATG
GGCGAATTCTCTAGACCCAGCTTT

### >GA_Rho_EF1-Insert

GTACAAAAAGCAGGCTTTAAAGGAACCAATTCAGTCGACCTCGAGCA
CCTGCGCGGCCGCGAATTCGATTCGGCTGTTCCTACTATCCCTCKGGGA
CCGTCTTCACGTCTTGGAGTCTCTAGGTGAAGAGGACCTGCAACAGCT
CTCTCAGCCCTCTCAGTTGTCGCTTCGTCTAGTTCTATTGCACCCTCAG
CCCCATTCTCTCTCCAGCTTCTGCCAGCATTAGGAGGACTGGTCGTCA
TAGGGTATCCCGCTCGAGCTGCACGAACGGCAGTTGGAGCTGTAGCG
GCTGTATCAAGAGCTTCTGCCACCCCTGCATCTGGAGCCCCTTTCACG
GGAACCTCTTTTGCTAGTTGTTTGTCCAAAATGGTGTTCGTATCATTTG
GAAGAAGGAGCAAGAGCAGTGATCTCCTTCTGCATACGGTCGGAGA
GACCGGGGTACATGGTGGTACCACCAGACATGACAATGTTTCCGTAG
AGATCCTTTCGGACATCGACATCACACTTCATGATGGAGTTGAAAGT
GGTGACGTGGATACCACCGCTCTCAAGACCAAGGACAGAAGGCTGG
AAGAGAGCCTCAGGAGCACGGAATCGCTCGTTACCAATGGTGATAAC
CTGACCGTCAGGAAGCTCGTAAGACTTCTCCAAGCTGGAGCTCTGGG
CAGCAGTCTGGATCTCCTGCTCGAAAATGCCCGCAGGCAATGTGGGC
AGTGTGGCAATCGAGGACGGGAGCGTAACCAGCACCGACCTGACCG
GGGTGGTTGAGGACGATGACCTGGGCGGTGAAAGAAGCGGCACCCA
TGGGGGGGGTCGTTCTTGGAGTCACCAGCGACGTTACCACGTCGGAT
GTCCTTGACGGAAACGTTCTTCACGTTGAAACCAACGTTGTCACCGGG
CTGTCCCTCAGTGAGCTGCTCGTGGTGCATCTCAACGGACTTGACTTC
AGTGGTGACGTTGGAAGGAGCGAAGGTAACGACCATACCGGCTTGA
TGGGCGAATTCTCTAGACCCAGCTTT

### >GA_Rho_mHspCh-Insert

GTACAAAAAGCAGGCTTTAAAGGAACCAATTCAGTCGACCTCGAGCA
CCTGCGCGGCCGCGAATTCGATAGCCGACAAGGATGATAGGGAGMCC
CTGGCAGAAGTGCCAGAACCTCAGAGATCCACTTCTCCTGGACGTTGTC
GAGAGAGTCGGGAGAGTCAACAGCGAAGCAGATCAAGATAACGTGG
GAGTCGGGGTAAGAGAGAGGTCGAAGACGGTCGTAATCCTCCTGACC
AGCACTATCCCATAGGGCGAGCTCGACGTGCTTGCCGTCAACCTCGA
CATGGGCGACATACTTCTCCAAGACGGTGGGGACGTAGACCTCGGGG
AAAGTGGCCTTGGAGAAAACGATCAACAAACAGGTTTTACCACAAGC
AATCATTTGGAAGAAGGAGCAAGAGCAGTGATCTCCTTCTGCATACG
GTCGGAGAGACCGGGGTACATGGTGGTACCACCAGACATGACAATG
TTTCCGTAGAGATCCTTTCGGACATCGACATCACACTTCATGATGGA
GTTGAAAGTGGTGACGTGGATACCACCGCTCTCAAGACCAAGGACA
GAAGGCTGGAAGAGAGCCTCAGGAGCACGGAATCGCTCGTTACCAA
TGGTGATAACCTGACCGTCAGGAAGCTCGTAAGACTTCTCCAAGCTG
GAGCTCTGGGCAGCAGTCTGGATCTCCTGCTCGAAAATGCCCCTCAC
CCTCGTAGACCTGGATAAGGACACCAGGCTGGTTGTCGGAGAAGGTA
GAGAAGACCTCGGACTTCTTGGTGGGAATGGTGGTGTTGCGGGGGAT
GAGCTTGGTCATCATACCACCAGCGGTCTCGATACCGAGAGAGAGGG
GGGCGACGTCGAGGAGCAGAATCTCGTTGGTGGCCTTGCTAGAGGTG
TCACCAGAGAGAATAGCAGCCTGGACAGCGGCACCGTAGGCAACAG
CCTCATCAGGATTGATGGACTTGTTGGGCTCCTTTCCGTTGAAGTAGTC
GGTGATGAGCTTCTGGGGGCGAATTCTCTAGACCCAGCTTT

### >AAC_Rho_EF1-Insert

GTACAAAAAAGCAGGCTTTAAAGGAACCAATTCAGTCGACCTCGAGCA
CCTGCGCGGCCGCGAATTCGATTGCTTGTGGTAAAACCTGTTTGTTGAT
CGTTTTCTCCAAGGGCAGTTTCCCCGAGGTCTACGTCCCCACCCTCTT
CGAGAACTATGTCGCCGATCTCGAGGTTGACCGCAAGCACCTCGAGC
TCGCCCTATGGGATACTGCTGGTCAGGAGGATTACGACCGTCTTCGAC
CTCTCTGTTACCCCGACTCCCACGTTATCTTGATCTGCTTCGCTGTTGA
CTCTCCCGACTCTCTCGACAACGTCCAGGAGAAGTGGATCTCTGAGGT
TCTGCACTTCTGCCAGGGKCTCCCTATCATCCTTGTCGGCTATCATTTC
TTGTAGACGTCGACGAGACCGTTGAACTGACGGTCACCACCGGACTT
GGCGTTCTTGGCATCGTTGGCAAGACGAGTACGGGCGTAGTCCAGAG
AGTAGACGAAGAGCAGAGAAGTGGCACCAGCAGCACCACCGGAGGC
AAGGTTACCAGCCATCCACATGGCGTAGCCATCCTTGTCCTTCTTGTA
GCCGAACATCTTCTTGAACTTGTCACGGAAAGCGAAGTTCAGGGCCTG
GGTGGGGAAGTATCGGATGACGTTGGCGGTGTTTCCTCGCCAGAGGG
ACATGACACCCTCATCGAAATGCCCATCAAGCCCGGTATGGTCGTTAC
CTTCGCTCCTTCCAACGTCACCACTGAAGTCAAGTCCGTTGAGATGCA
CCACGAGCAGCTCACTGAGGGACAGCCCGGTGACAACGTTGGTTTCA

ACGTGAAGAACGTTTCCGTCAAGGACATCCGACGTGGTAACGTCGCT
GGTGACTCCAAGAACGACCCCCCCATGGGTGCCGCTTCTTTCACCG
CCCAGGTCATCGTCCTCAACCACCCCGGTCAGGTCGGTGCTGGTTAC
GCTCCCGTCCTCGATTGCCACACTGCCCACATTGCCTGCGGGCGAATT
CTCTAGACCCAGCTTT

### >AAC GA-1 Rho-Insert

GTACAAAAAAGCAGGCTTTAAAGGAACCAATTCAGTCGACCTCGAGCACCTGCGCGG
CCGCGAATTCGATCTTGTAGACGTCGACGAGACCGTTGAACTGACGGTCACCACCGG
ACTTGGCGTTCTTGGCATCGTTGGCAAGACGAGTACGGGCGTAGTCCAGAGAGTAGA
CGAAGAGCAGAGAAGTGGCACCAGCAGCACCACCGGAGGCAAGGTTACCAGCCATCC
ACATGGCGTAGCCATCCTTGTCCTTCTTGTAGCCGAACATCTTCTTGAACTTGTCAC
GGAAAGCGAAGTTCAGGGCCTGGGTGGGGAAGTATCGGATGACGTTGGCGGTGTTTC
CTCGCCAGAGGGACATGACACCCTCATCGATCATTTGGAAGAAGGAGCAAGAGCAGT
GATCTCCTTCTGCATACGGTCGGAGAGACCGGGGTACATGGTGGTACCACCAGACTA
AAGACATGTTAGAACAGTTGGAGTGCTTTCGATTTGCAGAACTTACCATGACAATGT
TTCCGTAGAGATCCTTTCGGACATCGACATCACACTTCATGATGGAGTTGAAAGTGG
TGACGTGGATACCACCGCTCTCAAGACCAAGGACAGAAGGCTGGAAGAGAGCCTCAG
GAGCACGGAATCGCTCGTTACCAATGGTGATAACCTGACCGTCAGGAAGCTCGTAAG
ACTTCTCCAAGCTGGAGCTCTGGGCAGCAGTCTGGATCTCCTGCTCGAAAATGCCCT
GCTTGTCGGTAAAACCTGTTTGTTGATCGTTTTCTCCAAGGCACTTTCCCCGAGGTC
TACGTCCCCACCGTCTTCGAGAACTATGTCGCCGATGTCGAGGTTGACGGCAAGCAC
GTCGAGCTCGCCCTATGGGATACTGCTGGTCAGGAGGATTACGACCGTCTTCGACCT
CTCTCTTACCCCGACTCCCACGTTATCTTGATCTGCTTCGCTGTTGACTCTCCCGAC
TCTCTCGACAACGTCCAGGAGAAGTGGATCTCTGAGGTTCTGCACTTCTGCCAGGGK
CTCCCTATCATCCTTGTCGGCTGGCGAATTCTCTAGACCCAGCTTT

### >AAC_GA-1_mHspCh-Insert

GTACAAAAAAGCAGGCTTTAAAGGAACCAATTCAGTCGACCTCGAGCACCTGCGCGG
CCGCTAATTCGATTGTAGACGTCGACGAGACCGTTGAACTGACGGTCACCACCGGAC
TTGGCGTTCTTGGCATCGTTGGCAAGACGAGTACGGGCGTAGTCCAGAGAGTAGACG
AATAGAAGAGAAGTGGCACCAGCAGCACCACCGGAGGCAAGGTTACCGGCCATCCAC
ATGGCGTAGCCATCCTTGTCCTTCTTGTAACCGAACATCTTCTTGAACTTGTCACGG
AAAGCGAAGTTCAGAGCCTGGGTAGGGAAGTATCGGATGACGTTGGCGGTGTTTCCT
CGCCAGAGGGACATGACACCCTCATCGATCATTTGGAAGAAGGAGCAAGAGCAGTGA
TCTCCTTCTGCATACGGTCGGAGAGACCGGGGTACATGGTGGTACCACCAGACTAAA
GACATGTTAGAACAGTTGGAGTGCTTTCGATTTGCAGAACTTACCATGACAATGTTT
CCGTAGAGATCCTTTCGGACATCGACATCACACTTCATGATGGAGTTGAAAGTGGTG
ACGTGGATACCACCGCTCTCAAGACCAAGGACAGAAGGCTGGAAGAGAGCCTCAGGA
GCACGGAATCGCTCGTTACCAATGGTGATAACCTGACCGTCAGGAAGCTCGTAAGAC

TTCTCCAAGCTGGAGCTCTGGCAGCAGTCTGGATCTCCTGCTCGAAAAATGCCCTC
GTAGACCTGGATAAGGACACCAGGCTGGTTGTCGGAGAAAGTAGAGAAGACCTCGGA
CTTCTTGGTGGAATGGTGGTGTTGCGGGGATGAGCTTGGTCATCATACCACCAGC
GGTCTCGATACCGAGAGAGAGAGGGGCAACGTCGAGGAGCAGAATCTCGTTGGTGGC
CTTGCTAGAGGTGTCACCAGAGAGAATAGCAGCCTGGACAGCGGCACCGTAGGCAAC
AGCCTCATCAGGGTTGATGGACTTGTTGGGCTCCTTTCCGTTGAAGTAGTCGGTGAT
GAGCTTCTGGGGGCGAATTCTCTAGACCCAGCTTT

### >AAC_GA-1 EF1-Insert

GTACAAAAAAGCAGGCTTTAAAGGAACCAATTCAGTCGACCTCGAGCACCTGCGCGG
CCGCTAATTCGATTGTAGACGTCGACGAGACCGTTGAACTGACGGTCACCACCGGAC
TTGGCGTTCTTGGCATCGTTGGCAAGACGAGTACGGCGTAGTCCAGAGAGTAGACG
AATAGAAGAGAAGTGGCACCAGCAGCACCACCGGAGGCAAGGTTACCGGCCATCCAC
ATGGCGTAGCCATCCTTGTCCTTCTTGTAACCGAACATCTTCTTGAACTTGTCACGG
AAAGCGAAGTTCAGAGCCTGGGTAGGGAAGTATCGGATGACGTTGGCGGTGTTTCCT
CGCCAGAGGGACATGACACCCTCATCGATCATTTGGAAGAAGGAGCAAGAGCAGTGA
TCTCCTTCTGCATACGGTCGGAGAGACCGGGGTACATGGTGGTACCACCAGACTAAA
GACATGTTAGAACAGTTGGAGTGCTTTCGATTTGCAGAACTTACCATGACAATGTTT
CCGTAGAGATCCTTTCGGACATCGACATCACACTTCATGATGGAGTTGAAAGTGGTG
ACGTGGATACCACCGCTCTCAAGACCAAGGACAGAAGGCTGGAAGAGAGCCTCAGGA
GCACGGAATCGCTCGTTACCAATGGTGATAACCTGACCGTCAGGAAGCTCGTAAGAC
TTCTCCAAGCTGGAGCTCTGGGCAGCAGTCTGGATCTCCTGCTCGAAAAATGCCCGC
AGGCAATGTGGGCAGTGTGGCAATCGAGGACGGAGCGTAACCAGCACCGACCTGAC
CGGGGTGGTTGAGGACGATGACCTGGGCGGTGAAAGAAGCGGCACCCATGGGGGGT
CGTTCTTGGAGTCACCAGCGACGTTACCACGTCGGATGTCCTTGACGGAAACGTTCT
TCACGTTGAAACCAACGTTGTCACCGGGCTGGCCCTCAGTGAGCTGCTCGTGGTGCA
TCTCAACGGACTTGACTTCAGTGGTGACGTTGGAAGGAGCGAAGGTAACGACCATAC
CGGGCTTGATGGAATTCTCTAGACCCAGCTTT

### >Rho_GA-1_EF1-Insert

GTACAAAAAAGCAGGCTTTAAAGGAACCAATTCAGTCGACCTCGAGCACCTGCGCGG
CCGCGAATTCGATTGCTTGTGGTAAAACCTGTTTGTTGATCGTTTTCTCCAAGGCA
CTT[?]CCCCGAGGTCTACGTCCCCACCGTCTTCGAGAACTATGTCGCCGATGTCGAGG
TTGACGGCAAGCACGTCGAGCTCGCCCTATGGGATACTGCTGGTCAGGAGGATTACG
ACCCTCTTCGACCTCTCTCTTACCCCGACTCCCACGTTATCTTGATCTGCTTCGCTG
TTGACTCTCCCGACTCTCTCGACAACGTCCAGCAGAAGTGGATCTCTGAGGTTCTGC
ACTT[?][?]GCCACGG[?]CTCCCTA[?]CATCCTTGTCGGCTATCATTTGGAAGAAGGAGCAA
GAGCAGTGATCTCCTTCTGCATACGGTCGGAGAGACCGGGTACATGGTGGTACCAC
CAGACTAAAGACATGTTAGAACAGTTGGAGTGCTTTCGATTTGCAGAACTTACCATG
ACAATGTTTCCGTAGAGATCCTTTCGGACATCGACATCACACTTCATGATGGAGTTG
AAAGTGGTGACGTGGATACCACCGCTCTCAAGACCAAGGACAGAAGGCTGGAAGAGA
GCCTCAGGAGCACGGAATCGCTCGTTACCAATGGTGATAACCTGACCGTCAGGAAGC
TCGTAAGACTTCTCCAAGCTGGAGCTCTGGGCAGCAGTCTGGATCTCCTGCTCGAAA

ATGCCCGCAGGCAATGTGGGCAGTGTGGCAATCGAGGACGGGAGCGTAACCAGCACC
GACCTGACCGGGGTGGTTGAGGACGATGACCTGGCGGTGAAAGAAGCGGCACCCAT
GGGGGGGGTCGTTCTTGGAGTCACCAGCGACGTTACCACGTCGGATGTCCTTGACGG
AAACGTTCTTCACGTTGAAACCAACGTTGTCACCGGCTGTCCCTCAGTGAGCTGCT
CGTGGTGCATCTCAACGGACTTGACTTCAGTGGTGACGTTGGAAGGAGCGAAGGTAA
CGACCATACCGGGCTTGATGGGCGAATTCTCTAGACCCAGCTTT

### >Rho_GA-1_mHspCh-Insert

GTACAAAAAAGCAGGCTTTAAAGGAACCAATTCAGTCGACCTCGAGCACCTGCGCGG
CCGCTAATTCGATCAGCCGACAAGGATGATAGGAGACCCTGGCAGAAGTGCAGAAC
CTCAGAGATCCACTTCTCCTGAACGTTGTCGAGGAGTCGGAGAGTCAACGGCGAA
GCAGATCAAGATAACGTGGGAGTCGGGGTAAGAAAGAGGTCGAAGACGGTCGTAATC
CTCCTGACCAGCAGTATCCCATAGGGCGAGCTCGACGTGCTTGCCGTCAACCTCGAC
ATCGGCAACATAGTTCTCGAAGACGGTGGGGACGTAAACCTCGGCAAAGTGCCCTTG
GAGAAAACAATCAACAAACAGGTTTTACCACAAGCACCATCGATCATTTGGAAGAAG
GAGCAAGAGCAGTGATCTCCTTCTGCATACGGTCGGAGAGACCGGGTACATGGTGG
TACCACCAGACTAAAGACATGTTAGAACAGTTGGAGTGCTTTCGATTTGCAGAACTT
ACCATGACAATGTTTCCGTAGAGATCCTTTCGGACATCGACATCACACTTCATGATG
GAGTTGAAAGTGGTGACGTGGATACCACCGCTCTCAAGACCAAGGACAGAAGGCTGG
AAGAGAGCCTCAGGAGCACGGAATCGCTCGTTACCAATGGTGATAACCTGACCGTCA
GGAAGCTCGTAAGACTTCTCCAAGCTGGAGCTCTGGCAGCAGTCTGGATCTCCTGC
TCGAAAAATTGCCCTCGTAGACCTGGATAAGGACACCAGGCTGGGTGTCGGAGAAGG
TAGAGAAGACCTCGGACTTCTTGGTGGGAATGGTGGTGTTGCGGGGGATGAGCTTGG
TCATCATACCACCAGCGGTCTCGATACCGAGAGAGAGAGGGGCAACGTCGAGGAGCA
GAATCTCGTTGGTGGTCTTGCTAGAGGTGTCACCAGAGAGAATAGCAGCCTGGACAG
CGGCACCGTAGGCAACAGCCTCATCAGGGTTGATGGACTTGTTGGGCTCCTTTCCGT
TGAAGTAGTCGGTGATGAGCTTCTGGGGGCGAATTCTCTAGACCCAGCTTT

### >Rho ASC_EF1-Insert

GTACAAAAAGCAGGCTTTAAAGGAACCAATTCAGTCGACCTCGAGCACCTGCGCGG
CCGCTAATTCGATCGATGGTGCTTGTGGTAAAACCTGTTTGTTGATTGTTTTCTCCA
AGGGCACTTTCCCCGAGGTTTACGTCCCCACCGTCTTCGAGAACTATGTTGCCGATG
TCGAGGTTCACGGCAAGCACGTCGAGCTCGCCCTATGGGATACTGCTGGTCAGGAGG
ATTACGACCGTCTTCGACCTCTTTCTTACCCCGAATCTCAAGTTATCTTGATTTGCT
TCGCCGTTGACTCTTCCGACTCCCTCGACAACGTTCAGGAGAAGTGGATCTGTGAGG
TTCTGCACTTCTGCCAGGGTCTCCCTATCATCCTTGTCGGCTGATCATTTTTGTAGA
CGTCGACGAGACCGTTGAACTGACGGTCACCACCGGACTTGGCGTTCTTGGCATCGT
TGGCAAGACGAGTACGGGCGTAGTCCAGAGAGTAGACGAATAGAAGAGAAGTGGCAC
CAGCAGCACCACCGGAGGCAAGGTTACCGGCCATCCACATGGCGTAGCCATCCTTGT
CCTTCTTGTAACCGAACATCTTCTTGAACTTGTCACGGAAAGCGAAGTTCAGAGCCT
GGGTAGGGAAGTATCGGATGACGTTGGCGGTGTTTCCTCGCCAGAGGACATGACAC
CCTCATAAATGCCCATCAAGCCCGGTATGGTCGTTACCTTCGCTCCTTCCAACGTCA

CCACTGAAGTCAAGTCCGTTGAGATGCACCACGAGCAGCTCACTGAGGCCAGCCCG
GTGACAACGTTGGTTTCAACGTGAAGAACGTTTCCGTCAAGGACATCCGACGTGGTA
ACGTCGCTGGTGACTCCAAGAACGACCCCCCATGGGTGCCGCTTCTTTCACCGCCC
AGGTCATCGTCCTCAACCACCCCGGTCAGGTCGGTGCTGGTTACGCTCCCGTCCTCG
ATTGCCACACTGCCCACATTGCCTGGGGCGAATTCTCTAGACCCAGCTTT

### Beschreibung der Abbildungen

- Abb. 1:: Flussdiagramm zur Erzeugung von RNAi Konstrukten im Hochdurchsatz. I, PCR Amplifikation von cDNA Fragmenten interessierender Pilzgene; IIa, Ligation der PCR Fragmente in den Zwischenvektor pIPKTA38 in Anwesenheit der Restriktionsendonuklease *Swa*I*,* die Re-ligation des Vektors verhindert; IIb, Nachschneiden aller re-ligierter Vektormoleküle; III, Rekombination der klonierten cDNA Fragmente in den RNAi Vektor pIPKTA30 mittels LR Klonase.
- Abb. 2:: Schematische Darstellung des Syntheseweges von einfachen (single) sowie kombinierten (triple) RNAi-Kostrukten mittels der modifizierten GATEWAY-Technologie nach Douchkov et al. 2005 am Beispiel einer Sequenz aus Gamma-Aktin (*single RNAi-Konstrukt),* sowie einer Sequenzkombination aus drei Komponenten (Gamma Aktin, ADP/ATP carrier sowie RhoGTPase; *triple RNAi-Konstrukt).* Als binäre *RNAi-destination vectoren* sind pIPKb007 (ubi), p6UGER4deltaSwaIntronRNAi (Ger4) und pIPKb010 (GSTA1) verwendet worden.

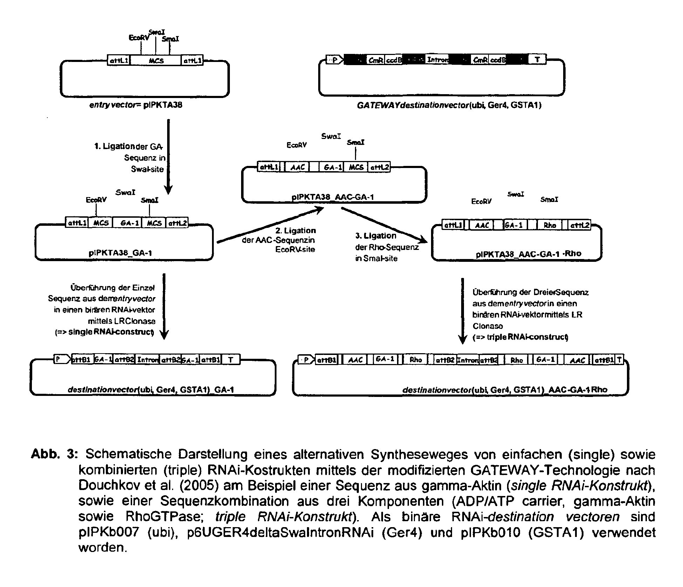

- Abb. 3:: Schematische Darstellung eines alternativen Syntheseweges von einfachen (single) sowie kombinierten (triple) RNAi-Kostrukten mittels der modifizierten GATEWAY-Technologie nach Douchkov et al. 2005 am Beispiel einer Sequenz aus Gamma-Aktin (*single RNAi-Konstrukt*), sowie einer Sequenzkombination aus drei Komponenten (Gamma Aktin, ADP/ATP carrier sowie RhoGTPase; *triple RNAi-Konstrukt).* Als binäre *RNAi-destination vectoren* sind pIPKb007 (ubi), p6UGER4deltaSwalntronRNAi (Ger4) und pIPKb010 (GSTA1) verwendet worden.

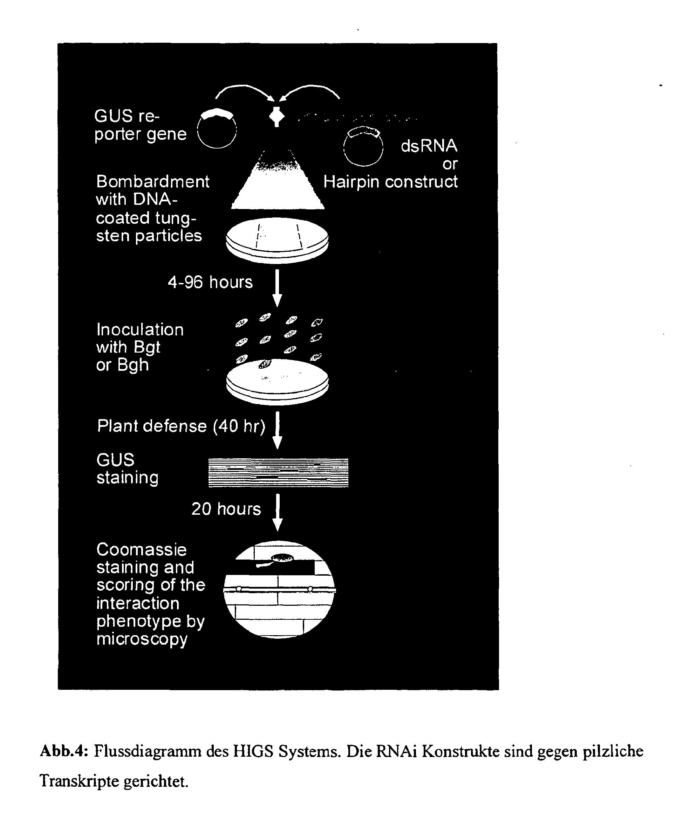

- Abb. 4:: Flussdiagramm des HIGS Systems. Die RNAi Konstrukte sind gegen pilzliche Transkripte gerichtet.

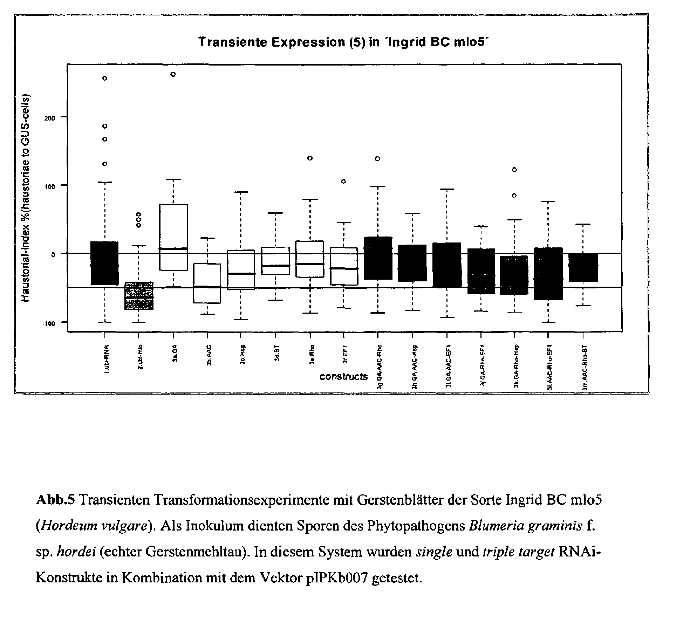

- Abb. 5:: Transienten Transformationsexperimente mit Gerstenblätter der Sorte Ingrid BC mlo5 (*Hordeum vulgare*). Als Inokulum dienten Sporen des Phytopathogens *Blumeria graminis* f. sp. *hordei* (echter Gerstenmehltau). In diesem System wurden *single* und *triple target* RNAi-Konstrukte in Kombination mit dem Vektor pIPKb007 getestet.

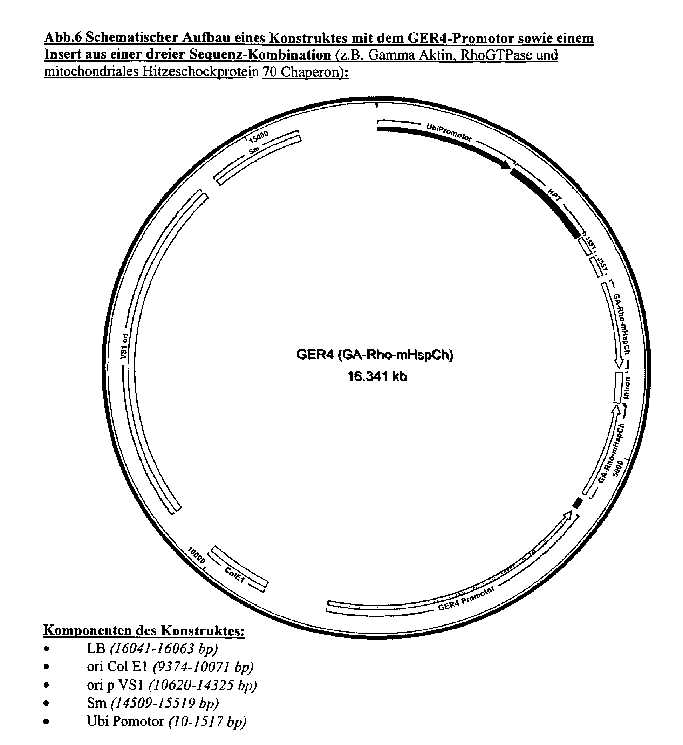

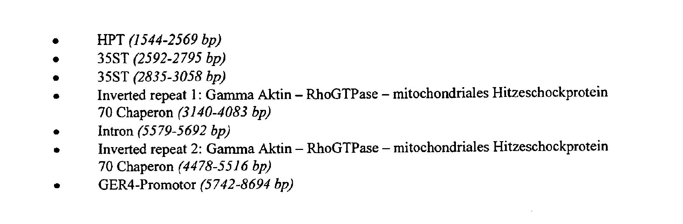

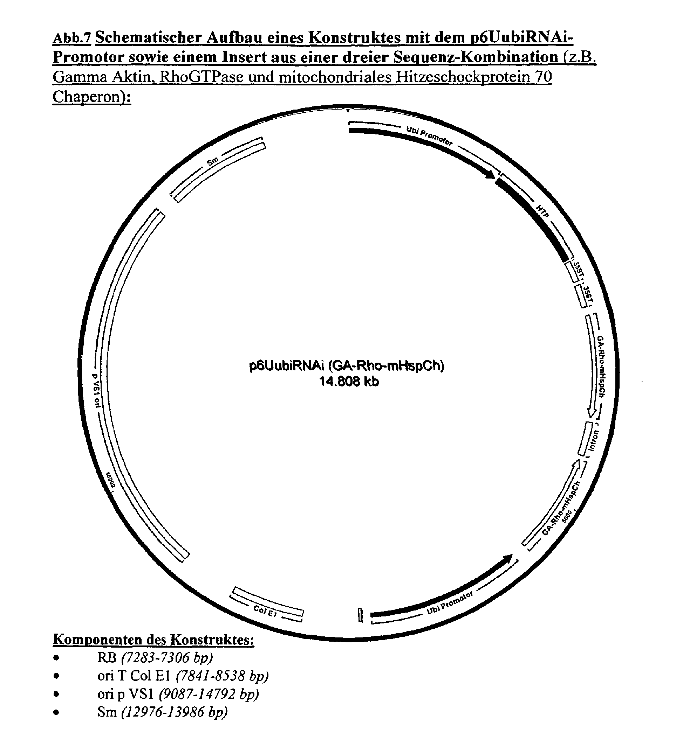

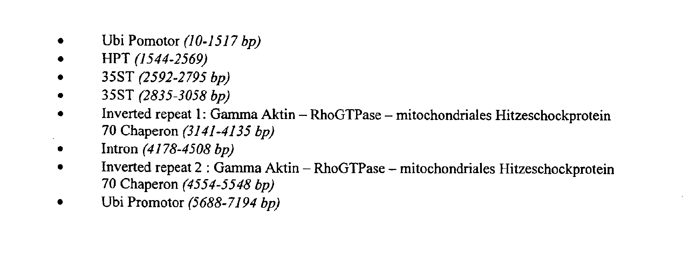

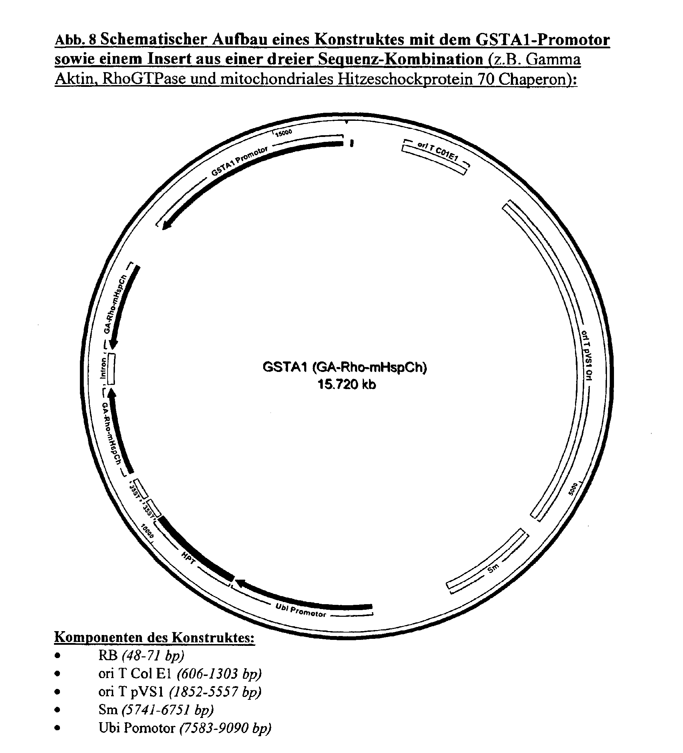

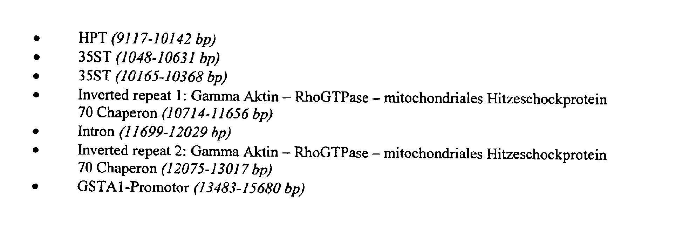

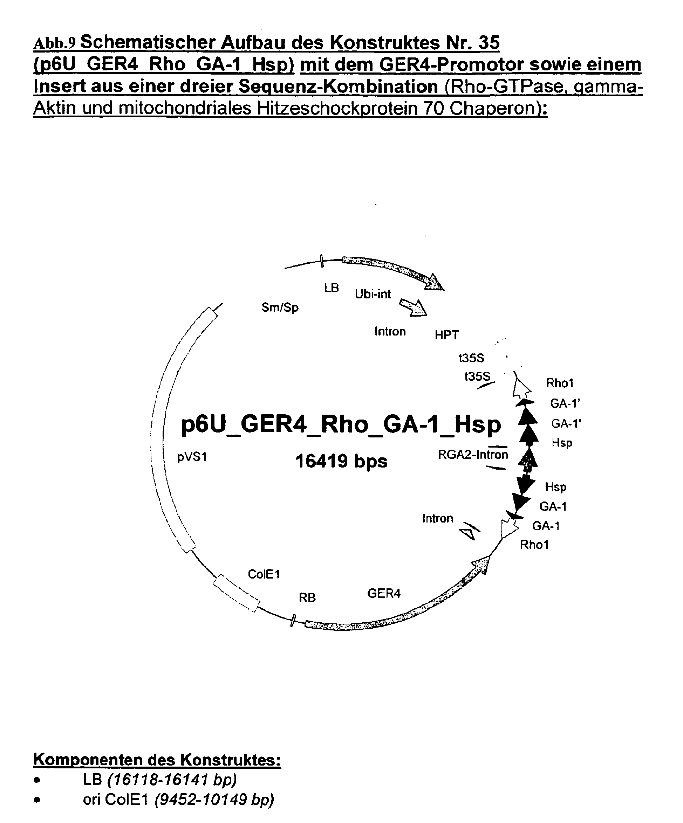

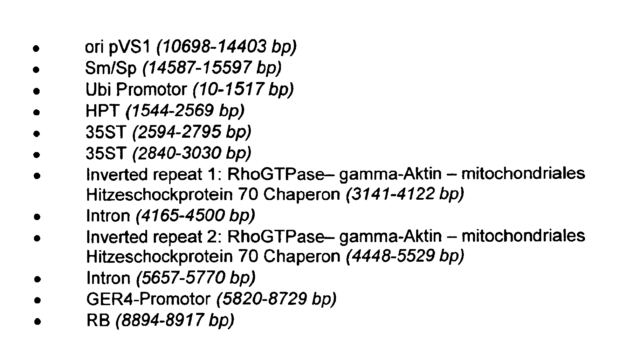

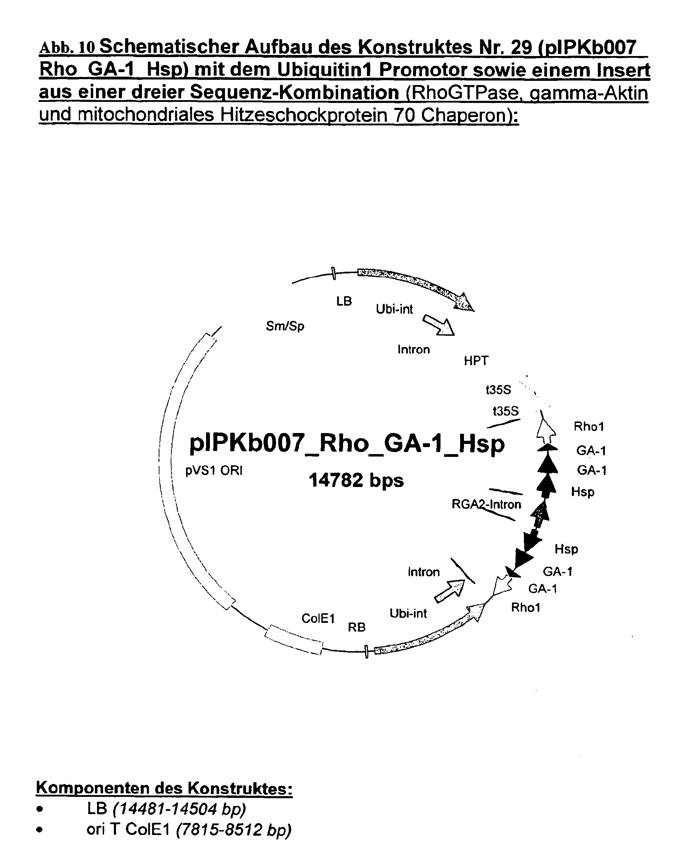

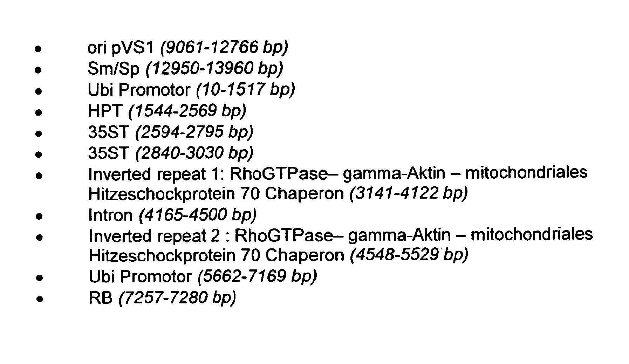

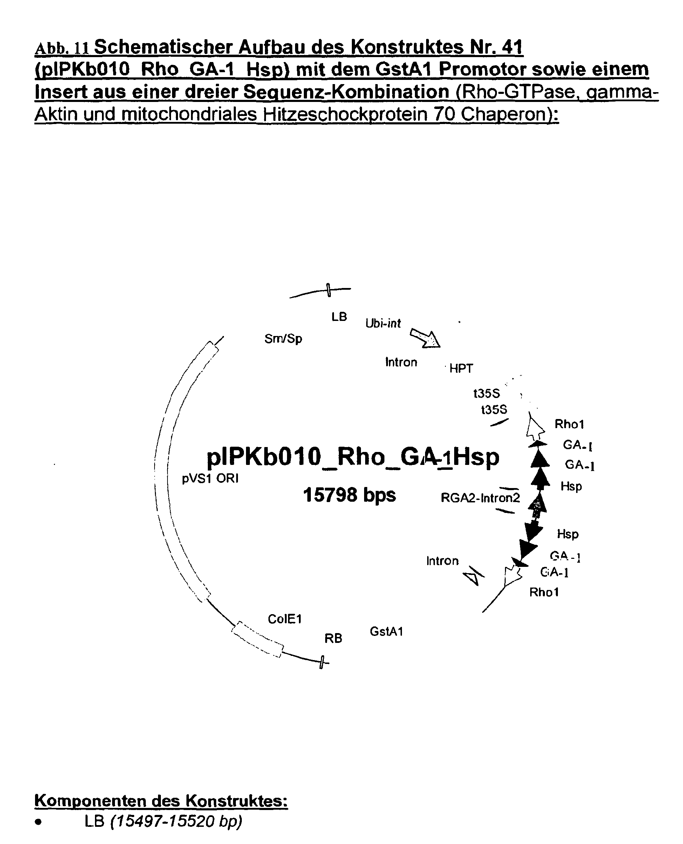

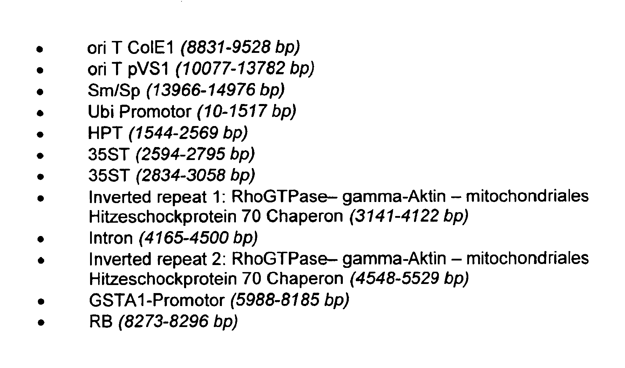

- Abb. 6 bis 11:: Schematische Darstellung verschiedener triple-siRNA-Vektoren

In den Nukleotid-Sequenzen wurden die untenstehenden Symbole für Nukleinsäuren verwendet:

| **Symbol** | **Bedeutung** | **Ableitung der Bezeichnung** |
|---|---|---|
| a | a | Adenin |
| **g** | **g** | Guanin |
| c | c | Cytosin |
| t | t | Ihymin |
| u | u | Uracil |
| r | g oder a | Purin |
| y | t/u oder c | Pyrimidin |
| m | a oder c | Amino |
| k | **g** oder t/u | Keto |
| s | **g** oder c | Starke Bindungon 3 H-Bruckon |
| w | a oder t/u | schwache (o: woak) Bindungen 2 H-Brucken |
| b | b oder c oder t/u | nicht a |
| d | a oder **g** oder t/u | nicht c |
| h | a oder c oder t/u | nicht **g** |
| v | a oder **g** oder c | nicht t. nicht u |
| n | a oder **g** oder c oder t/u, unbekannt oder sonstigo | boliobig (e: any) |

SEQ ID No.1
   FsCon[0026] AAC
   >FsCon[0026] 
TTTTCTCTCGACTCCCTCCATTCTCTCTTCCTTCTTTCATCCTCAATCTC
CCCTCAATCCTTTCTTCTTGTTCAAGGGAAAGACCATCACAGCCGCTACC
ATGTCTCCTCCTGCTGATCAAGGACCCCAGAAGGTCTTGGGCATGCCCCC
CTTCGTCGCTGACTTCCTCATGGGTGGTGTCTCCGCCGCTGTCTCCAAGA
CTGCTGCTGCCCCCATCGAGCGTGTCAAGCTCCTCATCCAGAACCAGGAT
GAGATGCTCAAGACCGGTCGTCTCGACCGCAAGTACAACGGCATTGGTG
ACTGCTTCAAGCGCACCATGGCCGATGAGGGTGTCATGTCCCTCTGGCGA
GGAAACACCGCCAACGTCATCCGATACTTCCCCACCCAGGCCCTGAACTT
CGCTTTCCGTGACAAGTTCAAGAAGATGTTCGGCTACAAGAAGGACAAG
GATGGCTACGCCATGTGGATGGCTGGTAACCTTGCCTCCGGTGGTGCTGC
TGGTGCCACTTCTCTGCTCTTCGTCTACTCTCTGGACTACGCCCGTACTCG
TCTTGCCAACGATGCCAAGAACGCCAAGTCCGGTGGTGACCGTCAGTTCA
ACGGTCTCGTCGACGTCTACAAGAAGACCCTCGCCTCTGACGGTATTGCC
GGTCTCTACCGTGGTTTCATGCCCTCCGTTGCTGGTATCGTTGTCTACCGT
GGTCTCTACTTCGGAATGTACGACTCCATCAAGCCCGTCGTCCTCACCGG
TAACCTCCAGGGCAACTTCCTTGC
SEQ ID No.2
   GzCon[0015] EF1
   >GzCon[0015] 
CTGAGAAGCWMGTCGCACGAGGCCCGGCACGAGCATCCGATCTGCGAA
TCTCACGTTCATCACAAACGTACACACAAACCATCCACAACCGTCAAAAT
GGGTAAGGAGGAGAAGACTCACCTTAACGTCGTCGTCATCGGCCACGTC
GACTCTGGCAAGTCGACCACTACCGGTCACTTGATCTACCAGTGCGGTGG
TATCGACAAGCGAACCATCGAGAAGTTCGAGAAGGAAGCCGCCGAGCTC
GGTAAGGGTTCCTTCAAGTACGCCTGGGTTCTTGACAAGCTCAAAGCCGA
GCGTGAGCGTGGTATCACCATTGATATCGCCCTCTGGAAGTTCGAGACTC
CTCGCTACTATGTCACCGTCATTGACGCTCCCGGTCACCGTGATTTCATCA
AGAACATGATCACTGGTACTTCCCAGGCCGATTGCGCCATTCTCATCATT
GCCGCCGGTACTGGTGAGTTCGAGGCTGGTATCTCCAAGGATGGCCAGA
CCCGTGAGCACGCTCTCCTTGCCTACACCCTTGGTGTCAAGAACCTCATT
GTTGCCATCAACAAGATGGACACCACCAAGTGGTCTGAGGCCCGTTACC
AGGAGATCATCAAGGAGACCTCTTCTTTCATCAAGAAGGTCGGCTACAAC
CCCAAGGCTGTCGCTTTCGTCCCCATCTCCGGTTTCAACGGTGACAACAT
GCTTACTGCCTCCACCAACTGCCCCTGGTACAAGGGTTGGGAGCGTGAGA
TCAAGTCTGGCAAGCTCTCCGGAAAGACCCTCCTTGAGGCCATTGACTCC
ATCGAGCCCCCCAAGCGTCCCAACGACAAGCCCCTCCGACTTCCCCTCCA
GGATGTCTACAAGATTGGCGGTATTGGAACGGTTCCTGTCGGCCGTATCG
AGACTGGTATCATCAAGCCCGGTATGGTCGTTACCTTCGCTCCTTCCAAC
GTCACCACTGAAGTCAAGTCCGTTGAGATGCACCACGAGCAGCTCACTG
AGGGACAGCCCGGTGACAACGTTGGTTTCAACGTGAAGAACGTTTCCGTC
AAGGACATCCGACGTGGTAACGTCGCTGGTGACTCCAAGAACGACCCCC
CCCATGGGTGCCGCTTCTTTCACCGCCCAGGTCATCGTCCTCAACCACCC
CGGTCAGGTCGGTGCTGGTTACGCTCCCGTCCTCGATTGCCACACTGCCC
ACATTGCCTGCAAGTTCGCCGAGATCCAGGAGAAGATCGACCGCCGAAC
CGGTAAGGCTACTGAGGCCGCCCCCAAGTTCATCAAGTCTGGTGACTCCG
CCATCGTCAAGATGGTTCCCTCCAAGCCCATGTGTGTTGAGGCTTTCACC
GACTACCCTCCTCTGGGTCGTTTCGCCGTCCGTGACATGCGACAGACCGT
CGCCGTCGGTGTCATCAAGGCCGTCGAGAAGTCCACTGGCGCTGCTGGCA
AGGTCACCAAGTCCGCTGCCAAGGCTGGTAAGAAATAAGCGCATTATTTC
TGAAATGACAACCTTCGGTGGGATCTTACGACTCTGGTCTCACGCCCAAG
GGCTGATGTACTCGCTTCTACGACGAAATGATCATGTTGGCATGAGCACT
CGGCTTATGTCTGGGGGCACGAGATGAGGCGAAAATAAAAGCCTGGGAT
CTTGTGCAAATCAAAAATAGGAATAGAGAGTCTAGAAGTGATTCGTGCA
AAAAAAAAAAAAAAAAAAAAAAAAAAAAAAAAAAAAAAAAACTCGAGAAA
CTCGAGACTAGTTCTCTCTCTCCCTCGTGC
SEO ID No.3
   GzCon[0219] GA
   >GzCon[0219] 
GCTCTTCTTTCCCGTAAGCTTGCTCCTCACTAATTCTAGCATCGCATCT
CATCACATTTGTCTTCTTTATACCACTTTGTCCTTTCTTTTTCTTCCTCG
CCTTGCAATCTTGGGCACGAGGCGTCTCTTCTTTTCCCCTTTCCCATACA
CACACCGTAATCCACAGCCATGGAGGAAGAAGTTGCTGCTCTCGTCATTG
ACAATGGTTCGGGTATGTGCAAGGCCGGTTTCGCCGGTGATGATGCTCCC
CGAGCTGTTTTCCCTTCCATTGTCGGTCGCCCCCGTCACCATGGTATCAT
GATCGGTATGGGTCAGAAGGACTCTTACGTTGGTGATGAGGCTCAGTCCA
AGCGTGGTATCCTCACACTGCGATACCCCATCGAGCACGGTGTTGTTACC
AACTGGGACGACATGGAGAAGATTTGGCACCACACCTTCTACAACGAGC
TGCGTGTCGCCCCCGAGGAGCACCCCGTCCTGCTCACCGAGGCTCCCATC
AACCCCAAGTCCAACCGTGAGAAGATGACCCAGATTGTCTTCGAGACCTT
CAACGCCCCCGCTTTCTACGTCTCTATCCAGGCCGTTCTGTCCCTGTACGC
CTCCGGTCGTACCACCGGTATCGTTCTGGACTCTGGTGATGGTGTCACTC
ACGTTGTCCCCATTTACGAGGGTTTCGCCCTTCCCCACGCCATTGCCCGT
GTCGACATGGCTGGTCGTGATCTTACCGACTACCTCATGAAGATTCTTGC
TGAGCGCGGTTACACCTTCTCCACCACTGCCGAGCGAGAAATCGTCCGTG
ACATCAAGGAGAAGCTCTGCTACGTCGCCCTTGACTTCGAGCAGGAGATC
CAGACTGCTGCCCAGAGCTCCAGCTTGGAGAAGTCTTACGAGCTTCCTGA
CGGTCAGGTTATCACCATTGGTAACGAGCGATTCCGTGCTCCTGAGGCTC
TCTTCCAGCCTTCTGTCCTTGGTCTTGAGAGCGGTGGTATCCACGTCACC
ACTTTCAACTCCATCATGAAGTGTGATGTCGATGTCCGAAAGGATCTCTA
CGGAAACATTGTCATGTCTGGTGGTACCACCATGTACCCCGGTCTCTCCG
ACCGTATGCAGAAGGAGATCACTGCTCTTGCTCCTTCTTCCATGAAGGTC
AAGATCATTGCTCCTCCCGAGCGAAAGTACTCCGTCTGGATCGGTGGTTC
CATTCTCGCTTCCCTGTCCACCTTCCAGCAAATGTGGATCTCCAAGCAGG
AGTACGACGAGAGCGGTCCTTCAATCGTTCACCGCAAGTGCTTCTAAGCT
ACCCGACCGACAAATTAAATCCTCCCACTCGCCTAACGACAAGATCATGG
AGGTGGCGACAGACGCTAAGCTTTGGGTGGTTGGCCAACGTCAATGTCG
AATCTCGTATCTACGAAAAGTTTTTGACATGGTCGAAAGAGTGGGCGTTG
CGCTTGTTACGAAGAGATGTGATGGCCCCTTTTCTTGTAGTTCGCGTATA
ATGCGTGCCTAGGGGTAGAAGCCGAGGTTGACATACAAATACTTTTTTAC
TTTCCGMWWARAAAAAAAAAAACTCGAGGG
SEO ID No.4
   GzCon[2886] Hsp
   >GzCon[2886] 
GCACGAGGAGAGAGAGAGAACTAGTTTCGAGTTTTTTTTTTTTTTT
TTTTTTTWTGAATGTATGATTCATTGATTCATTMAAGTACATARAYCACC
TTAGCTTTTCATAACCGTAAAGTCGCATAAAAACAGAAAAAAGGAAAAT
CTAATCTCATCATCCCATGAAAACGACTAGCCTCCGCATTATACCCATGG
ATATATGACCGCTCTAGAAGTCGAGACTGATGTTATTGGGGTATCAAAGA
ATTTAGTCGACCTCCTCGACGGTGGGACCATCGTCACCACCGGCACCGGG
GGCGGGGCCACCAGCACCAGGGAAGCCACCAGGGCCGCCGGGCATGCCG
GGCATGCCACCGGGAGCACCCTCACCACCAGCTCCGTAGAACTTCATCAT
GATGGGGTTAGCCTTGCCCTCGAGCTCCTTCTGGTGCTCCTCATACTCCTC
ACGAGTAGCCTGCTGGTTGTCATCGAGCCACTGGACGACCTTGTCAATCT
CAGCAGTGAGGGTCTCCTTGTCGGAAGCCTCAATCTTCTCCTCGACCTTG
GGGTCGGAGAGAGTGTTGCGGAGAGAGTAAGCGTAAGACTCAAGACCGT
TCTTGGCAGCGACACGCTTGCCCTCGGCCTCATCCTCCTCCTTGTACTTCT
CAGCATCGTTGAGCATGCGCTCGATCTCCTCCTTGGACAGGCGGCCCTTG
TCGTTGGTGATGACGATCTTGTTGGACTTGCCGGTACCCTTCTCGACGGC
GGAAACGTTCATGATACCGTTGGCATCGAGATCGAAGGTGACCTCAATCT
GGGGAACACCACGGGGAGCAGGGGGATACCAGTGAGCTCGAACTTGCC
CATGAGGTTGTTGTCCTTGGTGCGCTGGCGCTCACCCTCGTAGACCTGGA
TAAGGACACCAGGCTGGTTGTCGGAGAAGGTAGAGAAGACCTCGGACTT
CTTGGTGGGAATGGTGGTGTTGCGGGGGATGAGCTTGGTCATCATACCAC
CAGCGGTCTCGATACCGAGAGAGAGGGGGGCGACGTCGAGGAGCAGAA
TCTCGTTGGTGGCCTTGCTAGAGGTGTCACCAGAGAGAATAGCAGCCTGG
ACAGCGGCACCGTAGGCAACAGCCTCATCAGGATTGATGGACTTGTTGG
GCTCCTTTCCGTTGAAGTAGTCGGTGATGAGCTTCTGGACACGGGGGATA
CGGGTAGATCCACCGACGAGGACGATCTCGTGGACGAGGGACTTGTCGA
TCTTGGCGTCGGTAAGGACACGGTCGACGGGCTGGATGGTGGATCGGAA
GAGATCCTGGCAGAGCTCCTCGAAACGAGCACGGGTGATGGAAGTGTAG
AAATCAATACCCTCGAAGAGAGAGTCGATCTCAATGGAGGTCTGAGCGG
AAGAAGAGAGAGTTCGCTTGGCGCGCTCACAAGCGGTTCGGAGACGTCG
AAGAGCACGGACGTTGGTGCTTAGATCCTTCTTATGCTTTCGCTTGAACT
CGTTAACGAAGTGGTTAACGAGGCGGTTGTCGAAATCTTCACCACCCAAG
TGAGTGTCACCGGCAGTAGACTTGACCTCGAAGATACCCTCCTCAATGGT
AAGGAGAGAGACATCGAAGSYWCSWSCRCCAAGATCGAAGATGAGGAC
GTTGCGCTCACCCTCAACCTTCTCCTCGTGC
SEQ ID No.5
   FsCon[0840] Rho
   >FsCon[0840] 
CCAAGCTCAACTCAAGCTCAAGCTCGCATTCTACGCCAGCAAATTCGTCC
ATTTCTCACAACTTCTTCACCCAAAAACACTTCTAATCAATAACCATGGC
TGAGATCCGCCGAAAGCTCGTCATTGTCGGCGATGGTGCTTGTGGTAAAA
CCTGTTTGTTGATCGTTTTCTCCAAGGGCACTTTCCCCGAGGTCTACGTC
CCCACCGTCTTCGAGAACTATGTCGCCGATGTCGAGGTTGACGGCAAGCA
CGTCGAGCTCGCCCTATGGGATACTGCTGGTCAGGAGGATTACGACCGTC
TTCGACCTCTCTCTTACCCCGACTCCCACGTTATCTTGATCTGCTTCGCT
GTTGACTCTCCCGACTCTCTCGACAACGTCCAGGAGAAGTGGATCTCTGA
GGTTCTGCACTTCTGCCAGGGKCTCCCTATCATCCTTGTCGGCTGCAAGA
AGGATTTGCGATACGACC
SEO ID No.6
   FsCon[0026] AAC Nt. 322-622 
CGATGAGGGTGTCATGTCCCTCTGGCGAGGAAACACCGCCAACGTCATCC
GATACTTCCCCACCCAGGCCCTGAACTTCGCTTTCCGTGACAAGTTCAAG
AAGATGTTCGGCTACAAGAAGGACAAGGATGGCTACGCCATGTGGATGG
CTGGTAACCTTGCCTCCGGTGGTGCTGCTGGTGCCACTTCTCTGCTCTTCG
TCTACTCTCTGGACTACGCCCGTACTCGTCTTGCCAACGATGCCAAGAAC
GCCAAGTCCGGTGGTGACCGTCAGTTCAACGGTCTCGTCGACGTCTACAA
G
SEQ ID No.7
   GzCon[0015] EF1 Nt. 907-1204 
ATCAAGCCCGGTATGGTCGTTACCTTCGCTCCTTCCAACGTCACCACTGA
AGTCAAGTCCGTTGAGATGCACCACGAGCAGCTCACTGAGGGACAGCCC
GGTGACAACGTTGGTTTCAACGTGAAGAACGTTTCCGTCAAGGACATCCG
ACGTGGTAACGTCGCTGGTGACTCCAAGAACGACCCCCCCATGGGTGCC
GCTTCTTTCACCGCCCAGGTCATCGTCCTCAACCACCCCGGTCAGGTCGG
TGCTGGTTACGCTCCCGTCCTCGATTGCCACACTGCCCACATTGCCTGC
SEQ ID No.8
   GzCon[0219] GA Nt. 837-1141 
TCGAGCAGGAGATCCAGACTGCTGCCCAGAGCTCCAGCTTGGAGAAGTC
TTACGAGCTTCCTGACGGTCAGGTTATCACCATTGGTAACGAGCGATTCC
GTGCTCCTGAGGCTCTCTTCCAGCCTTCTGTCCTTGGTCTTGAGAGCGGTG
GTATCCACGTCACCACTTTCAACTCCATCATGAAGTGTGATGTCGATGTC
CGAAAGGATCTCTACGGAAACATTGTCATGTCTGGTGGTACCACCATGTA
CCCCGGTCTCTCCGACCGTATGCAGAAGGAGATCACTGCTCTTGCTCCTT
CTTCC
SEQ ID No.9
   GA-1 
TTCGAGCAGGAGATCCAGACTGCTGCCCAGAGCTCCAGCTTGGAGAAGT
CTTACGAGCTTCCTGACGGTCAGGTTATCACCATTGGTAACGAGCGATTC
CGTGCTCCTGAGGCTCTCTTCCAGCCTTCTGTCCTTGGTCTTGAGAGCGGT
GGTATCCACGTCACCACTTTCAACTCCATCATGAAGTGTGATGTCGATGT
CCGAAAGGATCTCTACGGAAACATTGTCATGGTAAGTTCTGCAAATCGAA
AGCACTCCAACTGTTCTAACATGTCTTTAGTCTGGTGGTACCACCATGTA
CCCCGGTCTCTCCGACCGTATGCAGAAGGAGATCACTGCTCTTGCTCCTT
CTTCCA
SEQ ID No. 10
   GzCon[2886] Hsp Nt. 842-1197 
ACTTGCCCATGAGGTTGTTGTCCTTGGTGCGCTGGCGCTCACCCTCGTAGACCTGGATAA
GGACACCAGGCTGGTTGTCGGAGAAGGTAGAGAAGACCTCGGACTTCTTGGTGGGAATG
GTGGTGTTGCGGGGGATGAGCTTGGTCATCATACCACCAGCGGTCTCGATACCGAGAGA
GAGGGGGGCGACGTCGAGGAGCAGAATCTCGTTGGTGGCCTTGCTAGAGGTGTCACCAG
AGAGAATAGCAGCCTGGACAGCGGCACCGTAGGCAACAGCCTCATCAGGATTGATGGAC
TTGTTGGGCTCCTTTCCGTTGAAGTAGTCGGTGATGAGCTTCTGGACACGGGGGATACGG
SEQ ID No.11
   FsCon[0840] Rho Nt. 137-444 
TGCTTGTGGTAAAACCTGTTTGTTGATCGTTTCTCCAAGGGCACTTTCCC
CGAGGTCTACGTCCCCACCGTCTTCGAGAACTATGTCGCCGATGTCGAGG
TTGACGGCAAGCACGTCGAGCTCGCCCTATGGGATACTGCTGGTCAGGA
GGATTACGACCGTCTTCGACCTCTCTCTTACCCCGACTCCCACGTTATCTT
GATCTGCTTCGCTGTTGACTCTCCCGACTCTCTCGACAACGTCCAGGAGA
AGTGGATCTCTGAGGTTCTGCACTTCTGCCAGGGKCTCCCTATCATCCTTG
TCGGCT
SEQ ID No.12
   AAC Konsensus gekürzt 
GCAGACCGTTCTTGGGCATGCCCCCCTTCGTCGCTGACTTCCTCATGGGT
GGTGTCTCCGCCGCTGTCTCCAAGACCGCTGCYGCCCCCATYGAGCGTGT
CAAGCTCCTCATCCAGAACCAGGATGAGATGCTGAAGACCGGTCGTCTY
GACCGCAAGTACGACGGCATTGGTGASTGCTTCAAGCGYACCACCGCSGA
TGAGGGTGTCATGTCCCTCTGGCGWGGHAACACTGCCAACGTCATCCGTT
ACTTCCCTACCCAGGCCCTGAACTTCGCTTTCCGTGACAAGTTCAAGTCG
ATGTTCGGCTACAAGAAGGACCGTGATGGCTACGCCATGTGGATGGCYG
GTAACYTKGCCTCCGGTGGTGCTGCTGGTGCCACTTCCCTCCTCTTCGTCT
ACTCCCTCGACTACGCCCGTACYCGTCTTGCCAACGACGCCAAGAACGCC
AAGACCGGTGGTGACCGTCAGTTCAACGGTCTSGTCGATGTCTACAAGAA
GACCCTCGCCTCTGACGGTATTGCCGGTCTCTACCGTGGTTTCGGT:CCTT
CCGTTGCTGGTATCGTCGTYTACCGTGGTCTCTACTTCG
SEQ ID No.13
   EF1 Konsensus gekürzt 
ATGGGTAAGGAGAAGACTCACATCAACGTCGTCGTCATCGGCCACGTC
GACTCCGGCAAGTCGACCACCACCGGTCACTTGATCTACAAGTGCGGTGG
TATCGACAAGCGTACCATCGAGAAGTTCGAGAAGGAAGCCGCCGAGCTC
GGYAAGGGTTCCTTCAAGTACGCCTGGGTTCTTGACAAGCTCAAGGCCGA
GCGTGAGCGTGGTATCACCATCGACATCGCCCTCTGGAAGTTCGAGACYC
CCAAGTACTATGTTACCGTCATTGACGCCCCTGGTCACCGTGACTTCATC
AAGAACATGATCACTGGTACCTCCCAGGCCGACTGCGCCATTCTCATCAT
TGCCGCTGGTACTGGTGAGTTCGAGGCTGGTATCTCCAAGGATGGCCAGA
CTCGTGAGCACGCTCTCCTCGCCTACACCCTCGGTGTCAAGCAGCTCATC
GTYGCCATCAACAAGATGGACACCACCAAGTGGTCCGAGGACCGTTWCC
AGGAGATCATCAAGGAGACCTCCAACTTCATCAAGAAGGTCGGCTACAA
CCCCAAGACYGTCGCCTTCGTCCCCATCTCCGGTTTCAACGGTGACAACA
TGMTCGACGCCTCCACCAACTGCCCCTGGTACAAGGGTTGGGAGAAGGA
GACCAAGKCWGGCAAGGYCACCGGCAAGACCCTCCTCGAGGCCATCGA
CGCCATCGAGCCCCCCAAGCGTCCCACCGACAAGCCCCTCCGTCTTCCCC
TCCAGGATGTCTACAAGATCGGYGGTATTGGMACTGTTCCCGTCGGCCGT
RTCGAGACYGGTATCATCAAGCCCGGYATGGTCGTCACCTTCGCTCCCGC
CAACGTCACCACTGAAGTCAAGTCCGTCGAGATGCACCACGAGCAGCTC
ACTGAGGGTSTTCCCGGTGACAACGTCGGTTTCAACGTCAAGAACGTTTC
CGTCAAGGACATTCGCCGTGGTAACGTCGCTGGTGACTCCAAGAACGAC
CCCCCCAAGGGTGCCGCTTCCTTCAACGCYCAGGTCATYGTCCTYAACCA
CCCTGGTCAGGTCGGTGCTGGTTACGCCCCCGTTCTCGATTGCCACACTG
CCCACATTGCCTGCAAGTTCTCCGAGCTCCTCGAGAAGATCGACCGCCGT
ACCGGTAAGTCCATTGAGGACTCCCCCAAGTTCATCAAGTCTGGTGACGC
TGCCATCGTCAAGATGGTTCCCTCCAAGCCCATGTGTGTTGAGGCCTTCA
CYGACTACCCTCCTCTGGGMCGTTTCGCCGTCCGTGACATGCGTCAGACC
GTCGCTGTCGGTGTCATCAAGTCCGTCGACAAGTCCACGACACTGCCGGT
AAGGTCACCAAG
SEQ ID No.14
   GA Konsensus gekürzt 
ATGGAGGAAGAAGTCGCWGCTCTCGTCATYGACAATGGTTCGGGTATGT
GCAAGGCCGGTTTCGCCGGTGACGATGCTCCCCGAGCTGTCTTCCCTTCC
ATTGTCGGTCGCCCCCGTCACCATGGTATCATGATTGGTATGGGCCAGAA
GGACTCGTACGTTGGTGATGAGGCCCAGTCCAAGCGTGGTATCCTCACTC
TGCGGTACCCCATCGAGCACGGTGTYGTCACCAACTGGGACGACATGGA
GAAGATCTGGCACCACACCTTCTACAACGAGCTGCGTGTCGCCCCCGAGG
AGCACCCCGTCCTGCTGACTGAGGCTCCCATCAACCCCAAGTCCAACCGT
GAGAAGATGACGCAGATCGTCTTCGAGACCTTCAACGCCCCCGCCTTCTA
CGTCTCCATCCAGGCCGTKCTGTCCCTGTACGCCTCCGGTCGTACCACTG
GTATCGTGCTCGACTCYGGTGACGGTGTTACTCACGTTGTGCCCATCTAC
GAGGGTTTCKCCCTKCCCCACGCCATTGCCCGTGTCGACATGGCTGGTCG
TGACTTGACCGACTACCTGATGAAGATCTTGGCTGAGCGCGGTTACACCT
TCTCCACCACTGCCGAGCGWGAAATCGTCCGTGACATCAAGGAGAAGCT
CTGCTACGTCGCCCTTGACTTCGAGCAGGAGATCCAGACTGCTGCCCAGT
CCTCCAGCYTGGAGAAGTCCTACGAGCTTCCCGACGGACAGGTTATCACC
ATCGGCAACGAGCGCTTCCGTGCTCCTGAGGCTCTGTTCCAGCCTTCTGT
CCTGGGTCTTGAGAGCGGTGGTATCCACGTCACCACTTTCAACTCCATCA
TGAAGTGTGATGTCGATGTCCGAAAGGATCTGTACGGCAACATTGTCATG
TCTGGTGGTACCACCATGTACCCTGGTATCTCCGACCGTATGCAGAAGGA
GATCACTGCTCTTGCCCCKTCCTCSATGAAGGTCAAGATCATTGCTCCTCC
CGAGCGCAAGTACTCCGTCTGGATCGGTGGT:TCCATTCTC
SEQ ID No.15
   Hsp Konsensus gekürzt 
TCCACGAGATCGTCCTCGTYGGYGGWTCYACCCGTATCCCCCGTGTCCAG
AAGCTCATCACCGACTACTTCAACGGAAAGGAGCCCAACAAGTCCATCA
ACCCYGATGAGGCTGTTGCCTACGGTGCTGCCGTCCAGGCTGCCATTCTC
TCTGGTGACACCACCTCCAAGTCCACCAACGAGATCCTGCTYCTCGATGT
CGCCCCSCTSTCTCTCGGTATCGAGACCGCTGGTGGTATGATGACCAAGC
TCATCCCCCGCAACACCACCATCCCCACCAAGAAGTCYGAGGTCTTCTCC
ACCTTCTCCGACAACCAGCCTGGTGTGCTCATCCAGGTCTACGAGGGTGA
GCGCCAGCGCACCAAGGACAACAACCTSCTGGGCAAGTTCGAGCTTACC
GGCATCCCMCCTGCTCCCCGTGGTGTTCCCCAGATTGAGGTCACCTTCGA
CCTSGATGCCAACGGTATCATGAACGTCTCCGCCGTCGAGAAGGGCACCG
GCAAGACCAACAAGATTGTCATCACCAACGACAAGGGCCGYCTGTCCAA
GGAGGAGATYGAGCGCATGCTTGCYGAGGCCGAGAAGTACAAGGAGGA
GGATGA
SEQ ID No.16
   Rho Konsensus gekürzt

AATCCATMACCATGGCTGAAATCCGCCGAAAGCTCGTCATTGTCGGHGA
YGGTGCTTGTGGTAAGACYTGTTTGTTGATTGTCTTCTCCAAGGGCACHT
TCCCMGAGGTCTACGTCCCMACCGTCTTCGAGAACTATGTCGCCGATGTC
GAGGTCGATGGCAAGCACGTCGAGCTCGCGCTATGGGATACBGCTGGTC
AGGAGGATTACGACCGTCTTCGACCTCTCTCATACCCCGACTCSCACGTT
ATCCTGATCTGCTTCGCCGTTGACTCTCCCGATTCCCTCGAYAACGTYCA
GGAGAAGTGGATCTCCGAGGTCCTSCAYTTCTGCCAGGGTCTCCCTATCA
TCCTTGTCGGCTGCAAGAAGGATTTGCGAYACGACCAGAAGACCATTGA
GGAGCTCCACAAGACCA

## Patentansprüche

1. Verfahren zur Erzeugung einer Breitband-Resistenz gegenüber Pilzen in transgenen Pflanzen,
**dadurch gekennzeichnet, dass** ein rekombinantes Nukleinsäuremolekül umfassend mindestens eine Nukleinsäuresequenz in die Pflanze eingebracht wird, wobei die Nukleinsäuresequenz
a) identisch und/oder komplementär zu einer oder mehreren der Nukleinsäuren ausgewählt aus SEQ ID Nos 1-5 und/oder einem Teil davon ist, oder eine Sequenzidentität von mindestens 85% zu einer oder mehreren der Nukleinsäuren ausgewählt aus SEQ ID Nos 1-5 aufweist, und/oder
b) identisch und/oder komplementär zu einer oder mehreren der Nukleinsäuren ausgewählt aus SEQ ID Nos 6-11 und/oder einem Teil davon ist, oder eine Sequenzidentität von mindestens 85% zu einer oder mehreren der Nukleinsäuren ausgewählt aus SEQ ID Nos 6-11 aufweist, oder
c) identisch und/oder komplementär zu einer oder mehreren der Nukleinsäuren ausgewählt aus SEQ ID Nos 12-16 und/oder einem Teil davon ist, oder eine Sequenzidentität von mindestens 85% zu einer oder mehreren der Nukleinsäuren ausgewählt aus SEQ ID Nos 12-16 aufweist,
und in der Pflanze transkribiert wird, so dass bei Infektion der Pflanze mit einem Pilz die Nukleinsäuresequenz oder Teile davon mit einer oder mehreren der korrespondierenden und/oder komplementären Nukleinsäuren des Pilzes derart interagiert, dass die Expression der pilzeigenen Nukleinsäuresequenz im Wesentlichen unterbunden wird.

2. Verfahren nach Anspruch 1, wobei das rekombinante Nukleinsäuremolekül umfasst:
(a) einen in Pflanzen funktionsfähigen Promotor,
(b) operativ damit verbunden die mindestens eine Nukleinsäuresequenz.

3. Verfahren nach Anspruch 1, wobei das rekombinante Nukleinsäuremolekül umfasst:
(a) einen in Pflanzen funktionsfähigen Promotor,
(b) operativ damit verbunden die mindestens eine Nukleinsäuresequenz
(c) ein Intron umfassend Spleißdonor- und Spleißakzeptorsequenzen,
(d) die revers-komplementäre Nukleinsäuresequenz zu der unter b) genannten Nukleinsäuresequenz
(e) eine Terminationssequenz.

4. Verfahren nach Anspruch 1, wobei das rekombinante Nukleinsäuremolekül umfasst:
(a) einen in Pflanzen funktionsfähigen Promotor,
(b) operativ damit verbunden die mindestens eine Nukleinsäuresequenz, wobei die Sequenz über revers-komplementäre Bereiche verfügt, und
(c) eine Terminationssequenz.

5. Verfahren nach Anspruch 1, wobei das rekombinante Nukleinsäuremolekül umfasst:
(a) einen in Pflanzen funktionsfähigen Promotor,
(b) operativ damit verbunden die mindestens eine Nukleinsäuresequenz
(c) eine weitere Nukleinsäuresequenz, die für Ribonuklease P kodiert, und
(d) eine Terminationssequenz.

6. Verfahren nach einem der Ansprüche 1 bis 5 wobei der Promotor ein konstitutiver Promotor, ein induzierbarer Promotor oder ein gewebespezifischer Promotor ist.

7. Verfahren nach einem der Ansprüche 1 bis 6, wobei die mindestens eine Nukleinsäuresequenz ausgewählt ist aus der Gruppe bestehend aus
(i) Nukleinsäuren, die SEQ ID Nr. 1-16 oder Fragmente davon umfassen, und/oder
(ii) Nukleinsäuren, die unter stringenten Bedingungen mit einem komplementären Strang einer Nukleinsäure von (i) hybridisieren.

8. Verfahren nach einem der Ansprüchen 1 bis 7, wobei es sich bei den transgenen Pflanzen um dikotyledone oder monokotyledone Pflanzen handelt.

9. Verfahren nach Anspruch 8, wobei es sich bei den Pflanzen um Getreidepflanzen, insbesondere um Weizen oder Gerste handelt.

10. Verfahren nach einem der Ansprüche 1 bis 9, wobei es sich um eine Breitband-Resistenz gegenüber Schadpilzen handelt.

11. Transgene Pflanzenzelle mit Breitband-Resistenz hergestellt nach einem Verfahren nach einem der Ansprüche 1 bis 10, wobei das rekombinante Nukleinsäuremolekül zwei Nukleinsäuresequenzen umfasst, wobei eine Nukleinsäuresequenz
a) identisch und/oder komplementär zu einer oder mehreren der Nukleinsäuren ausgewählt aus SEQ ID Nos 1-5 und/oder einem Teil davon ist, oder eine Sequenzidentität von mindestens 85% zu einer oder mehreren der Nukleinsäuren ausgewählt aus SEQ ID Nos 1-5 aufweist, und eine weitere Nukleinsäuresequenz
b) identisch und/oder komplementär zu einer oder mehreren der Nukleinsäuren ausgewählt aus SEQ ID Nos 6-11 und/oder einem Teil davon ist, oder eine Sequenzidentität von mindestens 85% zu einer oder mehreren der Nukleinsäuren ausgewählt aus SEQ ID Nos 6-11 aufweist, oder
c) identisch und/oder komplementär zu einer oder mehreren der Nukleinsäuren ausgewählt aus SEQ ID Nos 12-16 und/oder einem Teil davon ist, oder eine Sequenzidentität von mindestens 85% zu einer oder mehreren der Nukleinsäuren ausgewählt aus SEQ ID Nos 12-16 aufweist.

12. Transgene Pflanzenzelle nach Anspruch 10, enthaltend ein rekombinantes Nukleinsäuremolekül umfassend einen Promotor operativ verbunden mit mindestens einer Nukleinsäuresequenz, wobei das rekombinante Nukleinsäuremolekül zwei Nukleinsäuresequenzen umfasst, wobei eine Nukleinsäuresequenz
a) identisch und/oder komplementär zu einer oder mehreren der Nukleinsäuren ausgewählt aus SEQ ID Nos 1-5 und/oder einem Teil davon ist, oder eine Sequenzidentität von mindestens 85% zu einer oder mehreren der Nukleinsäuren ausgewählt aus SEQ ID Nos 1-5 aufweist, und eine weitere Nukleinsäuresequenz
b) identisch und/oder komplementär zu einer oder mehreren der Nukleinsäuren ausgewählt aus SEQ ID Nos 6-11 und/oder einem Teil davon ist, oder eine Sequenzidentität von mindestens 85% zu einer oder mehreren der Nukleinsäuren ausgewählt aus SEQ ID Nos 6-11 aufweist, oder
c) identisch und/oder komplementär zu einer oder mehreren der Nukleinsäuren ausgewählt aus SEQ ID Nos 12-16 und/oder einem Teil davon ist, oder eine Sequenzidentität von mindestens 85% zu einer oder mehreren der Nukleinsäuren ausgewählt aus SEQ ID Nos 12-16 aufweist.

13. Transgene Pflanzenzelle enthaltend ein rekombinantes Nukleinsäuremolekül wie definiert in den Ansprüchen 1 bis 7, wobei das rekombinante Nukleinsäuremolekül zwei Nukleinsäuresequenzen umfasst, wobei eine Nukleinsäuresequenz
a) identisch und/oder komplementär zu einer oder mehreren der Nukleinsäuren ausgewählt aus SEQ ID Nos 1-5 und/oder einem Teil davon ist, oder eine Sequenzidentität von mindestens 85% zu einer oder mehreren der Nukleinsäuren ausgewählt aus SEQ ID Nos 1-5 aufweist, und eine weitere Nukleinsäuresequenz
b) identisch und/oder komplementär zu einer oder mehreren der Nukleinsäuren ausgewählt aus SEQ ID Nos 6-11 und/oder einem Teil davon ist, oder eine Sequenzidentität von mindestens 85% zu einer oder mehreren der Nukleinsäuren ausgewählt aus SEQ ID Nos 6-11 aufweist, oder
c) identisch und/oder komplementär zu einer oder mehreren der Nukleinsäuren ausgewählt aus SEQ ID Nos 12-16 und/oder einem Teil davon ist, oder eine Sequenzidentität von mindestens 85% zu einer oder mehreren der Nukleinsäuren ausgewählt aus SEQ ID Nos 12-16 aufweist.

14. Transgene Pflanze, enthaltend eine Pflanzenzelle nach einem der Ansprüche 11 bis 13.

15. Rekombinantes Nukleinsäuremolekül umfassend mindestens zwei Nukleinsäuresequenzen in operativer Verbindung mit einem in Pflanzenzellen funktionsfähigen Promotor, wobei eine Nukleinsäuresequenz
a) identisch und/oder komplementär zu einer oder mehreren der Nukleinsäuren ausgewählt aus SEQ ID Nos 1-5 und/oder einem Teil davon ist, oder eine Sequenzidentität von mindestens 85% zu einer oder mehreren der Nukleinsäuren ausgewählt aus SEQ ID Nos 1-5 aufweist, und eine weitere Nukleinsäuresequenz
b) identisch und/oder komplementär zu einer oder mehreren der Nukleinsäuren ausgewählt aus SEQ ID Nos 6-11 und/oder einem Teil davon ist, oder eine Sequenzidentität von mindestens 85% zu einer oder mehreren der Nukleinsäuren ausgewählt aus SEQ ID Nos 6-11 aufweist, oder
c) identisch und/oder komplementär zu einer oder mehreren der Nukleinsäuren ausgewählt aus SEQ ID Nos 12-16 und/oder einem Teil davon ist, oder eine Sequenzidentität von mindestens 85% zu einer oder mehreren der Nukleinsäuren ausgewählt aus SEQ ID Nos 12-16 aufweist.

## Claims

1. Method for creating broad-spectrum resistance to fungi in transgenic plants, **characterized in that** a recombinant nucleic acid molecule comprising at least one nucleic acid sequence is introduced in the plant, wherein the nucleic acid sequence
a) is identical and/or complementary to one or more of the nucleic acids selected from SEQ ID NOs: 1-5 and/or a part thereof, or has a sequence identity of at least 85 % to one or more of the nucleic acids selected from SEQ ID NOs: 1-5, and/or
b) is identical and/or complementary to one or more of the nucleic acids selected from SEQ ID NOs: 6-11 and/or a part thereof, or has a sequence identity of at least 85 % to one or more of the nucleic acids selected from SEQ ID NOs: 6-11, or
c) is identical and/or complementary to one or more of the nucleic acids selected from SEQ ID NOs: 12-16 and/or a part thereof, or has a sequence identity of at least 85 % to one or more of the nucleic acids selected from SEQ ID NOs: 12-16,
and is transcribed in the plant, so that in case of an infection of the plant with a fungus, the nucleic acid sequence or parts thereof interacts with one or more of the corresponding and/or complementary nucleic acids of the fungus such that the expression of the fungal nucleic acid sequence is substantially inhibited.

2. Method according to claim 1, wherein the recombinant nucleic acid molecule comprises:
(a) a promotor functional in plants,
(b) operatively linked thereto the at least one nucleic acid sequence.

3. Method according to claim 1, wherein the recombinant nucleic acid molecule comprises:
(a) a promotor functional in plants,
(b) operatively linked thereto the at least one nucleic acid sequence
(c) an intron comprising splice donor and splice acceptor sequences,
(d) the nucleic acid sequence being reverse-complementary to the nucleic acid sequence mentioned in b)
(e) a termination sequence.

4. Method according to claim 1, wherein the recombinant nucleic acid molecule comprises:
(a) a promotor functional in plants,
(b) operatively linked thereto the at least one nucleic acid sequence, wherein said sequence has reverse-complementary regions, and
(c) a termination sequence.

5. Method according to claim 1, wherein the recombinant nucleic acid molecule comprises:
(a) a promotor functional in plants,
(b) operatively linked thereto the at least one nucleic acid sequence
(c) another nucleic acid sequence encoding the ribonuclease P, and
(d) a termination sequence.

6. Method according to any one of claims 1 to 5, wherein the promotor is a constitutive promotor, an inducible promotor or a tissue-specific promotor.

7. Method according to any one of claims 1 to 6, wherein the at least one nucleic acid sequence is selected from the group consisting of
(i) nucleic acids, comprising SEQ ID NOs: 1-16 or fragments thereof, and/or
(ii) nucleic acids which hybridize under stringent conditions with a complementary strand of a nucleic acid of (i).

8. Method according to any one of the claims 1 to 7, wherein the transgenic plants are dicotyledonous or monocotyledonous plants.

9. Method according to claim 8, wherein the plants are cereal plants, in particular wheat or barley.

10. Method according to any one of claims 1 to 9, wherein the broad-spectrum resistance is against pathogenic fungi.

11. A transgenic plant cell having broad-spectrum resistance being produced according to the method of any one of claims 1 to 10, wherein the recombinant nucleic acid molecule comprises two nucleic acid sequences, wherein one nucleic acid sequence
a) is identical and/or complementary to one or more of the nucleic acids selected from SEQ ID NOs: 1-5 and/or a part thereof, or has a sequence identity of at least 85 % to one or more of the nucleic acids selected from SEQ ID NOs: 1-5, and a further nucleic acid sequence
b) is identical and/or complementary to one or more of the nucleic acids selected from SEQ ID NOs: 6-11 and/or a part thereof, or has a sequence identity of at least 85 % to one or more of the nucleic acids selected from SEQ ID NOs: 6-11, or
c) is identical and/or complementary to one or more of the nucleic acids selected from SEQ ID NOs: 12-16 and/or a part thereof, or has a sequence identity of at least 85 % to one or more of the nucleic acids selected from SEQ ID NOs: 12-16.

12. Transgenic plant cell according to claim 10 containing a recombinant nucleic acid molecule comprising a promotor operatively linked to at least one nucleic acid sequence, wherein the recombinant nucleic acid molecule comprises two nucleic acid sequences, wherein one nucleic acid sequence
a) is identical and/or complementary to one or more of the nucleic acids selected from SEQ ID NOs: 1-5 and/or a part thereof, or has a sequence identity of at least 85 % to one or more of the nucleic acids selected from SEQ ID NOs: 1-5, and a further nucleic acid sequence
b) is identical and/or complementary to one or more of the nucleic acids selected from SEQ ID NOs: 6-11 and/or a part thereof, or has a sequence identity of at least 85 % to one or more of the nucleic acids selected from SEQ ID NOs: 6-11, or
c) is identical and/or complementary to one or more of the nucleic acids selected from SEQ ID NOs: 12-16 and/or a part thereof, or has a sequence identity of at least 85 % to one or more of the nucleic acids selected from SEQ ID NOs: 12-16.

13. Transgenic plant cell containing a recombinant nucleic acid molecule as defined in claims 1 to 7, wherein the recombinant nucleic acid molecule comprises two nucleic acid sequences, wherein one nucleic acid sequence
a) is identical and/or complementary to one or more of the nucleic acids selected from SEQ ID NOs: 1-5 and/or a part thereof, or has a sequence identity of at least 85 % to one or more of the nucleic acids selected from SEQ ID NOs: 1-5, and a further nucleic acid sequence
b) is identical and/or complementary to one or more of the nucleic acids selected from SEQ ID NOs: 6-11 and/or a part thereof, or has a sequence identity of at least 85 % to one or more of the nucleic acids selected from SEQ ID NOs: 6-11, or
c) is identical and/or complementary to one or more of the nucleic acids selected from SEQ ID NOs: 12-16 and/or a part thereof, or has a sequence identity of at least 85 % to one or more of the nucleic acids selected from SEQ ID NOs: 12-16.

14. Transgenic plant cell containing a plant cell according to any one of claims 11 to 13.

15. Recombinant nucleic acid molecule comprising at least two nucleic acid sequences operatively linked to a promotor functional in plant cells, wherein one nucleic acid sequence
a) is identical and/or complementary to one or more of the nucleic acids selected from SEQ ID NOs: 1 - 5 and/or a part thereof, or has a sequence identity of at least 85 % to one or more of the nucleic acids selected from SEQ ID NOs: 1 - 5, and a further nucleic acid sequence
b) is identical and/or complementary to one or more of the nucleic acids, selected from SEQ ID NOs: 6 - 11 and/or a part thereof, or has a sequence identity of at least 85 % to one or more of the nucleic acids selected from SEQ ID NOs: 6 - 11, or
c) is identical and/or complementary to one or more of the nucleic acids, selected from SEQ ID NOs: 12 - 16 and/or a part thereof, or has a sequence identity of at least 85 % to one or more of the nucleic acids selected from SEQ ID NOs: 12 - 16.

## Revendications

1. Procédé de création d'une résistance à large spectre contre des champignons dans des plantes transgéniques,
**caractérisé en ce qu'**une molécule d'acides nucléiques recombinante comportant au moins une séquence d'acides nucléiques est introduite dans la plante, où la séquence d'acides nucléiques
a) est identique et/ou complémentaire à un ou plusieurs des acides nucléiques sélectionnés à partir de SEQ ID n^{os} 1-5 et/ou à une partie de ceux-ci, ou présente une identité de séquence d'au moins 85 % à un ou plusieurs des acides nucléiques sélectionnés à partir de SEQ ID n^{os} 1-5, et/ou
b) est identique et/ou complémentaire à un ou plusieurs des acides nucléiques sélectionnés à partir de SEQ ID n^{os} 6-11 et/ou à une partie de ceux-ci, ou présente une identité de séquence d'au moins 85 % à un ou plusieurs des acides nucléiques sélectionnés à partir de SEQ ID n^{os} 6-11, ou
c) est identique et/ou complémentaire à un ou plusieurs des acides nucléiques sélectionnés à partir de SEQ ID n^{os} 12-16 et/ou à une partie de ceux-ci, ou présente une identité de séquence d'au moins 85 % à un ou plusieurs des acides nucléiques sélectionnés à partir de SEQ ID n^{os} 12-16,
et est transcrite dans la plante de sorte que, en cas d'infection de la plante avec un champignon, la séquence d'acides nucléiques ou des parties de celle-ci interagisse avec un ou plusieurs des acides nucléiques correspondants et/ou complémentaires du champignon de telle façon que l'expression de la séquence d'acides nucléiques propre au champignon soit sensiblement inhibée.

2. Procédé selon la revendication 1, où la molécule d'acides nucléiques recombinante comprend :
(a) un promoteur fonctionnel dans des plantes,
(b) en liaison opérative avec celui-ci, l'au moins une séquence d'acides nucléiques.

3. Procédé selon la revendication 1, où la molécule d'acides nucléiques recombinante comprend :
(a) un promoteur fonctionnel dans des plantes,
(b) en liaison opérative avec celui-ci, l'au moins une séquence d'acides nucléiques,
(c) un intron comprenant des séquences donneuses et acceptrices d'épissage,
(d) la séquence d'acides nucléiques complémentaire inversée à la séquence d'acides nucléiques citée en b)
(e) une séquence de terminaison.

4. Procédé selon la revendication 1, où la molécule d'acides nucléiques recombinante comprend :
(a) un promoteur fonctionnel dans des plantes,
(b) en liaison opérative avec celui-ci, l'au moins une séquence d'acides nucléiques, la séquence disposant de zones complémentaires inversées, et
(c) une séquence de terminaison.

5. Procédé selon la revendication 1, où la molécule d'acides nucléiques recombinante comprend :
(a) un promoteur fonctionnel dans des plantes,
(b) en liaison opérative avec celui-ci, l'au moins une séquence d'acides nucléiques,
(c) une séquence d'acides nucléiques supplémentaire qui code pour le ribonucléase P, et
(d) une séquence de terminaison.

6. Procédé selon l'une des revendications 1 à 5, où le promoteur est un promoteur constitutif, un promoteur inductible ou un promoteur spécifique du tissu.

7. Procédé selon l'une des revendications 1 à 6, où l'au moins une séquence d'acides nucléiques est sélectionnée à partir du groupe constitué par
(i) des acides nucléiques qui comprennent SEQ ID n^{os} 1-16 ou des fragments de ceux-ci, et/ou
(ii) des acides nucléiques qui s'hybrident en conditions stringentes avec une chaîne complémentaire d'un acide nucléique de (i).

8. Procédé selon l'une des revendications 1 à 7, où les plantes transgéniques sont des plantes dicotylédones ou monocotylédones.

9. Procédé selon la revendication 8, où les plantes sont des plantes céréalières, en particulier du blé ou de l'orge.

10. Procédé selon l'une des revendications 1 à 9, où il s'agit d'une résistance à large spectre contre des champignons nuisibles.

11. Cellule végétale transgénique à résistance à large spectre fabriquée selon un procédé selon l'une des revendications 1 à 10, où la molécule d'acides nucléiques recombinante comprend deux séquences d'acides nucléiques, où une séquence d'acides nucléiques
a) est identique et/ou complémentaire à un ou plusieurs des acides nucléiques sélectionnés à partir de SEQ ID n^{os} 1-5 et/ou à une partie de ceux-ci, ou présente une identité de séquence d'au moins 85 % à un ou plusieurs des acides nucléiques sélectionnés à partir de SEQ ID n^{os} 1-5, et une séquence d'acides nucléiques supplémentaire
b) est identique et/ou complémentaire à un ou plusieurs des acides nucléiques sélectionnés à partir de SEQ ID n^{os} 6-11 et/ou à une partie de ceux-ci, ou présente une identité de séquence d'au moins 85 % à un ou plusieurs des acides nucléiques sélectionnés à partir de SEQ ID n^{os} 6-11, ou
c) est identique et/ou complémentaire à un ou plusieurs des acides nucléiques sélectionnés à partir de SEQ ID n^{os} 12-16 et/ou à une partie de ceux-ci, ou présente une identité de séquence d'au moins 85 % à un ou plusieurs des acides nucléiques sélectionnés à partir de SEQ ID n^{os} 12-16.

12. Cellule végétale transgénique selon la revendication 10, contenant une molécule d'acides nucléiques recombinante comprenant un promoteur en liaison opérative avec au moins une séquence d'acides nucléiques, où la molécule d'acides nucléiques recombinante comprend deux séquences d'acides nucléiques, où une séquence d'acides nucléiques
a) est identique et/ou complémentaire à un ou plusieurs des acides nucléiques sélectionnés à partir de SEQ ID n^{os} 1-5 et/ou à une partie de ceux-ci, ou présente une identité de séquence d'au moins 85 % à un ou plusieurs des acides nucléiques sélectionnés à partir de SEQ ID n^{os} 1-5, et une séquence d'acides nucléiques supplémentaire
b) est identique et/ou complémentaire à un ou plusieurs des acides nucléiques sélectionnés à partir de SEQ ID n^{os} 6-11 et/ou à une partie de ceux-ci, ou présente une identité de séquence d'au moins 85 % à un ou plusieurs des acides nucléiques sélectionnés à partir de SEQ ID n^{os} 6-11, ou
c) est identique et/ou complémentaire à un ou plusieurs des acides nucléiques sélectionnés à partir de SEQ ID n^{os} 12-16 et/ou à une partie de ceux-ci, ou présente une identité de séquence d'au moins 85 % à un ou plusieurs des acides nucléiques sélectionnés à partir de SEQ ID n^{os} 12-16.

13. Cellule végétale transgénique contenant une molécule d'acides nucléiques recombinante telle que définie dans les revendications 1 à 7, où la molécule d'acides nucléiques recombinante comprend deux séquences d'acides nucléiques, où une séquence d'acides nucléiques
a) est identique et/ou complémentaire à un ou plusieurs des acides nucléiques sélectionnés à partir de SEQ ID n^{os} 1-5 et/ou à une partie de ceux-ci, ou présente une identité de séquence d'au moins 85 % à un ou plusieurs des acides nucléiques sélectionnés à partir de SEQ ID n^{os} 1-5, et une séquence d'acides nucléiques supplémentaire
b) est identique et/ou complémentaire à un ou plusieurs des acides nucléiques sélectionnés à partir de SEQ ID n^{os} 6-11 et/ou à une partie de ceux-ci, ou présente une identité de séquence d'au moins 85 % à un ou plusieurs des acides nucléiques sélectionnés à partir de SEQ ID n^{os} 6-11, ou
c) est identique et/ou complémentaire à un ou plusieurs des acides nucléiques sélectionnés à partir de SEQ ID n^{os} 12-16 et/ou à une partie de ceux-ci, ou présente une identité de séquence d'au moins 85 % à un ou plusieurs des acides nucléiques sélectionnés à partir de SEQ ID n^{os} 12-16.

14. Plante transgénique, contenant une cellule végétale selon l'une des revendications 11 à 13.

15. Molécule d'acides nucléiques recombinante comprenant au moins deux séquences d'acides nucléiques en liaison opérative avec un promoteur fonctionnel dans des cellules végétales, où une séquence d'acides nucléiques
a) est identique et/ou complémentaire à un ou plusieurs des acides nucléiques sélectionnés à partir de SEQ ID n^{os} 1-5 et/ou à une partie de ceux-ci, ou présente une identité de séquence d'au moins 85 % à un ou plusieurs des acides nucléiques sélectionnés à partir de SEQ ID n^{os} 1-5, et une séquence d'acides nucléiques supplémentaire
b) est identique et/ou complémentaire à un ou plusieurs des acides nucléiques sélectionnés à partir de SEQ ID n^{os} 6-11 et/ou à une partie de ceux-ci, ou présente une identité de séquence d'au moins 85 % à un ou plusieurs des acides nucléiques sélectionnés à partir de SEQ ID n^{os} 6-11, ou
c) est identique et/ou complémentaire à un ou plusieurs des acides nucléiques sélectionnés à partir de SEQ ID n^{os} 12-16 et/ou à une partie de ceux-ci, ou présente une identité de séquence d'au moins 85 % à un ou plusieurs des acides nucléiques sélectionnés à partir de SEQ ID n^{os} 12-16.
